(19) Europäisches Patentamt
European Patent Office
Office européen des brevets

(11) EP 2 623 129 B1

(12) # FASCICULE DE BREVET EUROPEEN

(45) Date de publication et mention de la délivrance du brevet:
**17.09.2014 Bulletin 2014/38**

(51) Int Cl.:
***A61L 15/28*** *(2006.01)* ***A61L 15/32*** *(2006.01)*
***A61L 15/38*** *(2006.01)*

(21) Numéro de dépôt: **12179072.9**

(22) Date de dépôt: **02.08.2012**

(54) **Pansement et procede de fabrication**

Verband und sein Herstellungsverfahren

Dressing and manufacturing method

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.02.2012 US 201261594843 P**

(43) Date de publication de la demande:
**07.08.2013 Bulletin 2013/32**

(73) Titulaire: **MA.I.A Woundcare**
**95000 Neuville sur Oise (FR)**

(72) Inventeurs:
- **Giraudier, Sébastien**
  **95800 CERGY (FR)**
- **Perrin, Sébastien**
  **78600 MAISONS -LAFFITTE (FR)**
- **Picard, Julien**
  **95520 OSNY (FR)**
- **Klak, Marie-Cécile**
  **95160 MONTMORENCY (FR)**
- **Garde, Larreta**
  **95290 L'ISLE ADAM (FR)**

(74) Mandataire: **Novagraaf Technologies**
**122, rue Edouard Vaillant**
**92593 Levallois-Perret Cedex (FR)**

(56) Documents cités:
**WO-A2-2006/056700 WO-A2-2009/095562**
**GB-A- 2 382 305**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### Domaine technique

La présente invention se rapporte à un pansement ainsi qu'à un procédé de fabrication dudit pansement.

La présente invention trouve une application notamment dans le domaine médical humain ou vétérinaire, par exemple pour soigner les plaies.

### Etat de la technique

Il existe actuellement dans le commerce un grand nombre de type de pansements dont certains sont spécialement adapté à la plaie à soigner.

Les objectifs principaux des pansements sont de protéger une plaie et de prévenir une infection en assurant, le cas échéant une désinfection de la plaie, protéger la peau de la macération et de l'action irritante des sécrétions, protéger la plaie des traumatismes et aider à la cicatrisation.

Les pansements ont également pour objectif d'assurer le confort du patient, par exemple via la réalisation des objectifs précités, en particulier en protégeant la plaie des traumatismes et en aidant à la cicatrisation.

Il existe également dans le commerce des pansements « actifs » et/ou « spécialisées » capables respectivement de libérer des substances actives et/ou qui sont spécialement adaptés à différent type de plaie et permettent par exemple d'améliorer la cicatrisation et/ou le confort du patient.

Les principaux types de pansement actuellement commercialisés sont les hydrogels, les tulles et interfaces, les hydrocolloïdes, les hydrocellulaires, les alginates et les pansements au charbon.

Chaque type de pansement a des propriétés et applications particulières.

Par exemple, les hydrogels sont particulièrement utiles sur des plaies chroniques superficielles et profondes, des escarres, des ulcères de jambe, chirurgie réparatrice et reconstructrice, les plaies atone, la dermabrasion, les coups de soleil sévères, les brûlures du 2ème degré superficiel et profond. De tels pansements sont disponibles dans le commerce, il s'agit par exemple de l'Askina Gel commercialisé par la société B Braun, le Duoderm Hydrogel commercialisé par la société Convatec, l'Hydrosorb commercialisé par la société Hartmann, l'IntraSite Gel commercialisé par la société Smith&Nephew, le Normgel commercialisé par la société Mölnlycke, le Purilon commercialisé par la société Coloplas et l'Urgo hydrogel commercialisé par la société Urgo.

Les hydrocolloïdes sont également fréquemment utilisés sur des plaies exsudatives bourgeonnantes non infectées mais sont contre indiqués, par exemple pour des brûlures du 3ème degré, des artérites IV, des mycoses, une plaie surinfectée, des plaies diabétiques. De tels pansements sont disponibles dans le commerce, par exemple le Askina Hydro commercialisé par la société B. Braun, l'Algoplaque commercialisé par la société Urgo le Comfeel commercialisé par la société Coloplast, le Duoderm commercialisé par la société Convatec et l'Hydrocoll commercialisé par la société Hartmann.

GB2382305 décrit un pansement permettant un traitement efficace de plaies chroniques, et comporte une couche absorbante pouvant être un non-tissé et une couche d'hydrogel.

WO2006/056700 décrit un biomatériau comprenant au moins un monomère capable de former des polymères et un premier type d'enzyme capable de dégrader lesdits polymères. Le polymère peut être une protéine ou un polysaccharide. Le biomatériau peut également comprendre un second type d'enzyme capable d'effectuer des liaisons covalentes entre les monomères.

Toutefois, malgré le grand nombre de type de pansements, il n'existe pas dans l'état de la technique de pansement permettant un traitement efficace de plaies chroniques telles que les plaies du diabétique. En outre, les pansements actuellement disponibles posent des problèmes lors de leur retrait. En effet, il est souvent nécessaire, par exemple d'humidifier le pansement et/ou de protéger la zone environnante, afin de permettre le retrait, par exemple sans abimer la plaie cicatrisée et/ou sans laisser sur la plaie des morceaux de pansements.

En outre, les pansements connus appliqués sur une plaie suintante peuvent coller à la plaie et/ou s'incruster dans la plaie, gênant ainsi la cicatrisation et/ou provoquant lors de retrait une détérioration de la cicatrisation. En effet, la cicatrisation d'une plaie opère toujours du bord vers le centre. De nombreux pansements sèche et colle sur les bords de la plaie. Leur retrait entraîne l'arrachage des cellules néoformées de la zone périlésionnelle, ce qui ralenti le processus de cicatrisation.

Il existe donc un réel besoin dans l'état de la technique de trouver un nouveau pansement palliant ces défauts, inconvénients et obstacles de l'art antérieur, en particulier d'un pansement permettant, par exemple d'améliorer la cicatrisation de plaie avec une application simple et un retrait ne nécessitant pas de préparation/manipulation particulière.

**Description de l'invention**

La présente invention a pour but de surmonter les inconvénients de l'art antérieur en fournissant un pansement comprenant un support non tissé comprenant sur une de ces faces, un biomatériau capable de faire des transitions gel/solution comprenant une phase aqueuse et un réseau de polymères comprenant un polymère protéique ou saccharidique ou un mélange de polymères protéiques et saccharidiques, dans lequel le réseau de polymères et la phase aqueuse forment un gel, une première enzyme de dégradation dudit polymère, et une seconde enzyme capable d'effectuer des liaisons covalentes entre lesdits monomères, ledit biomatériau étant lyophilisé.

Dans la présente le biomatériau peut être un biomatériau capable de faire des transitions solution / gel puis gel solution de façon contrôlée dans le temps.

Les inventeurs ont découvert de manière surprenante qu'un pansement comprenant un biomatériau capable de faire des transitions solution / gel puis gel solution de façon contrôlée dans le temps, ledit biomatériau étant en contact avec la plaie, permet avantageusement, d'absorber des exsudats de plaies tout en fournissant un environnement similaire à la matrice extracellulaire permettant ainsi de favoriser la cicatrisation. En outre le pansement selon l'invention, de part sa capacité à faire des transitions gel-solution, permet avantageusement un retrait atraumatique du pansement qui se décolle de la plaie une fois cicatrisée. En effet, le gel protecteur formé sur la plaie à la pause du pansement peut être adhésif aux cellules périlésionelles, une fois la transition effectuée, c'est à dire l'état du gel se transforme, deviens plus mou et relargue un peu de liquide, qui rend le pansement non adhésif.

Dans la présente par pansement on entend, par exemple un dispositif utilisé dans le domaine médical humain ou vétérinaire, par exemple un dispositif applicable sur une plaie, par exemple une plaie humaine ou animale.

Dans la présente la plaie peut être une plaie superficielle ou une plaie profonde. Il peut s'agir par exemple d'une rupture et/ou d'une atteinte de la barrière cutanée, ou d'une rupture des différentes couches de l'épiderme et/ou du derme jusqu'au défaut total, c'est-à-dire jusqu'à l'absence de d'épiderme et/ou du derme.

Il peut s'agir par exemple d'une plaie située sur n'importe quelle partie du corps, il peut s'agir par exemple d'une plaie cutanée, d'une plaie de l'oeil, d'une plaie au thorax, d'une plaie à l'abdomen, d'une plaie à la bouche.

Dans la présente la plaie peut être une plaie chronique ou aigue. Par exemple, la plaie aigue peut être une blessure, une morsure, une lacération, une plaie chirurgicale, une brulure. Par exemple la plaie chronique, peut être un ulcère, un escarre, une plaie du diabétique, toute plaie issue de pathologie dermatologiques, une plaie cancéreuse, par exemple muscites, ulcération cancéreuse, ulcération arthéritique, stromatite ulcérée, plaie d'origine herpétiques, plaie chirugical, ou issue d'une greffe.

Il peut s'agir également d'une lésion, par exemple une lésion du corps provenant par exemple d'une blessure sanglante, d'une contusion, d'un accident physiologique.

Dans la présente, le pansement peut être sous toute forme, par exemple sous une forme rectangulaire, circulaire, linéaire et/ou adaptée à la forme de la plaie, et/ou à la position de la plaie. De préférence le pansement est sous une forme rectangulaire sans arrête vives.

Dans la présente, le pansement peut être par exemple un pansement de forme circulaire, un polygone, par exemple un quadrilatère, un pentagone, un hexagone, un heptagone, un rectangle, un carré, un parallélogramme, un triangle, un losange, une élipse ou toute forme adaptée au lit de la plaie.

Dans la présente, le pansement peut être par exemple un pansement de forme rectangulaire, par exemple ayant une dimension de 5 X 5 mm, de 250 x 250, de 500 x 500, de 1000 x 750, de 1500 x 1000 mm. Il peut s'agir par exemple une dimension de 65 x 100 mm.

Selon l'invention, la première enzyme peut être choisie, par exemple dans le groupe comprenant les enzymes de la famille des métalloprotéinases, la famille des sérines protéases, la famille des cystéines et aspartate protéases, la famille ADAM, les glycosidases dont l'amylase, la cellulase, la dextranase, la pullulanase, la pectinase, la chitinase, la xanthanase, la chitosanase, la hyaluronidase, et les lyases dont l'hydroxyacétyle lyase, la chondroïtinase, l'héparinase, l'alginate lyase. De préférence, la première enzyme est une collagénase.

Selon l'invention, la seconde enzyme peut être choisie par exemple dans le groupe comprenant les enzymes de la famille des transglutaminases, des lysyls oxydases, les protéines disulfide isomérases, les sulfhydrile thiol oxydases, les peroxidases, les lipoxygènases, les épimèrases dont les alginates épimèrases, les glucuronate isomérases, la cellobiose épimèrase et les galactose-6-sulfurylases. De préférence, la seconde enzyme est une transglutaminase.

Selon l'invention le polymère protéique peut être choisi par exemple dans le groupe comprenant la fibrine, la gliadine, la myosine, la globuline (7S et 11S), l'actine, la myoglobine, le collagène et ses dérivés, les protéines du lait, les protéines du soja, les protéines du blé, et les protéines du jaune et du blanc d'oeuf, les protéines du pois, les protéines de féverole, les protéines du lin, les protéines de soie, la fibronectine, la laminine, l'élastine, la vitronectine. De préférence, le polymère est de la gélatine.

Dans la présente, le polymère saccharidique est choisi par exemple dans le groupe comprenant les carraghénanes, les alginates, le xanthane, le chitosan, la chitine, l'acide hyaluronique, les glycosaminoglycanes sulfatés, le glycogène, la cellulose et ses dérivés, les pectines, l'amidon et ses dérivés, les dextrans et les xylanes.

Dans la présente, le biomatériau peut comprendre en outre un composé cryoprotecteur. Il peut s'agir par exemple d'un cryoprotecteur choisi parmi du sorbitol du mannitol le glycérol, le sorbitol, des polyols, par exemple : l'Erythritol, le Xylitol, l'Arabitol, le Ribitol, le Dulcitol ou galactitol, le Mannitol, le Volemitol, le Maltitol, l'Isomaltitol, le Lactitol ou lactositol, le glycol et ses polymères dérivés par exemple le polyéthylène glycol (PEG). De préférence le cryoprotecteur est le sorbitol.

La concentration de cryoprotecteur dans la solution de polymère peut être de 0,1 à 1 M, par exemple de 0,2 à 0,5 M, ou être de 2 à 10%, par exemple de 3 à 4% en poids..

La présence du cryoprotecteur permet avantageusement que le pansement de l'invention soit plus souple et que le biomatériau lyophilisé soit plus flexible. L'amélioration de la flexibilité/souplesse du pansement permet avantageusement une meilleure adaptation à la surface de la plaie, par exemple, d'assurer une conformabilité à la plaie.

Dans la présente, le non tissé peut comprendre des mailles de 1 à 1000 $\mu$m, de préférence de 5 à 500 $\mu$m.

Avantageusement la taille des mailles du non-tissé permet avantageusement une forte adhésion du biomatériau sur ledit non tissé. En outre, la taille des mailles permet avantageusement d'éviter la colonisation du pansement par les cellules de la plaie.

Avantageusement, la dimension des mailles du non tissé permet une imprégnation du biomatériau dans ledit non tissé tout en conservant une épaisseur de biomatériau à la surface du non-tissé suffisante.

Dans la présente, l'épaisseur du non tissé peut être inférieure à 10$\mu$m, de préférence inférieur à 1 $\mu$m.

Dans la présente, le non tissé peut être un non-tissé sans polyuréthane et sans polyacrylate.

Dans la présente, le non tissé peut être en toute matière connue de l'homme du métier, par exemple, il peut être en viscose, en ouate, en cellulose, en polymère synthétique ou naturel, de préférence le non tissé est en viscose.

Dans la présente, le non tissé peut être par exemple un non tissé commercialisé par la société Mankk, le non tissé MA 60, MA 50 J, WSV 100, MA57 S commercialisé par la société Mank (Allemagne), le 71.100.001 commercialisé par la société Norafin (Allemagne).

Avantageusement, le non-tissé permet le maintien de la structure du pansement et du biomatériau. En d'autres termes, le non-tissé permet avantageusement d'éviter toute déchirure du pansement sur la plaie. En outre, le non tissé permet avantageusement lors du retrait du pansement la conservation de la structure du pansement et donc d'éviter une éventuelle déchirure du pansement lors de son retrait, ainsi que tout délitement du gel pouvant induire la présence de résidu non désirable dans la plaie.

Dans la présente, le pansement peut comprendre en outre une substance active dans le biomatériau et/ou dans le non tissé.

Dans la présente, par « substance active » on entend toute substance ou composition ayant une activité biologique ou biochimique à la surface d'un organisme (microorganisme ou organisme pluricellulaire, par exemple peau, os, organe, etc.) ou dans un organisme. Cette substance active peut posséder par exemple des propriétés curatives ou préventives à l'égard de maladies humaines ou animales. Il peut s'agir de tout produit pouvant être administré à l'homme ou à l'animal, en vue d'établir un diagnostic médical ou de restaurer, corriger ou modifier leurs fonctions organiques. Il peut s'agir de substances bactériostatiques et/ou bactéricides, d'antibiotiques, d'aseptisant, de colorants, etc. Il peut s'agir de toute molécules permettant de réduire et/ou supprimer la douleur, par exemple les opioïdes, les anti-inflamatoires, non stéroïdiens, etc...

Dans la présente, la substance active peut être choisie par exemple dans le groupe comprenant, les bactériostatiques, les bactéricides, les vasodilatateurs, les colorants dont l'éosine, le bleu de dextran, le bleu de methylène, le bleu azur, des protéines, des saccharides dont l'acide hyaluronique et les alginates, un liposome, une nanoparticule, une micelle, les antiacnéiques, les antiallergiques, les anxiolytiques, les antiasthmatiques, les anticancéreux, les hypolipémiants , les contraceptifs hormonaux, les antidépresseurs, les antidiabètiques, les antalgiques, les opioïdes et leurs dérivés, par exemple la morphine, les antiasthéniques, les antihypertenseurs, les antifongiques, les antibiotiques, les somnifères, les traitements hormonaux, les antimigraineux, les médicaments du surpoids, les antiparkinsoniens, les neuroleptiques, les anti-inflammatoires non stéroïdiens, les inducteurs d'ovulation, les fluidifiants bronchiques, les antitussifs, les inducteurs d'érection et les antiulcéreux.

Dans la présente, il peut s'agir d'une substance active seule ou d'un mélange de substances actives.

Dans la présente, la quantité de substance active peut dépendre, par exemple de facteurs tels que son activité et de la dose souhaitée par l'utilisateur. La dose souhaitée peut être aisément déterminée par l'homme du métier puisqu'il peut s'agir, par exemple de doses connues pour des produits connus.

La présente invention a également pour objet un procédé de fabrication d'un pansement comprenant les étapes de :

a. préparation d'une solution polymère comprenant un polymère protéique et/ou un polymère saccharidique par mélange dudit polymère dans une solution tampon,
b. préparation d'une solution enzyme comprenant la première et la seconde enzyme par mélange desdites enzymes dans une solution tampon,

c. mélange de la solution polymère et de la solution enzyme,
d. dépôt du mélange de l'étape (c) sur une face d'un non-tissé,
e. réticulation en gel du mélange de l'étape (c) déposé sur le non tissé,
f. lyophilisation du gel formé sur le non tissé,

Dans la présente, l'étape de préparation de la solution de polymères peut être réalisée par mélange par agitation, par exemple via un agitateur magnétique ou par des pales. La préparation de la solution de polymères peut être réalisée par agitation, par exemple à une vitesse de 1 à 2000 rpm (tours par minutes), de préférence de 160 à 200 rpm (tours par minutes).

Dans la présente, l'étape de préparation de la solution de polymères peut être réalisée à une température de 10 à 50°C, de préférence de 34 à 42°C.

Dans la présente, le polymère utilisé à l'étape (a) dans le procédé peut être un polymère protéique et/ou un polymère saccharidique tel que définie précédemment. La concentration dudit polymère dans le mélange l'étape (a) peut être comprise de 0,5 à 5 % en poids, de préférence de 2 à 4% en poids.

Dans la présente, la solution de polymère peut comprendre en outre un cryoprotecteur choisi parmi du sorbitol du mannitol le glycérol, le sorbitol, des polyols, par exemple : l'erythritol, le xylitol, l'arabitol, le ribitol, le dulcitol, le galactitol, le mannitol, le volemitol, le maltitol, l'isomaltitol, le lactitol ou lactositol, le glycol et ses polymères dérivés par exemple le polyéthylène glycol (PEG), de préférence le cryoprotecteur est le sorbitol.

La concentration de cryoprotecteur dans la solution de polymère peut être de 0,1 à 1 M, par exemple de 0,2 à 0,5 M, ou être de 2 à 10%, par exemple de 3 à 4% en poids..

Par exemple lorsque le cryoprotecteur est le sorbitol, la concentration de sorbitol dans la solution de polymère peut être de 0,1 à 1 M, par exemple de 0,2 à 0,5 M, ou être de 2 à 10%, de préférence de 3 à 4% en poids.

Dans la présente, l'étape de préparation de la solution d'enzyme peut être réalisée par mélange par agitation, par exemple via un agitateur magnétique, ou par des pales. La préparation de la solution d'enzymes peut être réalisée par agitation exemple à une vitesse de 1 à 2000 rpm par exemple de 100 à 250 rpm, d'une première et seconde enzyme dans une solution tampon.

Dans la présente, l'étape de préparation de la solution d'enzymes peut être réalisée à une température de 10 à 50°C, par exemple de 34 à 42°C.

Dans la présente, la première et la seconde enzyme utilisées sont telles que définies précédemment. Par exemple, la première enzyme peut être une collagénase. Par exemple la seconde enzyme peut être une transglutaminase.

Dans la présente, l'activité dans la solution d'enzyme de la première enzyme peut être comprise de $2x10^{-7}$ à 50 U/ml (unité/ml), par exemple de $10^{-6}$ à $10^{-5}$ U/ml ; par exemple de 2 à $3x10^{-6}$ U/ml, par exemple de $2,57x10^{-6}$ U/ml.

Dans la présente, l'activité de la première enzyme dans le pansement peut être comprise de $10^{-7}$ à $10^{-4}$ unités/g (U/g) de pansement, par exemple de $25x10^{-6}$ à $26x10^{-6}$ U/g de pansement, par exemple de $25,7x10^{-6}$ U/g de pansement.

Dans la présente, la concentration dans la solution enzyme de la seconde enzyme peut être comprise de $2x10^{-7}$ à 50 U/ml, par exemple de 0,1 à 1 U/ml, par exemple de 0,3 à 0,5 U/ml, par exemple de 0,4 U/ml.

Dans la présente, l'activité de la seconde enzyme dans le pansement peut être comprise de 0,5 à 10 Unités/g (U/g) de pansement, par exemple 4 U/g de pansement.

Dans la présente, la concentration de polymère, première et seconde enzyme peut être choisie en accord avec la formule suivante correspondant à une étude de l'équilibre dynamique de la matrice extracellulaire correspondant à un modèle mathématique simplifié d'un système dynamique dans lequel on met en oeuvre deux réactions enzymatiques antagonistes. L'une est catalysée par une enzyme capable d'effectuer des liaisons covalentes entre les monomères solubles (s) pour obtenir un réseau de monomères liés (g). L'autre est catalysé par une enzyme (P) qui hydrolyse le réseau de monomères liés (g) en monomères solubles (s). Enfin, et dans certains cas, une troisième réaction aussi catalysée par l'enzyme (P) consiste en l'hydrolyse des monomères solubles en monomères dégradés (f) de taille trop faible pour participer au réseau ou qui ne peuvent plus servir de substrat à l'enzyme liant les monomères solubles (s) par des liaisons covalentes (T). Cette dernière réaction qui se traduit par une fuite de monomères du cycle est également ajoutée au modèle. Le modèle peut être simplement représenté selon le schéma réactionnel suivant :

<u>Schéma I :</u>

Enzyme dégradant
les chaînes
de monomères liés (P)

Chaînes de monomères liés (g) ⇄ monomères en solution (s) → monomère dégradés (f)

Enzyme liant les monomères par des liaisons covalentes (T)

Ce mécanisme réactionnel peut être simplement décrit par les trois équations différentielles qui suivent, où les réactions enzymatiques sont assimilées à des réactions michaéliennes simples et par une quatrième équation de conservation de la masse.

L'équation différentielle permettant de décrire l'évolution du nombre de chaînes de monomères liés (g) en fonction du temps est la suivante:

$$\frac{dg}{dt} = -\frac{V_p}{K_p + g} \times g + \frac{V_T}{K_T + s} \times s$$

L'équation différentielle permettant de décrire l'évolution du nombre de monomères en solution (s) en fonction du temps est la suivante:

$$\frac{ds}{dt} = \frac{V_p}{K_p + g} \times g - \frac{V_T}{K_T + s} \times s - \frac{V'_P}{K_P + s} \times s$$

L'équation différentielle permettant de décrire l'évolution du nombre de monomères dégradés (f) en fonction du temps est la suivante:

$$\frac{df}{dt} = \frac{V'_P}{K_P + s} \times s$$

Enfin, l'équation de conservation de masse est la suivante:

$$g_t + s_t + f_t = S_0$$

Dans ces équations différentielles, $V_P$ et $V_T$ représentent les vitesses maximales pour les enzymes P et T respectivement, $K_P$ et $K_T$ représentent les constantes de Michaelis pour les enzymes P et T respectivement.

Dans ces équations, $V_P$ et $V_T$ sont calculées selon les équations suivantes :

$$V_P = k_{cat\text{-}P}\,[P] \text{ et } V_T = k_{cat\text{-}T}\,[T]$$

Dans la transposition de cette équation à un système *in vitro,* les différentes valeurs de [P], [T], $V_P$, $V_T$, $K_P$ et $K_T$ correspondent à des constantes.

Dans la présente, la solution tampon des étapes (a) ou (b) peut être une solution tampon choisie dans le groupe comprenant un Tampon TRIS - HCl, un tampon phosphate salin (PBS), Tampon citrate, Tampon borate et/ou toute solution connue de l'homme du métier qui permet le maintien du pH entre 5 et 9 malgré l'addition de petites quantités d'un acide ou d'une base, ou une dilution aqueuse.

Dans la présente, la solution tampon peut être une solution tampon avec un pH de 6,5 à 8.

Dans la présente l'étape de mélange de la solution d'enzymes et de la solution de polymères peut être réalisée par tout procédé connue de l'homme du métier. Par exemple, le mélange peut être réalisé en versant la solution d'enzyme dans la solution de polymères sous agitation, par exemple via un agitateur magnétique ou des pales, par exemple à une vitesse de 1 à 2000 rpm, de préférence de 100 à 250 rpm.

Dans la présente, l'étape de mélange de la solution d'enzyme et de la solution de polymère peut être réalisée à une température de 10 à 50°C, par exemple de 34 à 42 °C.

Dans la présente, à l'étape (c) du procédé, les solutions enzyme et polymère peut être mélangées selon un rapport minimal de 1/5 et soumis à agitation, par exemple avec un agitateur magnétique ou des pales tel que décrit précédemment.

Dans la présente, l'étape de dépôt du mélange de l'étape (c) sur une face d'un non-tissé peut être réalisée par versement dudit mélange. Par exemple, le non tissé peut être disposé sur le fond d'un dans un récipient, par exemple un récipient en acier, en aluminium, en inox, en téflon, en silicone, en PTFE, et tout polymère hydrophobe, puis le mélange est versé dans ledit récipient.

Dans la présente, le volume de mélange de l'étape (c) déposé sur une face du non tissé peut être choisi afin que ledit mélange recouvre l'ensemble du non-tissé.

Dans la présente, le volume de mélange de l'étape (c) déposé sur une face du non tissé peut être compris de 10 à 40 ml/ pansement, de préférence de 30 ml/pansement (pst).

Avantageusement, le volume de mélange déposé permet de recouvrir une face du non-tissé tout en l'imprégnant permettant ainsi l'adhésion du mélange du ladite face. En outre, l'adhésion du mélange sur ledit non-tissé permet avantageusement lors du retrait du pansement de la plaie la conservation du biomatériau sur ledit non tissé, d'éviter qu'il reste du biomatériau sur la plaie après retrait du pansement, et d'empêcher que le biomatériau ne se déchire.

Dans la présente, la réticulation en gel du mélange peut être réalisée par tout procédé connu de l'homme du métier. Par exemple, la réticulation peut être réalisée par chauffage, par exemple à une température comprise de 20 à 50°C, de préférence à une température comprise de 35 à 42°C.

Dans la présente, la réticulation peut être réalisée dans une atmosphère humide avec un pourcentage de 60 à 100 d'humidité. Par exemple, la réticulation peut être réalisée dans un incubateur avec une atmosphère dont le pourcentage d'humidité est compris de 60 à 100 pourcent d'humidité. L'incubateur peut être un incubateur disponible dans le commerce, par exemple un incubateur commercialisé par la société Digitals, la société Binder ou intégré au lyophilsateur.

Dans la présente, la réticulation en gel du mélange peut être effectuée pendant un temps de 1 à 24 heures, par exemple de 1. à 6 heures.

Dans la présente, l'étape de lyophilisation peut être réalisée sous une pression $75x10^{-3}$ Pa à $300x10^{-3}$ Pa, par exemple de $140x10^{-3}$ Pa et comprend outre l'étape de congélation, les étapes suivantes:

g. Etape de sublimation : augmentation de la température de -45 à une température comprise entre 5 et 30 °C, de préférence entre 10 et 20°C, à une vitesse de 0,005 à 0,1°C par minute, par exemple de 0,009 à 0,05°C par minute, par exemple de 0,01 °C par minute,

h. Etape de dessiccation : maintient de la température obtenue à l'étape de sublimation pendant un temps de 10 à 70 heures, de préférence pendant 36 heures

Selon l'invention, la vitesse d'augmentation de la température à l'étape g du procédé convient, par exemple, pour une concentration de 0,2 à 0,5M et/ou de 3 à 4% en poids par rapport au poids total de cryoprotecteur.

Dans la présente, la lyophilisation peut être réalisée par tout dispositif connue de l'homme du métier. Il peut s'agir par exemple d'un lyophilisateur commercialisé par la société Serail, par la société Usifroid, martin Christ, GEA Niro ou Labconco.

Avantageusement, l'étape de lyophilisation permet de lyophiliser le biomatériau tout en conservant ses capacités fonctionnelles, par exemple la possibilité de faire des transitions gel/solution lors de sa mise en contact avec un liquide. En outre, le pansement lyophilisé peut être avantageusement coupé avant application. Cette découpe permet ainsi que le pansement soit adapté le plus justement à la plaie, éléments indispensable à une bonne cicatrisation.

En outre, la lyophilisation du biomatériau permet avantageusement de conserver l'ensemble du biomatériau sur le non-tissé et d'éviter toute perte de biomatériau lors d'un éventuel contact du pansement avec une surface, par exemple une surface sèche. La lyophilisation permet en outre avantageusement d'arrêter les processus de liquéfaction du gel en inhibant l'activité des enzymes. La lyophilisation permet en outre avantageusement de limiter la prolifération bactérienne dans le pansement. La lyophilisation permet en outre avantageusement d'augmenter le temps de conservation du pansement.

La lyophilisation permet également avantageusement la conservation de la structure et de l'intégrité du biomatériau sur le non-tissé avant tout contact avec la plaie. La lyophilisation permet avantageusement de conférer au biomatériau des propriétés absorbantes.

Dans la présente, le procédé peut également comprendre une étape de découpe à façon du pansement.

Dans la présente, l'étape de découpe peut être réalisée par tout dispositif connue de l'homme du métier, par exemple par un massicot, un scalpel, des ciseaux, une presse coupante, un laser.

Dans la présente, le pansement obtenu peut être par exemple un pansement de forme circulaire, un polygone, par exemple un quadrilatère, un pentagone, un hexagone, un heptagone, un rectangle, un carré, un parallélogramme, un triangle, un losange, une ellipse ou toute forme adaptée au lit de la plaie.

Avantageusement, la découpe du pansement permet de produire des pansements dont la forme est adaptée à la plaie.

Dans la présente, le procédé peut également comprendre une étape d'emballage du pansement. Selon l'invention, l'étape d'emballage peut être réalisée par tout procédé connu de l'homme du métier. Par exemple l'emballage peut être réalisé par association sous vide de deux feuillets. L'étape d'emballage peut être réalisée par exemple de manière automatisée, comme par exemple grâce à une scelleuse thermique, par exemple une scelleuse commercialisée par la société multivac.

Selon l'invention, l'emballage peut être un emballage plastique, par exemple avec un film ou feuillet en polyéthylène, un emballage métallique, par exemple en avec des feuillets en aluminium, des emballages résistants aux stérilisations par ionisation, des emballages imperméables aux liquides et/ou aux gaz, par exemple l'Oifol 48 et l'Oifoil 48/Q15 commercialisé par la société Oliver Tolas, Tekniplex, des films aluminium, ou des blisters de polymères.

Avantageusement, l'emballage du pansement permet de protéger et/ou d'isoler ledit pansement de l'environnement extérieur. L'emballage permet avantageusement d'éviter toute contamination extérieure du biomatériau.

Dans la présente, le procédé peut comprendre en outre une étape de stérilisation du pansement.

Dans la présente, l'étape de stérilisation peut être réalisée après l'étape d'emballage.

L'étape de stérilisation du pansement peut être réalisée par exemple par ionisation, par chauffage et/ou par traitement chimique. De préférence, la stérilisation est réalisée par ionisation avec des rayonnements gamma ou béta, de manière plus préféré par rayonnement béta.

Dans la présente, lorsque la stérilisation est réalisée par ionisation, la quantité de rayonnement absorbé est comprise de 0,5 à 50 kGy, de préférence de1 à 27 kGy.

Avantageusement la stérilisation par ionisation permet de stériliser le pansement dans l'emballage tout en conservant les propriétés du biomatériau en particulier la possibilité de transition successive gel/solution, en préservant l'activité des enzymes.

Dans la présente, le procédé peut comprendre en outre une étape d'intégration d'une substance active dans la solution d'enzyme et/ou dans la solution de polymère et/ou dans le mélange obtenu à l'étape (c). La substance active peut être telle que définie précédemment.

D'autres avantages pourront encore apparaître à l'homme du métier à la lecture des exemples ci-dessous, illustrés par les figures annexées, donnés à titre illustratif.

**Brève description des figures**

- La figure 1 représente le schéma de fabrication des pansements
- La figure 2 représente un diagramme des courbes de température du collecteur, des étagères et de l'échantillon en degré Celsius (ordonné) en fonction du temps en heures pendant l'étape de lyophilisation.
- La figure 3 représente des photographies du pansement obtenu en lorsque le biomatériau comprend (avec sorbitol) ou pas (sans sorbitol) du sorbitol.
- La figure 4 représente des photographies de pansements obtenus en fonction de la quantité de sorbitol compris dans le biomatériau.
- La figure 5 représente des photographies de pansements en fonction de la quantité de biomatériau déposé sur le non tissé.
- La figure 6 représente des photographies de fabrication du pansement dans un moule (A) puis démoulage (B).
- La figure 7A représente un diagramme d'un gel physique de gélatine (biomatériau) en fonction du temps en minutes et de la quantité d'ionisation béta en kGy. Sur cette figure la courbe avec les points « ▲» correspondent aux valeurs de G" obtenue pour une ionisation de 0kGy, les points « ■ » correspondent aux valeurs de G' obtenue pour une ionisation de 0kGy, les points « - » correspondent aux valeurs de G' obtenue pour une ionisation de 30kGy, les points « ♦» correspondent aux valeurs de G" obtenue pour une ionisation de 30kGy, les points « × » correspondent aux valeurs de G' obtenue pour une ionisation de 40kGy, les points « * » correspondent aux valeurs de G" obtenue pour une ionisation de 40kGy, les points « ● » correspondent aux valeurs de G' obtenue pour une ionisation de 50kGy, les points « + » correspondent aux valeurs de G' obtenue pour une ionisation de 50kGy,
- La figure 7B représente la variation du G' max (■) (élasticité maximale) et de G" (▲) mesurée en Pascal (Pa) des

gels physiques de gélatine en fonction de la quantité de ionisation béta en kGy.

- La figure 7C représente le temps de gel en minutes en fonction de la quantité d'ionisation béta en kGy.
- La figure 8A représente un diagramme de l'évolution de l'élasticité G', G" d'un gel physique de gélatine en fonction du temps en minutes et de la quantité de rayonnement Gamma en kGy. Sur cette figure la courbe avec les points « ■ » correspondent aux valeurs de G' obtenues pour une ionisation de 0kGy, les points « ♦» correspondent aux valeurs de G" obtenues pour une ionisation de 0kGy, les points « ▲ » correspondent aux valeurs de G' obtenues pour une ionisation de 15 kGy, les points « × » correspondent aux valeurs de G" obtenues pour une ionisation de 15 kGy, les points « - » correspondent aux valeurs de G' obtenues pour une ionisation de 50 kGy, les points « ● » correspondent aux valeurs de G' obtenues pour une ionisation de 50 kGy.
- La figure 8B la variation du G' max (■) (élasticité maximale) et de G" (▲) mesurée en Pascal (Pa) des gels physiques de gélatine en fonction de la quantité de rayonnements Gamma en kGy.
- La figure 8C représente le temps de gel en minutes en fonction de la quantité de rayonnements Gamma en kGy.
- La figure 9A représente un diagramme de l'évolution de l'élasticité G', G" un gel chimique de gélatine en fonction du temps en minutes et de la quantité d'ionisation béta en kGy. Sur cette figure la courbe avec les points « ▲ » correspondent aux valeurs de G" obtenues pour une ionisation de 0kGy, les points « ■ » correspondent aux valeurs de G' obtenues pour une ionisation de 0kGy, les points « ♦ » correspondent aux valeurs de G' obtenues pour une ionisation de 30kGy, les points « × » correspondent aux valeurs de G" obtenues pour une ionisation de 30kGy, les points « - » correspondent aux valeurs de G' obtenues pour une ionisation de 40kGy, les points « + » correspondent aux valeurs de G" obtenues pour une ionisation de 40kGy, les points « × » correspondent aux valeurs de G' obtenues pour une ionisation de 50kGy, les points « + » correspondent aux valeurs de G' obtenues pour une ionisation de 50kGy.
- La figure 9BA représente un diagramme de la variation du G' max (■) (élasticité maximale) et de G" (▲) mesurée en Pascal (Pa) des gels chimiques de gélatine en fonction de la quantité de ionisation béta en kGy.
- La figure 9C représente le temps de gel en minutes en fonction de la quantité d'ionisation béta en kGy.
- La figure 10A représente un diagramme de l'évolution de l'élasticité G', G" un gel chimique de gélatine en fonction du temps en minutes et de la quantité du rayonnement Gamma en kGy. Sur cette figure la courbe avec les points « ▲ » correspondent aux valeurs de G" obtenues pour une ionisation de 0kGy, les points « ■ » correspondent aux valeurs de G' obtenues pour une ionisation de 0kGy, les points « ♦» correspondent aux valeurs de G' obtenues pour une ionisation de 15 kGy, les points « + » correspondent aux valeurs de G" obtenues pour une ionisation de 15 kGy, les points « ● » correspondent aux valeurs de G' obtenues pour une ionisation de 50 kGy, les points « × » correspondent aux valeurs de G' obtenues pour une ionisation de 50 kGy.
- La figure 10BA représente un diagramme de la variation du G' max (■) (élasticité maximale) et de G" (▲) mesurée en Pascal (Pa) des gels chimiques de gélatine en fonction de la quantité du rayonnement Gamma en kGy.
- La figure 10C représente le temps de gel en minutes en fonction de la quantité du rayonnement Gamma en kGy.
- La figure 11A représente un diagramme de l'évolution de l'élasticité G', G" d'un biomatériau de gélatine en fonction du temps en minutes et de la quantité d'ionisation béta en kGy. Sur cette figure la courbe avec les points « ▲ » correspondent aux valeurs de G" obtenues pour une ionisation de 0kGy, les points « ■ » correspondent aux valeurs de G' obtenues pour une ionisation de 0kGy, les points « ♦» correspondent aux valeurs de G' obtenues pour une ionisation de 30kGy, les points « ● » correspondent aux valeurs de G" obtenues pour une ionisation de 30kGy, les points « - » correspondent aux valeurs de G' obtenues pour une ionisation de 40kGy, les points « + » correspondent aux valeurs de G" obtenues pour une ionisation de 40kGy, les points « × » correspondent aux valeurs de G' obtenues pour une ionisation de 50kGy, les points « * » correspondent aux valeurs de G" obtenues pour une ionisation de 50kGy
- La figure 11B la variation du G' max (■) (élasticité maximale) mesurée en Pascal (Pa) des biomatériaux de gélatine en fonction de la quantité de ionisation béta en kGy.
- La figure 11C représente le temps de gel en minutes (■) et le temps de resolubilisation (▲) du gel en fonction de la quantité d'ionisation béta en kGy
- La figure 12A représente un diagramme de l'évolution de l'élasticité G', G" d'un Enzgel de gélatine en fonction du temps en minutes et de la quantité du rayonnement Gamma en kGy. Sur cette figure la courbe avec les points « - » correspondent aux valeurs de G' obtenues pour une ionisation de 0kGy, les points « × » correspondent aux valeurs de G" obtenues pour une ionisation de 0kGy, les points « ♦» correspondent aux valeurs de G' obtenues pour une ionisation de 15 kGy, les points « + » correspondent aux valeurs de G" obtenues pour une ionisation de 15 kGy, les points « ■ » correspondent aux valeurs de G' obtenues pour une ionisation de 50 kGy, les points « ▲ » correspondent aux valeurs de G" obtenues pour une ionisation de 50 kGy.
- La figure 12B représente un diagramme de la variation du G' max (élasticité maximale) des biomatériaux de gélatine en fonction de la quantité du rayonnement Gamma en kGy.
- La figure 12C représente le temps de gel en minutes en fonction de la quantité du rayonnement Gamma en kGy. Sur cette figure Sur cette figure les points « ■ » correspondent aux valeurs de temps de gel et les points « ▲ »

correspondent aux valeurs de temps de fusion du gel.

- La figure 13A représente le temps de resolubilisation du biomatériau en fonction de la quantité d'enzyme (collagénase).
- La figure 13B représente un diagramme représentant la vitesse de chute de G' en pascal par minute en fonction de la quantité d'enzyme, c'est-à-dire la vitesse à laquelle le biomatériau se resolubilise (transition gel/sol) en fonction de la quantité d'enzyme (collagènase [GC]).
- La figure 14 représente des photographies de pansements en fonction du pourcentage de polymère avant lyophilisation (A) et après lyophilisation (B)
- La figure 15 représente des photographies de pansements fonction du pourcentage de polymère après congélation.
- La figure 16 représente des photographies de pansements en fonction du non tissé.
- La figure 17 représente des photographies de pansements en fonction du non tissé.
- La figure 18 est un histogramme représentant la capacité d'absorption du pansement en fonction du non tissé incorporé dans sa matrice. Sur la figure « 1 » correspond aux résultats obtenus avec un non tissé lyofal, « 2 » avec un non tissé MA 50 J, « 3 » avec un non tissé WSV 100, « 4 » avec un non tissé MA 57 S, « 5 » avec un non tissé MA 50 + PE, « 6 » avec un non tissé Tissue 5-Ig comprenant du polyéthylène (Tissue 5-Ig + PE), « 7 » avec un non tissé MASP50 et « 8 » avec un non tissé MASP50 P. Les non tissés sont des non tissés commerciaux commercialisés par la société Mankk.
- La figure 19 est un histogramme représentant le taux de réhydratation du pansement en fonction du non tissé incorporé dans sa matrice. Sur la figure « 1 » correspond aux résultats obtenus avec un non tissé lyofal, « 2 » avec un non tissé MA 60, « 3 » avec un non tissé MA40W4, « 4 » avec un non tissé ZWHO"c" 12-Ig, « 5 » avec un non tissé MA 30, « 6 » avec un non tissé MASP 50 P, « 7 » avec un non tissé MA 71.08. Les non tissés sont des non tissés commerciaux commercialisés par la société Mankk
- La figure 20 est un histogramme représentant la WVTR(water vapor transmission rate): du pansement en fonction du non tissé incorporé dans sa matrice. Sur la figure « 1 » correspond aux résultats obtenus avec un non tissé lyofal, « 2 » avec un non tissé MA 50 J, « 3 » avec un non tissé WSV 100, « 4 » avec un non tissé MA 57 S, « 5 » avec un non tissé MA 50 + PE, « 6 » avec un non tissé MASP 50, « 7 » avec un non tissé MASP 50 P. Les non tissés sont des non tissés commerciaux commercialisés par la société Mankk
- La figure 21 est un diagramme représentant la cinétique de réhydratation des pansements en fonction des différent non tissé testé. Sur cette figure la courbe avec les points « + » correspondent aux valeurs avec le non tissé MA 60, les points « × » correspondent aux valeurs avec le non tissé MA 50J, les points « ♦ » correspondent aux valeurs avec le non tissé ZWHO "C", les points « ● » correspondent aux valeurs avec le non tissé MA 71.080, les points « ■ » correspondent aux valeurs avec le non tissé Baumwell, les points « ▲ » correspondent aux valeurs avec le non tissé MA936, les points « * » correspondent aux valeurs avec le non tissé MA7110.
- La figure 22 est un histogramme représentant la capacité d'absorption des pansements en fonction des différent non tissé testé. Sur la figure « 1 » correspond aux résultats obtenus avec un non tissé ZWHO "C", « 2 » avec un non tissé Baumwell, « 3 » avec un non tissé MA936, « 4 » avec un non tissé MA 50 J, « 5 » avec un non tissé MA 60, « 6 » avec un non tissé MA 71.080 et « 7 » avec un non tissé MA7110. Les non tissés sont des non tissés commerciaux commercialisés par la société Mankk
- La figure 23 représente un diagramme de l'évolution de l'élasticité G', G" en Pa, en fonction de la fréquence d'oscillation du mobile du rhéomètre, en hertz. Sur cette figure la courbe avec les points « □ » correspondent aux valeurs de G" des pansements contenant le non tissé ZWHO"C", les points « ■ » correspondent aux valeurs de G' des pansements contenant le non tissé ZWHO"C", les points « ◊ » correspondent aux valeurs de G" des pansements contenant le non tissé Baumwell-Molten1, les points « ♦ » correspondent aux valeurs de G' des pansements contenant le non tissé Baumwell-Molten 1, les points « ▲ » correspondent aux valeurs de G" des pansements contenant le non tissé Baumwell-Molten 2, les points « ∆ » correspondent aux valeurs de G' des pansements contenant le non tissé Baumwell-Molten 2, les points « o » correspondent aux valeurs de G" des pansements contenant le non tissé MA936-80, les points « ● » correspondent aux valeurs de G' des pansements contenant le non tissé MA936-80, les points « V » correspondent aux valeurs de G" des pansements contenant le non tissé MA71101, les points « ▼ » correspondent aux valeurs de G' des pansements contenant le non tissé MA71101, les points « ◊ » correspondent aux valeurs de G" des pansements contenant le non tissé MA950J1, les points « » correspondent aux valeurs de G" des pansements contenant le non tissé MA950J1.
- La figure 24 représente des photographies en microscopie électronique environnementale de pansement lyophilisé en fonction de son hydratation. La figure 24A représente une photographie en microscopie électronique d'un pansement lyophilisé sec. La figure 24B représente une photographie en microscopie électronique d'un pansement lyophilisé après hydratation pendant 4 minutes. La figure 24C représente une photographie en microscopie électronique d'un pansement lyophilisé après hydratation pendant 20 minutes. La figure 24D représente une photographie en microscopie électronique d'un pansement lyophilisé après hydratation maximum, c'est-à-dire pendant plus de 12 heures.

- - La figure 25 représente un diagramme de l'évolution de l'élasticité G', G" en PA d'un pansement réhydraté, en fonction du temps en heures. Sur cette figure la courbe avec les points « ▲ » correspondent aux valeurs de G", les points « ■ » correspondent aux valeurs de G'.
- La figure 26 représente un diagramme en bâton de la force normale intégrée (iNF) nécessaire à l'arrachage du pansement au rhéomètre en fonction de leur élasticité. Sur cette figure « 1 » correspond aux résultats obtenus avec un pansement avec un G' égal à 35 Pascal (Pa), « 2 » correspond aux résultats obtenus avec un pansement avec un G' égal à 18 Pa, « 3 » correspond aux résultats obtenus avec un pansement avec un G' égal à 3.
- La figure 27 représente un diagramme de la capacité d'absorption en gramme d'eau par 100 $cm^2$ du pansement en fonction du temps en heure. Sur cette figure « ■ » correspond aux résultats obtenus pour le pansement lyophilisé comprenant le non-tissé, « ▲ » correspond aux résultats obtenus avec le gel seul non lyophilisé.
- La figure 28 représente des photographies de plaies rachidiennes dermo épidermiques de 5x5 cm de longueur sur 0,5 cm de profondeur. La figure 28A est une photographie de la plaie au premier jour avant l'application du pansement de l'invention, la figure 28B est une photographie de la plaie après 7 jours d'application du pansement de l'invention, la figure 28C est une photographie de la plaie après 18 jours d'application du pansement de l'invention, la figure 28D est une photographie de la plaie après 30 jours d'application du pansement de l'invention.
- La figure 29 représente un diagramme de production d'exsudat par une plaie ainsi que sa rétention par le pansement de l'invention. Sur cette figure l'abscisse représente le temps en jour et l'ordonné représente le score estimé par l'opérateur. En outre, les points « ■ » correspondent au score estimé de rétention des exsudats par le pansement et les points « ◊ » correspondent au score estimé de production d'exsudats par la plaie.
- La figure 30 représente des photographies de coupes histologiques de plaies cicatrisées après application du pansement de l'invention. La figure 30 A représente une photographie de la plaie cicatrisée à faible magnitude et la figure 30B une photographie de la plaie cicatrisée à forte magnitude.

## EXEMPLES

### Exemple 1 : procédé de fabrication

a) Préparation des mélanges

Pour la préparation des mélanges deux cuves ont été utilisées. Une cuve CT1 pour le mélange de polymère et une cuve CT2 pour le mélange d'enzyme. La cuve CT1 utilisée est une cuve de 150 litres, la cuve CT2 est une cuve de 65 litres.

La gélatine utilisée provenait de la société Rouselot commercialisé sous la référence LB8, le sorbitol de la société IMCD commercialisé sous la référence sorbitol apyrogène PF.

Le tampon utilisé est un tampon comprenant de l'eau distillé, du TRIS 50 mM et de l'acide chloridrique en quantité suffisante pour obtenir un pH de 7,75.

Le mélange de polymère a été réalisé sous agitation à 187 tours par minutes (« round per minutes (rpm) ») dans une cuve CT1 à 40°C. Dans un volume de 35 l d'eau sanitisée ont été ajouté : 211g de solution tampon, 2 kg de polymère de gélatine et 3 kg de sorbitol. La concentration finale de polymère dans le mélange CT1 est alors de 57 g/l.et celle du sorbitol est de 86 g/l.

Le mélange d'enzyme a été réalisé sous agitation à 187 rpm avec un agitateur dans la cuve CT2 à 40°C. Dans un volume de 35 litres d'eau sanitisée ont été ajouté : 211g de tampon, 0,22 Unités de la première enzyme et 30 Unités de la seconde.

Dans cet exemple, la première enzyme était la collagénase provenant de la société Coger commercialisé sous la référence NB6, la seconde enzyme était la Transglutaminase provenant de la société Ajinomoto commercialisé sous la référence Activa WM.

Le contenu de la cuve CT2 obtenue a été ensuite transféré dans la cuve CT1 et mélangé sous agitation à 187 tours par minutes pendant au moins 5 minutes.

b) Gélification

La totalité du mélange contenu dans les cuves CT1 et CT2 comprenant les enzymes et le polymère obtenu a été ensuite déposé par un débimètre sur un non-tissé préalablement découpé sous une forme adaptée à la taille du moule.

Le moule utilisé était en aluminium, ou en silicone de forme rectangulaire (500x1000x140 mm) commercialisé par la société MAE et Sérail.

Le moule a été ensuite déposé dans une étuve à 35°C et à au moins 60% d'humidité. Le moule est resté dans l'étuve pendant un temps compris de 1 h à 10h.

c) Lyophilisation

Après dépôt, le non-tissé sur lequel le mélange a été déposé a été disposé dans un lyophilisateur commercialisé par la société Serail.
Le cycle de température appliqué ainsi que la pression sous laquelle a été réalisée la lyophilisation sont indiquées dans le tableau 1 ci-dessous.

Tableau 1 : cycle de température et de pressions appliquées pour la lyophilisation

| Etape | Température | Rampe de température (vitesse d'augmentation de la température) | Temps du palier | Pression |
|---|---|---|---|---|
| 1 | -45°C | - | 3H | - |
| 2 | -45 à 10°C | 0,1°C/min | | 140μBar |
| 3 | +10°C | - | 33H | 140μBar |
| La figure 1 représente la courbe de température de lyophilisation en degré Celsius (ordonné) en fonction du temps en heures. | | | | |

d) Conditionnement

Après lyophilisation, le non-tissé a été découpé et la face non tissé a été marquée à l'aide d'une presse coupante a une taille de 65 *100 mm
Le pansement ainsi formé a été alors ensaché. Le sachet a été ensuite soudé pendant 4 secondes sous un vide de 50 μbar, grâce à une scelleuse de modèle C200N commercialisée par la société multivac.
La figure 1 représente un schéma général de fabrication.

**Exemple 2 : pansement dans lequel le biomatériau comprend du sorbitol**

Dans cet exemple l'effet du sorbitol sur le pansement à été étudié.
Les pansements ont été préparés selon le procédé décrit dans l'exemple 1 dans lequel la gélatine est 3% (30mg/mL), et la concentration finale de Sorbitol est de 0,2 à 0,1 M, de la Transglutaminase à 4mg/mL, soit 12 U/pansement, et la collagénase à 2,2x$10^{-5}$mg/mL soit 8,5 x$10^{-5}$ U/pansement.
Les produits obtenus dans cet exemple ne comprenaient pas de support non-tissé.
La nature et les concentrations de la gélatine, des enzymes et de la solution tampon utilisées dans cet exemple étaient identiques à ceux de l'exemple 1
Dans cet exemple le biomatériau est également désigné par biogel.

RESOLUBILISATION ET MELANGE DES PRODUITS

Le tampon a été placé au préalable à température ambiante, c'est à dire entre 15 et 30°C. Les trois mélanges ont été effectués en parallèle.

- Dans le mélange (« Batch ») 1 (40°C) :

    ◦ la poudre de gélatine (30 mg.$l^{-1}$ final) et la poudre de sorbitol (0,2M à 1 M) ont été resuspendue dans un volume total de 500mL de solution tampon.
    ◦ Une resolubilisation a été effectué sous agitation mécanique avec un barreau magnétique pendant 30min (maximum 10h) à 40°C jusqu'à ce qu'il n'y ait plus de grain de gélatine en suspension. L'agitation a été adaptée, pour éviter la formation de mousse, tout en assurant un mélange homogène.

- Dans le mélange (« Batch ») 2 (40°C) : la poudre de transglutaminase (4mg.$l^{-1}$) a été ajoutée dans 480 mL de solution tampon et solubilisée sous agitation mécanique à 40°C pendant 30min (maximum 10h).
- Dans le mélange (« Batch ») 3 (40°C) : 17mL de collagénase diluée dans du tampon ont été ajoutés, afin d'atteindre une concentration finale de 2,2x$10^{-5}$ mg.$ml^{-1}$, et agités à 40°C pendant 15min (maximum 1 heures).

FORMATION DU BIOGEL/BIOMATERIAU

Toute la solution de collagénase (Batch 3) a été ajoutée dans le mélange (« batch ») 2 contenant la solution de transglutaminase et mélangé pendant 3 min au maximum sous agitation magnétique.

Le contenu du mélange («batch») 2 a été versé dans le mélange («batch») 1, contenant la solution de gélatine/sorbitol.

20 à 40mL de solution de mélange (gélatine/sorbitol + enzymes) ont été versés dans un moule hydrophobe en silicone ou aluminium contenant une compresse commercialisé par Mankk.

Les moules ont été mis à 35°C dans une étuve sous atmosphère humide ($rH_{min}$ = 80%) pendant 3 heures minimum (maximum = 8h*).

LYOPHILISATION

Le lyophilisateur utilisé était le modèle Triad commercialisé par la société Labconco et a été pré-congeler à une température de -45°C. La vitesse de congélation des gels n'a pas d'incidence sur le produit. Les moules contenant le « biogel » ont été déposés dans le lyophilisateur et le cycle de lyophilisation indiqué dans le tableau 2 a été appliqué.

Tableau 2 : cycle de température et de pressions appliquées pour la lyophilisation

| Etape | Température | Rampe de température | Temps du palier | Pression |
|---|---|---|---|---|
| 1 | -77°C | - | 3 heures | - |
| 2 | -55°à 10 à 30°C | 0,17°C/min | | 140µBar |
| 3 | +10à 30°C | 0,05°C/min | 2 à 5heures | 140µBar |

Résultats

Les photographies des biomatériaux lyophilisés obtenues correspondent aux figures 3 à 6.

Comme montré dans la figure 3, la présence du sorbitol dans le biomatériau a un effet sur son aspect.

En particulier, comme représenté sur la figure 3, l'ajout de sorbitol à une concentration de 0,5M a permis d'amener/d'augmenter la souplesse au pansement. Toutefois, une hétérogénéité de surface et un aspect collant sont apparus.

En outre, comme le montre la figure 4, la concentration minimale de sorbitol, en dessous de laquelle le pansement présente une rigidité trop importante est de 0,2M. La concentration maximale au dessus de laquelle l'hétérogénéité de surface et l'aspect collant sont délétère pour le pansement était de 0,5M.

Les résultats montrent également, comme représenté sur la figure 5 que le dépôt d'un volume de 30 ml de gel, quelque soit la concentration en sorbitol, rend le produit obtenu plus homogène.

Les résultats montrent également, comme représenté sur la figure 6 que le moule en silicone permet d'éviter le phénomène d'adhérence au moule, mais les pansements ainsi produit sont moins déshydraté.

Enfin, l'effet des paramètres de lyophilisation appliqués à la troisième étape, la température et la durée, ont été étudiés et les résultats obtenus sont indiqués dans le tableau 3 ci-dessous.

Tableau 3 : caractéristique du biomatériau lyophilisé en fonction des paramètres de l'étape 3 de lyophilisation et du moule

| Conditions | Lyophilisation faible | Lyophilisation intermédiaire | Lyophilisation intermédiaire | Lyophilisation forte |
|---|---|---|---|---|
| Température de dessiccation | 10°C | 30°C | 10°c | 30°C |
| Temps de dessiccation | 2 heures | 2 heures | 5 heures | 5 heures |
| Aspect des pansements (moule en aluminium) | Biomatériau humide | Biomatériau humide | Biomatériau lyophilisé | Biomatériau peu humide |
| Aspect des pansements (Moule en silicone) | - | Biomatériau très humide | Biomatériau lyophilisé | Biomatériau rigide |

**Conclusions:**

Le pansement obtenu présentait un aspect blanc homogène et se démoulait facilement après lyophilisation mais présentait une rigidité affectant sa conformabilité. L'ajout de sorbitol dans le mélange de gélatine a permis d'obtenir des pansements dont la souplesse, plus grande, permet une utilisation optimum. Par contre, il possédait une hétérogénéité de surface (partie lyophilisée et partie fusionnée) et colle très fortement au moule PVC.

L'ajout de différentes concentrations de sorbitol dans le mélange ont permit d'établir une concentration d'utilisation, en dessous de laquelle le pansement est rigide et cassant. Cette concentration correspond à 0,2 M de sorbitol.

Un volume de dépôt de 30mL a permis d'éviter les hétérogénéités de surface que présentaient, de façon aléatoire, les pansements de 20mL, et cela quelle que soit la quantité de sorbitol utilisée.

Les moules en silicone ont permis d'éviter les phénomènes d'adhérence du pansement observé avec le moule en PVC. Ainsi le démoulage des pansements à partir de moule siliconé semble plus réalisable.

Plusieurs cycles de lyophilisation ont été testés. Celui constitué d'une phase de lyophilisation à 10°C pendant 5 heures a permis d'obtenir des pansements lyophilisés et souples.

**Exemple 3: étude de l'effet de la stérilisation sur les propriétés du pansement.**

Des échantillons de gélatine en poudre, de transglutaminase en poudre et de collagénase en poudre ont été stérilisés par ionisation béta ou rayonnement gamma. Plusieurs doses pour chaque type de rayonnement ont été testées : 30, 40 ou 50 kGy.

Pour ce faire, le dispositif utilisé était un rayonnement de type béta réalisé par la société Ionisos.

Les échantillons de gélatine en poudre, de transglutaminase en poudre et de collagénase en poudre provenaient respectivement de la société Rousselot, Ajinomoto et Coger, commercialisés respectivement sous les références catalogues Gélatine LB8 pharma, transglutaminase Activa WM et collagénase NB4

I. Influence de la stérilisation sur les caractéristiques de gélification physique de la gélatine

L'effet de la stérilisation sur la gélification physique (baisse de T°C de 40°C à 27°C) de la gélatine a été observé.

Des gels on ensuite été réalisés selon le protocole suivant :

Préparation de la gélatine :

150mg de gélatine a été pesés pour chaque condition de stérilisation, placés les dans le pilulier et 4mL de tampon Tris a été ajouté.

Un barreau aimanté a été ajouté et le pilulier a été place dans un bain-marie préchauffé à 40°C. La gélatine stérilisée a été resolubilisée sous agitation à 40°C pendant 30min.

2ml de solution a été déposée sur le rhéomètre, la gélification a été observé sous oscillation à 1 Hz, avec une rampe de température de 40°C à 27°C à une vitesse de 0,5°C.min$^{-1}$.

Les mesures des propriétés physiques de gélification ont été réalisées sur un rhéomètre Anton-Paar MR301

La figure 7A représente un diagramme un gel physique de gélatine (biomatériau) en fonction du temps en minutes et de la quantité d'ionisation béta en kGy. Comme représenté sur la figure 7A, l'ionisation avec des rayons beta entraine en fonction de la dose une dégradation du gel de gélatine.

La figure 7B représente la variation du G' max (élasticité maximale) mesurée en Pascal (Pa) des gels physiques de gélatine en fonction de la quantité d'ionisation béta en kGy. Comme représenté sur la figure 7B, cette dégradation est linéaire avec la dose de rayons béta et n'influence que la partie élastique du réseau (G').

La figure 7C représente le temps de gel en minutes en fonction de la quantité d'ionisation béta en kGy. Comme représenté sur la figures 7C, le temps de gel augmente linéairement avec la dose de rayonnement. Ainsi on a constaté que la dégradation de la gélatine est linéaire avec la dose de rayonnement béta.

La figure 8A représente un diagramme de l'évolution de l'élasticité G', G'' d'un gel physique de gélatine en fonction du temps en minutes et de la quantité de rayonnement Gamma en kGy. La figure 8B la variation du G' max (élasticité maximale) des gels physique de gélatine en fonction de la quantité de rayonnements Gamma en kGy. La figure 8C représente le temps de gel en minutes en fonction de la quantité de rayonnements Gamma en kGy.

Comme représenté sur les figures 8A, B, C, l'irradiation avec des rayons gamma entraine en fonction de la dose une dégradation de la gélatine. Cette dégradation n'est pas linéaire en fonction de la dose de rayon gamma. En particulier, un seuil de dégradation important à lieu au dessus de 15 kGy .

II. influence de la stérilisation sur les caractéristiques de la transglutaminase

L'effet de la stérilisation sur la gélification chimique, via l'ajout de 3 u/ml de transglutaminase stérilisée à 40°C, de la gélatine non stérilisée a été observé. Pour ce faire de la poudre de transglutaminase à été soumis à différent protocole de stérilisation : sous irradiation gamma et sous ionisation béta, avec pour chacune des conditions, trois doses testées : 0, 30 et 50 kGy.

Des gels ont été ensuite été réalisés selon le protocole suivant :

Préparation de la gélatine :

150 mg de gélatine non stérile ont été pesés, placés dans un pilulier et 4mL de tampon Tris a été ajouté.
Un barreau aimanté a été ajouté et le pilulier a été placé dans un bain-marie préchauffé à 40°C.
La gélatine a été resolubilisée sous agitation à 40°C pendant 30min.

Préparation de la solution de transglutaminase (a répéter pour chaque condition):

225 mg de transglutaminase (TGase) stérile ont été pesés et placés dans un microtube
1,5mL de tampon Tris ont été ajoutés à l'aide de la micropipette et agités pour resolubiliser la poudre sans faire de mousse (la solution pouvait contenir de petite masse non soluble).

Préparation du mélange :

1mL de TGase a été ajouté dans les 4ml de gélatine sous agitation pendant 1 min.
2ml de la solution obtenu ont été déposés sur un rhéomètre, la gélification a été observée à 40°C, sous oscillation à une fréquence de 1 Hz.

Les mesures des propriétés physiques de gélification ont été réalisées sur un rhéomètre Anton-Paar MR301

Comme représenté sur les figures 9, A, B, C, la ionisation avec des rayons beta entraine en fonction de la dose une diminution de l'activité de la transglutaminase (TG) et donc une diminution des valeurs de G' et G". Cette diminution est linéaire avec la dose de rayons béta comme représentée sur les figures 9B et C.

Comme représenté sur les figures 10, A, B, C, l'irradiation avec des rayons gamma entraine en fonction de la dose une diminution de l'activité de la transglutaminase (TG) et donc une diminution des valeurs de G' et G". Cette diminution n'est pas linéaire avec la dose de rayons gamma comme représentée sur les figures 9B et C.

III. influence sur les caractéristiques de la collagénase

L'effet de la stérilisation sur double transition de la gélatine sol-gel, gel-sol induite par la présence de transglutaminase (TG) et de collagénase (CG) à 40°C a été observé.

Pour ce faire, de la poudre de collagènase à été soumis à différents protocole de stérilisation : sous irradiation gamma et sous ionisation béta, avec pour chacune des conditions, trois doses testées : 0, 30 et 50 kGy. Des gels ont ensuite été réalisés avec cette poudre et les autres constituants non traité selon le procédé décrit ensuite :

Préparation du Mélange A:

300 mg de gélatine non stérile ont été solubilisés avec 5 mL de tampon TRIS non stérile, puis ont été mis sous agitation 30 min. à 40°C

Préparation du Mélange B:

40 mg de Transglutaminase non stérile ont été solubilisés avec 5 mL de tampon TRIS non stérile. puis ont été mis sous agitation 30 min. à 40°C
Mélange C: 22 $\mu$L de collagènase à 0.01 mg/mL conservés 30 min à 40°C
Le Mélange C a été mis dans le Mélange B puis dans le Mélange A et laissés agiter 2 min. à 40°C
2ml de la solution finale ont été déposés sur le rhéomètre, l'évolution des propriétés physiques ont été observés à 40°C, sous oscillation à 1 Hz.

Les mesures des propriétés physiques ont été réalisées sur un rhéomètre Anton-Paar MR301

Comme représenté sur les figures 11A, B, C, l'ionisation beta entraine en fonction de la dose une diminution

de l'activité de la collagènase montré par la figure 11A, où l'on observe qu'au dessus de 40 kGy, le G' met environ 2,5 fois plus de temps que les autres conditions, à atteindre une valeur de 1 Pa. Ainsi, la valeur de 40 kGy a été considérée comme une dose seuil supérieure à partir de laquelle l'activité de la collagénase (CG) est affectée par les rayons béta.

Comme représenté sur les figures 12A, B, C, l'irradiation gamma entraine en fonction de la dose une diminution de l'activité de la collagénase (CG) montré par la figure 12A, où l'on observe qu'au dessus de 15 kGy, le G' met environ 2,5 fois plus de temps que les autres conditions, à atteindre une valeur de 1 Pa. La valeur de 15 kGy a été considérée comme une dose seuil supérieure à partir de laquelle l'activité de la collagénase (CG) est affectée par les rayons gamma.

IV- <u>Détermination de courbes références pour la détermination de l'activité de la collagénase</u>

Une étude de l'importance de la concentration de la collagénase, dans le présent exemple la collagénase NB4, sur les caractéristiques des biomatériaux a été effectuée.

Pour ce faire des biomatériaux, également désignés enzgels ont été réalisés avec différentes concentrations de collagènase : 0,8 $x10^{-4}$ $U.ml^{-1}$, 1 $x10^{-4}$ $U.ml^{-1}$, 1,4 $x10^{-4}$ $U.ml^{-1}$ ou 1,8 $x10^{-4}$ $U.ml^{-1}$ selon le procédé décrit ci-dessous :

Préparation du Mélange A:

300 mg de gélatine non stérile ont été solubilisés avec 5 mL de tampon TRIS non stérile. Puis l'ensemble a été mis sous agitation 30 min. à 40°C

Préparation du Mélange B:

40 mg de Transglutaminase non stérile ont été solubilisés avec 5 mL de tampon TRIS non stérile. Puis l'ensemble a été mis sous agitation 30 min. à 40°C

Mélange C: 22 μL de différente concentration de collagènase conservés 30 min à 40°C

Le Mélange C a été mis dans le Mélange B puis dans le Mélange A et laisser agiter 2 min. à 40°C

2ml de la solution finale ont été déposés sur le rhéomètre, l'évolution des propriétés physiques a été observée à 40°C, sous oscillation à 1 Hz.

Les mesures des propriétés physiques ont été réalisées sur un rhéomètre Anton-Paar MR301

Les résultats obtenus sont représentés sur la figure 13. La figure 13 A représente le temps de resolubilisation du biomatériau en fonction de la quantité d'enzyme (collagénase [CG]). La figure 13B représente la vitesse à laquelle le biomatériau se resolubilise (transition gel/sol) en fonction de la quantité d'enzyme (collagènase [CG]).

Comme représenté sur ces figures, le temps de resolubilisation et la vitesse de chute du G' sont linéaires. Il est donc aisé d'extrapoler une vitesse de resolubilisation en fonction d'une concentration de collagénase donnée.

Ces courbes servent de référence pour calculer la concentration d'enzyme nécessaire à la fonte/solubilisation du pansement.

V <u>Test microbiologique</u>

Une analyse du développement bactérien des pansements traités par ionisation béta à 25 kGy a été réalisée.

Pour ce faire, 3 lots différents de pansement ont été testés selon le protocole suivant :

Ensemencer 10 pansements dans un flacon de bouillon à l'hysrolysat de caséine et de soja et 10 pansements dans un flacon de bouillon au thioglycolate et résazurine.

Incuber 14 jours à 30-35°C le bouillon au thioglycolate et résazurine et à 25-25°C le bouillon à l'hydolysat de caséine et de soja.

Une observation visuelle a été réalisée toutes les 24 heures pendant 14 jours.

Suivant ce protocole, aucun des lots de pansement n'a dévelloppé de colonie, montrant ainsi que dès 25 kGy les pansements de l'inventio sont stérils.

Le même test à été réalisé sur des pansements ayant subi une irradiation gamma à 28 kGy. Tous les pansements traités se sont solubilisés dans les bouillons rendant impossible l'analyse.

Les rayons gamma détruisent le pansement dès 28 kGy.

**VII Lyophilisation**

Différents pansements ont été fabriqués selon le protocole de l'exemple 1 avec des concentrations de gélatine

comprise entre 2 et 5% masse/volume (m/v) et des concentrations de seconde enzyme, la transglutaminase (TG) de 3 à 7 U/ml.

Différents cycles de lyophilisation ont été testés et varis en fonction des essais et sont décrit pour chaque résultat. Les pansements obtenus ont été photographiés et sont représentés sur les figures 14 et 15.

Dans un premier temps des pansements comprenant respectivement 1, 2, 3 ou 5%, 3U/mL de TG et un non tissé, à savoir le Lyophal, ont été incubés pendant 3 heures à 35°C et 80 % d'humidité, puis lyophilisé.

Le cycle de lyophilisation était le suivant :

Pression : 150 μbar
Pré-Congélation à -70°C pendant 3heures
Chauffage jusqu'à -55°C à une vitesse de 0,13°C/min et maintient pendant 150 min,
Chauffage jusqu'à 0°C à une vitesse de 0,073°C/min et maintient pendant 180min,
Chauffage à 30°C à une vitesse de 0,2°C/min et maintient pendant 1140min

La figure 14A représente les pansements obtenus après l'étape de congélation en fonction du pourcentage de gélatine.

La figure 14B représente les pansements obtenus après lyophilisation en fonction du pourcentage de gélatine.

Comme représenté sur ces figures on observe que tous les pansements se sont bien congelés, et se sont bien lyophilisés, même si les pansements dont le pourcentage de gélatine est inférieur à 2% (m/v) présente de nombreux débris.

En outre, l'efficacité de réhydrations des pansements lyophilisés a été testé en en plongeant pendant 4 heures chaque pansement dans une quantité importante (> 50 ml) de tampon Tris pH 7,4, à 35°C.

Lors de cette étape, les pansements comprenant 1 et 2% de gélatine se sont solubilisé, car la concentration faible en gélatine ne permet pas la formation de gel. Les pansement avec 3 et 5% de gélatine se sont réhydratés et ne présentaient aucune dégradation, aucun débris.

Dans un second temps des pansements comprenant respectivement 1, 2, 3 ou 5% (m/v) de gélatine, 3U/mL de TG et un non tissé (Lyofal), ont été incubés pendant à 35°C et 80 % d'humidité, puis lyophilisé comme suit :

Pré-Congélation à -70°C pendant 3heures
Chauffage jusqu'à -55°C à une vitesse de 0,13°C/min et maintient pendant 150min,
Chauffage jusqu'à 0°C à une vitesse de 0,073°C/min et maintient pendant 180min,
Chauffage à 30°C à une vitesse de 0,2°C/min et maintient pendant 1140 min

La figure 15 représente les pansements obtenus après l'étape de congélation en fonction du pourcentage de gélatine.

Après lyophilisation, l'aspect des pansements était similaire à celui obtenu après l'étape de congélation.

Comme représenté sur ces figures il a été observe que tous les pansements se sont bien congelé, et se sont bien lyophilisés.

En outre, l'efficacité de réhydrations des pansements lyophilisés a été testée en en plongeant pendant 4 heures chaque pansement dans une quantité importante, c'est-à-dire supérieur à 50 ml, de tampon Tris pH 7,4, à 35°C.

Lors de cette étape, les pansements obtenus étaient comme suit :

1 % de gélatine : resolubilisation totale.
2% de gélatine : resolubilisation incomplète.
3% de gélatine : Formation d'un beau gel un peu craquelé, sans débris.
5% de gélatine : Formation d'un gel très rigide, craquelé mais sans débris.

<u>Conclusions</u>

L'effet des traitements stérilisants (rayons béta et gamma), sur les constituants des pansements, ont été évalués en rhéologie.

Pour la gélatine, une dégradation protéique ayant pour conséquence une chute de son pouvoir gélifiant (de type chimique) a été constatée. La dégradation se retrouve avec les rayonnements béta et gamma. Elle semble plus importante avec les gamma. La dégradation est linéaire avec la dose d'ionisation béta mais hétérogène au niveau de la poudre au vu de la reproductibilité des expériences.

La transglutaminase a été elle aussi affectée par un traitement aux rayons (béta ou gamma). Une chute de l'activité enzymatique (et donc à une gélification moins rapide) pour les deux types de stérilisation mais de manière plus importante avec les rayons gamma a été observé. La relation entre l'augmentation du temps de gel et la dose stérilisante

est linéaire, mais une relation exponentielle inverse relie la perte d'activité et cette dose stérilisante.

L'activité de la collagénase a été aussi affectée par les rayonnements mais il semble exister, aussi bien pour les gamma que les béta, une dose critique avant laquelle l'activité protéolytique n'est pas modifiée. Comme pour les deux autres constituants, les gamma sont plus destructeurs que les béta.

Les expériences effectuées en microbiologie, sur les pansements stérilisés avec les rayons gamma ont montré que les pansement se dégradent sous leur action.

L'ionisation béta à 25 kGy, a permis leur stérilisation

Les tests de lyophilisation ont apporté plusieurs conclusions :

- la concentration de gélatine joue sur la rigidité des pansements lyophilisés.
- une congélation instantanée des gels dans le lyophilisateur ne semble pas permettre une amélioration de la souplesse des pansements.
- le non tissé évite un craquelage trop important des gels.

Tel que démontré dans cet exemple, la concentration de gélatine est importante pour l'obtention d'un gel fonctionnel et présentant les propriétés requises.

En outre, le procédé de lyophilisation et sa mise en oeuvre est également importante pour l'obtention d'un gel fonctionnel.

**Exemple 4 : effet du non tissé**

But de l'essai / exemple:

L'essai a pour but de tester des non tissés de la société Manck, en fonction de leur capacité d'adhérence au gel, après et avant lyophilisation, et aux caractéristiques d'absorption et de WVTR (water vapor transmission rate) que confèrent les non tissés aux pansements.

Descriptif de l'essai/ exemple :

Différents pansements ont été obtenus en lyophilisation et contenant chacun un non tissé propre.

Types de non tissé testé :

- Lyofal : la compresse est fournie par la société Lyofal et commercialisé par la société Norafin qui fera office de référence sous la référence catalogue 71.100.001
- MA 50 J commercialisé par la société Mankk sous cette référence catalogue.
- WSV 100 commercialisé par la société Mankk sous cette référence catalogue.
- MA57 S commercialisé par la société Mankk sous cette référence catalogue.
- MA50 + PE commercialisé par la société Mankk sous cette référence catalogue.
- Tissue 5-lg + PE commercialisé par la société Mankk sous cette référence catalogue.
- MASP 50 commercialisé par la société Mankk sous cette référence catalogue.
- MASP 50 P commercialisé par la société Mankk sous cette référence catalogue.
- MAPPR 20 commercialisé par la société Mankk sous cette référence catalogue.
- MA30 commercialisé par la société Mankk sous cette référence catalogue.
- MA60 commercialisé par la société Mankk sous cette référence catalogue.
- MA40 W4 commercialisé par la société Mankk sous cette référence catalogue.
- MA 71.08 commercialisé par la société Mankk sous cette référence catalogue.
- ZWHO "c" 12-lg commercialisé par la société Mankk sous cette référence catalogue.

Quantités utilisées :

- Gélatine (Bloom 250) 3% commercialisé par la société Rousselot sous la référence catalogue 250 LB8
- Transglutaminase (TG) 15mg/mL commercialisé par la société Ajinomoto sous la référence catalogue Activa WM.
- Volume du gel : 20mL

Les pansements ont été obtenus selon le procédé décrit dans l'exemple 1 avec différent non tissé au fond du moule.

Une fois lyophilisé, les pansements ont été découpés en quatre afin d'effectuer des tests de réhydratation et d'absorption avec comme absorbat une solution d'eau distillée contenant 8,3g/L de NaCl et 0,368g/L de $CaCl_2$.

Le test d'absorption à été réaliser comme suit :

Le matériel utilisé était ; des boîtes de Pétri 90+/-5 mm de diamètre, un four de laboratoire, à circulation d'air forcée, pouvant maintenir une température de (37+/-1 °C), une solution A : 8,298 g de chlorure de sodium (NaCl) et 0,368 g de chlorure de calcium dihydrate ($CaCl_2$, $2H_2O$) dans de l'eau désionisée et complétée jusqu'à constituer 1 litre de solution dans un flacon volumétrique et une balance, capable de peser 100 g à 0,0001 g près.

Protocole

Un seul échantillon de 5x5cm ou de 0,2g de pansement a été placé dans une boîte de Pétri. une quantité de solution A 40x supérieure au poids du pansement préchauffée à 37 °C a été placée et l'échantillon a été incubé 30min dans un four, à circulation d'air forcée, chauffé à 37°C. L'échantillon a été pesé sur la balance après avoir suspendu l'échantillon à l'aide d'une pince.

Chaque test suivant A été effectués sur neuf échantillons différents.

La capacité d'absorption a été exprimée comme étant la masse moyenne de solution retenue par 100 cm2. Le taux s'exprime par gramme d'échantillon.

Des tests de WVTR ont été effectués sur des pansements contenant les non tissés suivant :

- Lyofal
- MA50 J
- WSV 100
- MA57 S
- MA50 + PE
- MASP 50
- MASP 50 P

Le test de WVTR à été réaliser comme suit :

Matériels utilisés

Cinq cylindres propres et secs en matériau inoxydable de 42+/-0,1 mm de diamètre intérieur équipés d'une bride à l'extrémité basse et pouvant contenir chacun 40 ml de solution d'essai. Un four ou incubateur, équipé d'un ventilateur brasseur d'air, pouvant maintenir une température de (37+/-1) °C et conçu pour répartir l'air uniformément dans le four ou l'incubateur de façon à maintenir une humidité relative inférieure à 20 % au cours de l'essai. Solution d'essai A comme décrit en début d'exemple, Eprouvette étalonnée, Hygromètre, pouvant détecter si la limite de 20 % d'humidité relative a été dépassée ou non, des élastiques une Balance, capable de peser 100 g à 0,0001 g près.

Protocole

Un échantillon circulaire a été découpé et apposé le sur la bride supérieure d'un cylindre, la surface de contact avec la plaie tournée vers l'intérieur. Un élastique a été également apposé afin de maintenir l'échantillon

L'échantillon, a été pesé, la valeur obtenue a été référencée comme W1. L'échantillon et l'élastique ont été ensuite retirés du cylindre.

40 ml de solution A ont été ajoutés dans le cylindre et l'échantillon circulaire a été à nouveau apposé sur la bride supérieure du cylindre et fixé par un élastique. Le cylindre a été alors pesé une seconde fois. La valeur obtenue a été référencée comme W2.

Ce procédé a été répété quatre fois afin de préparer en tout cinq échantillons.

Les cylindres obtenus ont été ensuite déposés dans un incubateur à 37°C pendant 24 heures. Ils ont été ensuite retirés de l'incubateur et laissés à température ambiante pendant 30 minutes et pesées une troisième fois. La valeur obtenue a été référencée comme W3.

L'échantillon a été ensuite retiré du cylindre, l'excès de liquide contenu dans le cylindre a été éliminé puis l'échantillon replacé sur le cylindre.

Les cylindres obtenus ont été pesés une nouvelle fois. La valeur obtenue a été référencée comme W4.

Les étapes précitées ont été reproduites avec un temps d'incubation de 48 heures dans l'incubateur à 37 °C.

La capacité de rétention des fluides par le pansement pour 24 h et 48 h a été calculée comme suit :

la masse de vapeur d'eau perdue au travers du pansement (W2 - W3) et la masse de fluide absorbée par le pansement (W4 - W1) pour les périodes de 24 h et de 48 h ont été calculées et sommées. La somme des deux masses représente la capacité de rétention.

Ainsi la détermination des différentes caractéristiques de WVTR sont réalisé comme suit:

- W4-W1 représente la rétention de l'eau dans le pansement si la différence est positive. Si elle est négative, la différence représente l'évaporation de l'eau présente dans le pansement.
- W2-W3 représente l'eau transmis à l'air ambiant à travers le pansement.
- Si W4-W1 est négative la WVTR se détermine par : ((W2-W3) + (W4-W1))/$cm^2$ de pansement.
- Si W4-W1 est positive la WVTR se détermine par ((W2-W3) - (W4-W1))/$cm^2$ de pansement.

Les résultats sont la moyenne de trois mesures.

**Résultats :**

Aspect des pansements après lyophilisation :

Les pansements obtenus sont représentés dans les figures 16 et 17.

Les figures 16 et 17 représentent des photographies de pansements en fonction du non tissé.

Comme montré sur les figures 16 et 17, les non tissés n'absorbent pas la gélatine de la même façon, ainsi certain ont tendance à flotter à la surface de la solution protéique ce qui pose problème dans la fabrication du pansement. Ce phénomène intervient dans avec les non tissés suivant :

- MA50 J
- MA 50 + PE
- Tissue 5-Ig + PE
- MAPPR 20

A l'opposé, le pansement ZWHO "c" 12-Ig possède un pouvoir trop absorbant puisque toute la gélatine s'est retrouvée au sein même du non tissé.

Ces deux critères sont éliminatoires puisqu'il est nécessaire que le pansement possède une face uniquement composé de gel, évitant le contact direct entre la plaie et le non tissé, et une face composé du non tissé adhéré imprégné de gel.

Les non tissés contenant un film de polyuréthane (PE) adhèrent à très peu de gel, laissant une bonne partie en dehors du pansement. Plusieurs pansements présentant des zones craquelées, c'est le cas pour ceux constitué de WSV 100, MA 30 et MA40 W4.

Avec ZWHO "c" 12-Ig, l'échantillon se présente plus sous forme de compresse imbibée de gel que de pansement. Le non tissé MASP50 P possède la particularité d'avoir une face coloré donnant un pansement ayant une face blanche (coté gel) et une face bleu (coté compresse). Par contre, on remarque une hétérogénéité de répartition du gel sur la face bleu. Les autres non tissés permettent d'obtenir des pansements dont l'aspect est proche de la référence.

Tel que démontré dans cet exemple, les caractéristiques du non-tissé sont importantes pour l'obtention d'un pansement fonctionnel.

Caractéristiques d'absorption :

Certains pansements ont été caractérisés uniquement en fonction de leur capacité de réhydratation alors que d'autre uniquement en fonction de leur taux maximum de réhydratation. Dans les deux cas, les non tissés sont comparés ont la référence (non tissé Lyofal). Les résultats obtenus concernant les capacités d'absorption sont représentés sur la figure 18.

La figure 18 est un histogramme représentant la capacité d'absorption du pansement en fonction du non tissé incorporé dans sa matrice. Sur la figure « 1 » correspond aux résultats obtenus avec un non tissé lyofal, « 2 » avec un non tissé MA 50 J, « 3 » avec un non tissé WSV 100, « 4 » avec un non tissé MA 57 S, « 5 » avec un non tissé MA 50 + PE comprenant du polyéthylène, « 6 » avec un non tissé Tissue 5-Ig + PE comprenant du polyéthylène, « 7 » avec un non tissé MASP50 et « 8 » avec un non tissé MASP50 P.

Les pansements, étant constitués de non tissé formé d'un film de polyéthylène (PE) possède, une capacité d'absorption bien inférieure à la référence, pouvant résulter de la faible présence de gel au sein du pansement. Les autres non tissés permettent d'obtenir des pansements doués de capacité d'absorption proche du pansement Lyofal.

Taux de réhydratation :

Les résultats obtenus concernant le taux de réhydratation sont représentés sur la figure 19. La figure 19 est un histogramme représentant le taux de réhydratation du pansement en fonction du non tissé incorporé dans sa matrice. Sur la figure « 1 » correspond aux résultats obtenus avec un non tissé lyofal, « 2 » avec un non tissé MA 60, « 3 » avec un non tissé MA40W4, « 4 » avec un non tissé ZWHO"c" 12-lg, « 5 » avec un non tissé MA 30, « 6 » avec un non tissé MASP 50 P, « 7 » avec un non tissé MA 71.08.
Aucun pansement ne possède de taux de réhydratation supérieur à la référence.
Certain comme le MA60, le MA40 W4, le ZWHO se réhydratent légèrement plus faiblement que celui de Lyofal (figure 18).

WVTR (water vapor transmission rate):

Les résultats obtenus concernant le WVTR (water vapor transmission rate) sont représentés dans la figure 20. La figure 20 est un histogramme représentant la WVTR (water vapor transmission rate): du pansement en fonction du non tissé incorporé dans sa matrice. Sur la figure « 1 » correspond aux résultats obtenus avec un non tissé lyofal, « 2 » avec un non tissé MA 50 J, « 3 » avec un non tissé WSV 100, « 4 » avec un non tissé MA 57 S, « 5 » avec un non tissé MA 50 + PE comprenant du polyéthylène, « 6 » avec un non tissé MASP 50, « 7 » avec un non tissé MASP 50 P.
Il semble, à la lumière des résultats obtenus, que le pansement ayant une compresse doté d'un film polyéthylène laisse passer beaucoup plus de vapeur d'eau que les autres pansements (figure 19, colonne 5). Mais il faut prendre en compte que ce pansement contient moins de gel, qui est considéré comme le facteur limitant le plus la transmission d'eau sous forme de vapeur. Le MA50 J possède une WVTR supérieur à la référence (figure 20), mais au vu de l'écart type, cette caractéristique ne peut être prise en compte.

Conclusions de l'essai / exemple :

Les non tissés possédant un film PE semble moins performant puisqu'ils n'adhèrent pas suffisamment de gel.
Le non tissé Lyofal possède les meilleures caractéristiques d'absorption, que cela soit en capacité ou en taux de réhydratation.
Bien que moins absorbant, le MASP50 P possède une face coloré encore visible après la lyophilisation et la réhydratation du pansement. Il serait intéressant d'envisager l'utilisation d'une compresse Lyofal marquée d'un coté, ce qui permettrait l'obtention d'une face d'application sur le pansement MAIA.
Tel que démontré dans cet exemple, les caractéristiques du non-tissé sont importantes pour l'obtention d'un pansement fonctionnel. En effet, en fonction du non tissé, les propriétés du pansement et donc du biomatériau sont modifiés.

**Exemple 5 : Etude complémentaire sur l'effet du non tissé :**

<u>1- But de l'essai :</u>

L'étude a porté sur la caractérisation du pansement de l'invention (MW1) contenant différents types de compresses non-tissés. L'objectif a été de définir les spécifications de la matière première adaptées.
Les tests réalisés sont les suivants :

- Test de lyophilisation
- Test d'absorption
- Test rhéologique
- Test microbiologique

<u>2- Descriptif de l'essai :</u>

Dans cet exemple, les produits et procédé mis en oeuvre sont indiqués ci-dessous.
Les Références des non-tissés (NT) testés sont : MA 60, MA 71.080, ZWHO "C" 12-LG, Baumwell-molten, MA 936-80, MA 50 J MA 7110
L'ensemble des non tissé (compresses) proviennent de la société Mankk
La composition du pansement, du biomatériau est : gélatine 3%, sorbitol 4,5%, transglutaminase 4g/L, collagénase $2,5x10^{-5}$g/L (sauf MA 60 et MA 71.080 où il n'y a pas de collagènase.
La préparation du pansement a été réalisée selon le procédé décrit dans l'exemple 1

Le cycle de lyophilisation était le suivant :

Pré-Congélation à -77°C pendant 3 heures
Chauffage jusqu'à -55°C à une vitesse de 0,17°C/min et maintient pendant 12 minutes
Chauffage jusqu'à 10°C à une vitesse de 0,05°C/min et maintient pendant 15 heures

L'absorption a été testée selon le procédé décrit dans l'exemple 4

- Test rhéologique :

Les pansements ont été découpés de façon circulaire avec un diamètre de 40 cm. Ils ont ensuite été placés dans des tubes "Falcon" et mis en solution dans 30mL d'eau NaCl/$CaCl_2$ pendant 2 heures à 35°C. Après la réhydratation, la viscoélasticité des pansements a été déterminée en rhéologie par le biais d'un balayage de fréquence.

Conditions rhéologiques :

- Force normale d'application : 0,2N
- Déformation : 5%
- Fréquence : de 100 à 0,1 Hz
- Température : 35°C
- Test microbiologique :

    Des boîtes de Pétri contenant de l'Agar/TS ont été préparées selon le mode opératoire suivant :

    - 1,5g (+/- 0,01g) de Agar-Agar et 3g (+/- 0,01g) de poudre TS ont été introduites dans un Erlenmeyer de 250mL.
    - 100mL d'eau osmosée ont été ajoutées et bouchonné de façon adéquate pour l'autoclavage.
    - Le mélange obtenu a été placé à 121 $\pm$ 1 °C dans un autoclave pendant 20 min.
    - Les Erlenmeyers de l'autoclave ont été retirés.
    - 30 mL de milieu TS-Agar ont été coulés dans chaque des boîtes de Pétri et Laissées 2 heures au maximum à température ambiante pour la prise en masse sans recouvrir les boîtes de Pétri.
    - Le couvercle a été replacé sur les boîtes. Elles peuvent être stockés 24h à température ambiante et 4 jours à 4°C.

Trois non-tissés ont été découpés à la surface du MA 60 : 38,5 $cm^2$, le MA 71.070 : 35 $cm^2$ et MA 7110 : 37,12$cm^2$
Les géloses ont ensuite été mises en contact avec les deux phases des non-tissés et laissées à incuber 72 heures à 37°C. Le nombre de colonie a été déterminé à partir de ce temps.

<u>3- Résultats :</u>

Après la lyophilisation, l'aspect des pansements ainsi que leur poids ont été déterminés. Le tableau 4 ci-dessous présente le poids des pansements en fonction du non-tissé.

Tableau 4 : poids des pansements en fonction du non-tissé

| | MA 71.080 | MA 60 | MA936-80 | ZWHO "C" 12-LG, | Baumwell- molten, | MA 50 J | MA 7110 |
|---|---|---|---|---|---|---|---|
| Poids (g) | 2,28 | 2,51 | 3,05 | 3,96 | 3,25 | 2,93 | 3,19 |

La différence de poids peut s'expliquer par la variation de masse des compresses. Selon leur aspect, il est apparu que tous les pansements étaient bien lyophilisés. De plus :

- Le MA 60 est : souple, homogène et blanc.
- MA 71.080 est : souple, homogène et blanc.
- ZWHO "C" 12-LG est : rigide, présente les motifs du NT et des bulles en dessous du pansement.
- Baumwell-molten est : rigide, présente les motifs du NT et des bulles en dessous du pansement.

- MA 936-80 est : souple, homogène et blanc.
- MA 50 J est : souple, homogène, blanc et la phase NT est bien marquée.
- MA 7110 est : souple, homogène et blanc.

Test d'absorption :

Les résultats sont représentés dans les figures 21 et 22 .La figure 21 est un schéma représentant la cinétique de réhydratation des pansements en fonction des différents non tissés testés. La figure 22 est un schéma représentant la capacité d'absorption des pansements en fonction des différents non tissés testés. Sur la figure « 1 » correspond aux résultats obtenus avec un non tissé ZWHO "C", « 2 » avec un non tissé Baumwell, « 3 » avec un non tissé MA936, « 4 » avec un non tissé MA 50 J, « 5 » avec un non tissé MA 7110, « 6 » avec un non tissé MA 71.080 et « 7 » avec un non tissé MA60.

Seuls le ZWHO "C" 12-LG et Baumwell-molten présentent une saturation d'absorption en moins de 4 heures (spécification MW1 $\geq$ à 4h).

Les capacités d'absorption pour les non tissés sont inférieures à la référence MA7110, sauf pour le MA60 et le MA71.080, mais restent dans les spécifications requises (du MW1) (15g eau/100 $cm^2$).

Il est à remarquer que l'aspect du pansement comprenant le non-tissé ZWHO "C" 12-LG et Baumwell-molten après réhydratation présentait de la mousse au contact de l'absorbât ; et le pansement comprenant le non-tissé MA 50 J : le non-tissé s'est décroché du gel indiquant qu'il ne s'est pas imprégné dans le gel lors de la fabrication du pansement.

Test de rhéologie :

Les résultats sont représentés dans la figure 23. La figure 23 est un schéma représentant le G' du pansement (propriété élastiques) en fonction du non tissé. Le ZWHO "C" 12-LG et Baumwell-molten ne présentent pas d'état gélifié (G'<G"). Les autres non-tissés ont un pansement avec une structure de gel. Les valeurs de G' de ces gels répondent aux exigences du MW1 (G'>100Pa sans la stérilisation).

Test de microbiologie :

Les résultats sont représentés dans le tableau 4 ci-dessous :

Tableau 5 : résultats des tests de rhéologie

| | Non tissé | | |
|---|---|---|---|
| | MA 1110 | MA 60 | MA 71.070 |
| 72 heures | 3 | 21 | 9 |
| Surface (cm) | 37,12 | 38,5 | 35 |
| CFU/DM | 5 | 35 | 17 |

Tel que démontré dans cet exemple, la biocharge, c'est-à-dire le nombre d'unité formant colonie (CFU) par pansement obtenue est inférieur à 100 CFU/pansement, et est donc compatible avec une stérilisation sous ionisation Béta.

Tel que démontré, l'ajout de compresse ne rajoute pas de charge bactérienne exogène rédhibitoire à la fabrication du pansement.

<u>4- Conclusions de l'essai/exemple:</u>

Le ZWHO "C" 12-LG et Baumwell-molten ne sont pas des bons candidats pour le non tissé du pansement MW1 pour la raison rédhibitoire que les pansements formés avec ces NT ne présentent pas d'état gélifié.

Le MA 50 J est exclu puisqu'il ne s'imbibe pas de gel lors de la fabrication du pansement.

Les autres NT respectent les spécifications du MW1. Le choix du non tissé dépendra aussi des exigences déjà établies comme la composition 100% viscose ou l'enzymage.

Dans ce cas le MA60 et le MA71.080 sont préférés pour leur utilisation dans la fabrication du pansement.

Ainsi, tel que démontré dans cet exemple, le pansement de l'invention pour avoir une fonctionnalité optimum et une adaptation aux plaies des patients, des caractéristiques particulières, comme une charge bactérienne inférieure à 200 CFU/70 $cm^2$, le non tissé doit imbiber la solution de biomatériau (d'Engel) sans gêner la gélification ni sa conformabilité, la taille des mailles peut être comprise de préférence entre 50 et 500$\mu$m.

**Exemple 6 : test des pansements de l'invention**

I/ Protocoles et conditions

Dans cet exemple des tests de validation des pansements stérilisés de trois lots, identifiés Lot 11MW101, Lot 11MW102 et Lot 11MW103, ont été effectués. Il s'agit de pansement produit selon le procédé de l'invention décrit dans l'exemple1 et comprenant un non-tissé commercialisé par la société Norafin sous la référence 71.100.001, 100% viscose.

Contrôles qualités effectués :

- Aspect des pansements
- Test d'absorption, comme décris dans l'exemple 4.
- Test de viscoélasticité, comme décris dans l'exemple 4.
- Test d'affinité.
- Test de resolubilisation.

Protocole :

- Aspect : pesées, photos éventuelles, évaluation de la souplesse et du cassant.
- Spécification : pas de zones de fusion, non lyophilisée et pas de plis, souple (pas de cassure lorsque l'on rapproche les deux bords du pansement), stérile selon test de la pharmacopée, dose d'irradiation appliquée : comprise entre 30 et 25kGy, masse > 2,7g

-Test d'absorption :

Nombre d'échantillons testés : 20

Protocole :

- Découpe de 90 échantillons circulaires d'un diamètre de 12,56 $cm^2$ avec un emporte pièce afin d'obtenir des échantillons
- Pesée des échantillons Pi
- Dépôt des échantillons de pansements dans des boîtes de Pétri
- Ajout dans chaque boîte de Pétri d'une solution A (NaCl 9% (m/v) - $CaCl_2$ 0,0368% (m/v) à une quantité d'au moins 40 x le poids de l'échantillon
- Incubation à 35°C
- Pesée des pansements après 0,5, 1, 2, 4 et 6 heures d'incubation.

Spécification :

- . Capacité > 30g eau/100$cm^2$
- . Temps de saturation > 4 heures (Valeur basse de la moyenne définit selon son écart type)

- Test de viscoélasticité :

Nombre d'échantillons : 20

Protocole :

- Analyse de la viscoélasticité des pansements hydratés à 6 heures par le biais d'un balayage de fréquences.
- Conditions rhéologiques :

  - . mode oscillatoire
  - . gamma = 0,05
  - . Fréquence = de 0,1 à 100Hz
  - . Température = 35°C

Spécification :

- . G' (0,1Hz) > 100 Pa
- (Valeur basse de la moyenne définit selon son écart type)
- . Rapport G'/G" > 10
- (Valeur basse de la moyenne définit selon son écart type)
- . Pente du G' compris entre -1 et 1
- (Valeur haute et basse de la moyenne définit selon son écart type)
- Concernant la pente du G', les résultats négatifs ne sont pas prise en compte dans le calcul des
- moyennes.

<u>- Test d'Affinité :</u>

Nombre d'échantillons : 20

Matériels utilisés :

- Dix seringues graduées, d'une capacité nominale de 50 ml ou 60 ml et de 30+/-2 mm de diamètre intérieur, dont les canules ont été coupées et équipées de pistons de marque Terumo, modèle seringue 50 ml sans aiguille.
- Solution d'essai A : 8,298 g de chlorure de sodium (NaCl) et 0,368 g de chlorure de calcium dihydrate ($CaCl_2$, $2H_2O$) dans de l'eau déionisée et complétée jusqu'à constituer 1 litre de solution dans un flacon volumétrique.
- Poudre de gélatine Porcine (Roussselot PS), (175 bloom).
- Poudre d'agar-agar, (agar-agar bactériologique de type 1).
- Autoclave de laboratoire de marque Melag modèle melatronic 23, adaptée à la stérilisation de liquides dans des récipients fermés.
- Parafilm.
- Incubateur de laboratoire de marque Binder modèle BF 115, capable de maintenir une température de (25+/-2) °C.
- Incubateur de laboratoire, capable de maintenir une température de (60 +/-2) °C. Possibilité d'utiliser des bains maries.
- Balance, capable de peser 100 g à 0,0001 g près.

Protocole

Une quantité de 2,00+/-0,01g de poudre d'agar-agar a été pesée et mise dans un récipient approprié. Une quantité suffisante de solution d'essai A a été ajoutée afin d'obtenir une masse totale de réactif de 100,00 +/- 0,02 g. Le récipient a été fermé de manière étanche et placé à 121+/-1°C dans un autoclave pendant 20 min.

Le récipient a été retiré et le laisser refroidir jusqu'à 60+/-5°C avant utilisation. Une quantité suffisante de poudre de gélatine a été ajouté à 65,00 +/-0,02 g de solution d'essai A dans un récipient à col large approprié pour obtenir une masse totale de réactif de 100,00 +/-0,02 g. Le récipient a été fermé de manière étanche, l'agité jusqu'à ce que la poudre de gélatine soit dispersée, puis maintenu à une température de 60°C pendant au moins 12 h mais sans dépasser 18 h. À l'issue de cette période, une vérification que la gélatine forme une solution limpide et homogène a été effectuée.

Le piston de la seringue a été remonté jusqu'à la graduation 30mL et 10g de gélatine ou de d'agar-agar ont été ajoutés dans la seringue et boucher avec du Parafilm pour éviter l'évaporation (effectuer 5x) pour chaque type de substrat.

Les seringues ont été mises verticalement dans une étuve (ou conditions ambiantes) à 25°C pendant 3h avec les bouchons de Parafilm pour éviter l'évaporation former pendant l'épaississement.

Chaque seringue a été pesée (valeur W1) avec le parafilm. 10 g de pansement, ont été ajoutés de façon uniforme à la surface de la gélatine. Les seringues ont été ensuite pesées (valeurs W2).

Les seringues ont été rebouchées avec le même Parafilm et placées dans un incubateur à 25°C (ou dans les conditions ambiantes), pendant 48h.

Les seringues ont été pesées (valeur W3). Le piston a été enfoncé pour retirer le pansement et les seringues ont été pesées (valeur W4).

La capacité du pansement à capter du fluide a été mesurée selon la formule suivante, W5 :

$$W5 = \{ ([W3-W4] - [W2-W1]) / (W2-W1) \} \times 100$$

Spécification : Pourvoir hydratant > 21% (Valeur basse de la moyenne définit selon son écart type)

- Test de resolubilisation :

Nombre d'échantillons : 1

Protocole :

- Découpe d'un échantillon circulaire d'un diamètre de 12,56 cm
- Ajout dans chaque boîte de Pétri de la solution A (NaCl 9% - CaCl2 0,0368%) à une quantité d'au moins 40 x le poids de l'échantillon
- Incubation à 35°C pendant 1 h30
- Dépôt de l'échantillon sur le rhéomètre commercialisé par la société Anton-Paar référencé sous le nom MCR 301
- Conditions rhéologiques :

    o mode oscillatoire
    o g = 0,05
    o Fréquence = 1 Hz
    o Température = 35°C

  Spécification : G' compris entre 1 et 7 Pa à 72h +/-10%

II- Résultats :

II. 1 Aspect des pansements :

Les résultats obtenus sont représentés dans les tableaux 6 à 8 ci-dessous.

- Lot 11MW101 :

Tableau 6 : aspect des pansements obtenus

| | Valeur moyenne | Conformité |
|---|---|---|
| Pansements avec zones fusionnées (%) | 0 | ✓ Oui ☐ Non |
| Pansements avec zones non lyophilisée (%) | 0 | ✓ Oui ☐ Non |
| Pansements avec plis (%) | 0 | ✓ Oui ☐ Non |
| Pansements souples (%) | 100 | ✓ Oui ☐ Non |
| Masse (g) | 3.06 +/- 0.17 | ✓ Oui ☐ Non |

- Lot 11MW102 :

Tableau 7 : aspect des pansements obtenus

| | Valeur moyenne | Conformité |
|---|---|---|
| Pansements avec zones fusionnées (%) | 0 | ✓ Oui ☐ Non |
| Pansements avec zones non lyophilisée (%) | 0 | ✓ Oui ☐ Non |
| Pansements avec plis (%) | 0 | ✓ Oui ☐ Non |
| Pansements souples (%) | 100 | ✓ Oui ☐ Non |
| Masse (g) | 3.12 +/- 0.26 | ✓ Oui ☐ Non |

- Lot 11MW103 :

Tableau 8 : aspect des pansements obtenus

| | Valeur moyenne | Conformité |
|---|---|---|
| Pansements avec zones fusionnées (%) | 0 | ✓ Oui ☐ Non |
| Pansements avec zones non lyophilisée (%) | 0 | ✓ Oui ☐ Non |
| Pansements avec plis (%) | 0 | ✓ Oui ☐ Non |
| Pansements souples (%) | 100 | ✓ Oui ☐ Non |
| Masse (g) | 3,08 +/- 0,24 | ✓ Oui ☐ Non |

Le pansement présente donc les caractéristiques requises à savoir une bonne souplesse tout comprenant un biomatériau lyophilisé capable de s'hydrater pour obtenir un gel et faire des transitions solution/gel/solution.

<u>II. 2 Test d'absorption</u>

Les résultats obtenus sont représentés dans les tableaux 9 à 11 ci-
- Lot 11MW101 :

Tableau 9 : résultats d'absorption

| | Valeur moyenne | Valeur basse | Conformité |
|---|---|---|---|
| Capacité d'absorption ($g_{eau}/100cm^2$) | 47.36 +/- 8.62 | 38,7 | ✓ Oui ☐ Non |
| Temps de saturation (heures) | > 6 | 6 | ✓ Oui ☐ Non |

- Lot 11MW102 :

Tableau 10 : résultats d'absorption

| | Valeur moyenne | Valeur basse | Conformité |
|---|---|---|---|
| Capacité d'absorption ($g_{eau}/100cm^2$) | 49.84 +/- 6.58 | 43.3 | ✓ Oui ☐ Non |
| Temps de saturation heures) | > 6 | 6 | ✓ Oui ☐ Non |

- Lot 11MW103 :

Tableau 11 : résultats d'absorption

| | Valeur moyenne | Valeur basse | Conformité |
|---|---|---|---|
| Capacité d'absorption ($g_{eau}/100cm^2$) | 45,2 +/- 3,1 | 42,1 | ✓ Oui ☐ Non |
| Temps de saturation heures) | > 6 | 6 | ✓ Oui ☐ Non |

Le pansement présente donc les caractéristiques requises à savoir une capacité d'absorption de liquide avec

un temps de saturation adapté à des plaies. Le pansement de l'invention permet donc une absorption des exsudats d'une plaie.

II. 3 Tests de viscoélasticité

Les résultats obtenus sont représentés dans le tableau 12 ci-dessous
- Lot 11MW103 :

Tableau 12 : résultats de viscoélasticité

| | Valeur moyenne | Valeur haute | Valeur basse | Conformité | % de pansement conforme |
|---|---|---|---|---|---|
| Valeur du G' à 0,1 Hz (Pa) | 23,65 +/- 8,5 | NA | 15,15 | ✔ Oui ☐ Non | 100% |
| Rapport G'/G" | 23,7 +/- 7 | NA | 16,7 | ✔ Oui ☐ Non | 100% |
| Pante du G' | 0,02 +/- 0,18 | 0,2 | -0,16 | ✔ Oui ☐ Non | 100% |

Le pansement présente donc les caractéristiques requises à savoir une viscoélasticité permettant sont adaptation à tout type de plaies et sur tout type de surface, qu'elle soit cornée, constitué d'épiderme ou non, en phase d'épithéliation ou non.

Aucun des pansements testés et mis en solution ne se sont montrés dispersifs. A cette valeur le pansement hors spécification garde son intégrité.

Tel que démontré dans cet exemple, le pansement de l'invention permet de conserver sa structure, c'est-à-dire le biomatériau ne se dissocie pas du non tissé et permet d'avoir des propriétés de souplesse, d'absorption de liquides et de souplesse adapté avec les plaies de mammifères.

II. 4 Test d'hydratation :

Les résultats obtenus sont représentés dans les tableaux 13 à 15 ci-dessous.

- Lot 11MW101 :

Tableau 13 : résultats d'hydratation du pansement

| | Valeur moyenne | Valeur basse | Conformité |
|---|---|---|---|
| Pouvoir hydratant (%) | 25,1 +/- 2,3 | 22,8 | ✔ Oui ☐ Non |

- Lot 11MW102 :

Tableau 14 : résultats d'hydratation du pansement

| | Valeur moyenne | Valeur basse | Conformité |
|---|---|---|---|
| Pouvoir hydratant %) | 27 +/- 2,8 | 24,2 | ✔ Oui ☐ Non |

- Lot 11MW103 :

Tableau 15 : résultats d'hydratation du pansement

| | Valeur moyenne | Valeur basse | Conformité |
|---|---|---|---|
| Pouvoir hydratant %) | 26.6 +/- 1.3 | 25.3 | ✔ Oui ☐ Non |

Tel que démontré dans cet exemple, le pansement de l'invention permet en outre d'hydrater la plaie et ainsi de favoriser la cicatrisation la cicatrisation humide tout en gérant/absorbant les exsudats émis par la plaie.

II. 4 Test de resolubilisation :

Les résultats obtenus sont représentés dans les tableaux 16 à 18 ci-dessous
- Lot 11MW101 :

Tableau 16 : résultats de resolubilisation

| | Temps de mesure | Valeur | Conformité |
|---|---|---|---|
| Valeur G' à 72 heures +/- 10% | 72 heures | 3.8 | ✓ Oui ☐ Non |

- Lot 11MW102 Tableau 17 : résultats de resolubilisation

| | Temps de mesure | Valeur | Conformité |
|---|---|---|---|
| Valeur G' à 72 heures +/-10% | 75 heures | 6.97 | ✓ Oui ☐ Non |

- Lot 11MW103 :

Tableau 18 : résultats de resolubilisation

| | Temps de mesure | Valeur | Conformité |
|---|---|---|---|
| Valeur G' à 72 heures +/-10% | 72 heures | 2.3 | ✓ Oui ☐ Non |

Tel que démontré dans cet exemple, le pansement de l'invention permet de faire des transitions gel/solution/gel favorisant la cicatrisation de plaies. Ces transitions permettent avantageusement d'absorber des exsudats de plaies.

IV- Conclusions :

Le procédé de fabrication ainsi que le pansement obtenu tel qu'il a été décrit est donc validé et permet d'obtenir de façon surprenante des caractéristiques physiques et des propriétés avantageuses pour la cicatrisation des plaies, car il permet à la fois d'absorber les exsudats tout en assurant la cicatrisation humide. Sa cinétique de resolubilisation préservée lui confère, notamment, des propriétés de non adhérence acquises dans le temps et contrôlées à la fabrication, faisant de ce pansement, le premier pansement dynamique jamais créé.

Cet exemple montre bien que les pansements possèdent des propriétés avantageuses d'élasticité, absorption, resolubilisation et hydratation. Ces propriétés avantageuses sont notamment nécessaires à la relance de la cicatrisation, tout en préservant la plaie et ses berges lors du retrait dudit pansement.

**Exemple 7: composition d'un pansement selon l'invention et caractéristiques physicochimiques correspondantes.**

Un exemple de pansement selon l'invention est décrit ci-dessous.

Les composants et solutions utilisés étaient ceux décrit dans l'exemple 1

Le pansement a été fabriqué selon le procédé décrit dans l'exemple 1 à partir des éléments suivants : de la gélatine à 3% (30mg/mL), du sorbitol à 4,5% (45mg/mL), de la transglutaminase à 4mg/mL, de la collagénase à $2,2x10^{-5}$mg/mL et un non tissé 100% viscose commercialisé par la société Norafin sous la référence commerciale 71.100.001, 100% viscose.

Les caractéristiques du pansement testées étaient les suivantes :

- Absorption> 30 g/cm$^2$
- Pouvoir hydratant > 21 %
- G'> 10 Pa (après 3h de réhydratation à 0,1Hz)
- G' > 10 G" (après 3h de réhydratation à 0,1Hz),et
- variation G'=fn(Fq) comprise entre -1 et 1
- un temps de saturation > 4h
- temps de resolubilisation égale à 72 h+/- 10% pansements stériles.

Les procédés utilisés afin de mesurer ces caractéristiques correspondent aux procédés décrits dans les exemples 4 et 6

Le tableau 19 ci-dessous décrit l'évolution du pansement en fonction du temps et ces propriétés lors des transitions solution/gel du biomatériau.

| Produit | Spécifications |
|---|---|
| • 1ère phase réhydratation : formation d'un gel cohésif | Caractéristiques de gélification :<br>20 Pa <G' < 70 Pa<br>G' > 10 G" (0,1 Hz)<br>et variation G'=fn(Fq) < 3% |
| | Pouvoir Absorbant (à l'état sec) : 30 < A < 50 $g_{eau}$ pour $100cm^2$ |
| | Pouvoir non dispersif (à l'état hydraté) |
| | Temps de saturation > 4h |
| • 2ème phase maintien de l'état de gel : gel hydratant pendant 3 jours | Pouvoir hydratant (à l'état hydraté) compris entre 21% et 30 % de perte de poids du pansement |
| • 3ème phase liquéfaction du gel permettant le retrait atraumatique du pansement | Temps de resolubilisation = 72 +/-7h |
| Etre biocompatible | Voir spécifications d'achat des matières premières |
| Etre stérile | Biocharge avant stérilisation : < 1000 CFU |
| | Stérilisation beta<br>Dose min : 25 kGy Dose max : 30 kGy |
| Etre stable dans le temps | Cycle de lyophilisation permettant : Taux d'HR < 7%<br>Absence de zones non lyophilisés |
| | Conditions de stockage : 10 à 25°c |
| | Stabilité : 2 ans |
| | Scellage permettant un conditionnement primaire imperméable à l'eau et à l'air |
| | Voir spécifications d'achat des emballages 1 aire et 2aire |
| Etre de forme et de dimensions désirées | Dimensions = 65x100mm + Autres dimensions à déterminer par la suite |
| | Bords nets et non coupants |
| | Répartition homogène |
| | Couleurs blanche |
| | Absence de figures de fusion |
| | Absence de plis du non-tissé |

Cet exemple montre bien que les pansements obtenus par le procédé de l'invention présentent des propriétés avantageuses d'élasticité, absorption, resolubilisation et hydratation. En outre, les pansements obtenus sont stériles, homogènes.

En outre le pansement de l'invention conserve de sa structure quelque soit les conditions et ainsi permet une application et un retrait de la plaie sans perte de biomatériau, sans déchirure. Le pansement obtenu par le procédé de l'invention permet avantageusement son retrait sans laisser de biomatériau sur la plaie cicatrisée, sans adhésion du non tissé à la plaie améliorant ainsi le confort des patients. En outre ces caractéristiques évitent une étape de nettoyage de la plaie et/ou de réhydratation de la plaie qui pourrait altérer la cicatrisation/fragiliser la cicatrice.

**Exemple 8 : Tests in-vitro et in-vivo du pansement selon l'invention.**

Pour toutes les expériences menées dans cette exemple le pansement de l'invention utilisé, étaient constitué

des composants et solutions décrit dans l'exemple 1 et dont les caractéristiques sont décrites dans l'exemple 7.

Ainsi, le pansement de l'invention a été fabriqué selon le procédé décrit dans l'exemple 1 à partir des éléments suivants : de la gélatine, commercialisée par la société Rousselot, à 3% (30mg/mL), du sorbitol, commercialisé par la société IMCD, à 4,5% (45mg/mL), de la transglutaminase à 4mg/mL, commercialisée par la société Ajinomoto, de la collagénase à 2,2x$10^{-5}$mg/mL, commercialisée par la société Coger et un non tissé 100% viscose commercialisé par la société Norafin sous la référence commerciale 71.100.001, 100% viscose.

Il a été ensuite lyophilisé selon le protocole décrit dans l'exemple 1.

Des échantillons de pansements selon l'invention ont été lyophilisés et réhydratés, puis ont été analysés à l'aide d'un microscope Philips XL 30 ESEM-FEG (Eindhoven, Pays-Bas) à une tension d'accélération de 15 keV à une pression de 5 Torr.

Les échantillons réhydratés ont été obtenus après incubation du pansement dans du tampon Tris 50 mM HCl (pH 7,4) pendant 4 minutes ou 20 minutes et durant la nuit, c'est-à-dire pendant plus de 12 heures pour un maximum de réhydratation.

La figure 24 représente les photographies en microscopie électronique des pansements obtenus après les différents temps de réhydratation. Comme montré sur la figure 24 : le pansement sec (figure 24A) est composé de macropores qui disparaissent dès 4 minutes d'hydratation (figure 24B) dans la solution saline précitées. Puis avec le temps, le gel se reconstitue jusqu'à former, après 20 minutes (figures C et D), une matrice cohésive sans aucun pore.

La figure 25 représente un diagramme de l'évolution de l'élasticité et du stockage du pansement en fonction du temps. Pour ce faire, le pansement de l'invention a été hydraté pendant 1h30 dans une solution contenant 142 mMol.$L^{-1}$ de NaCl à température ambiante, c'est-à-dire 25°C, a été observé en rhéologie avec un rhéomètre MCR 301 commercialisé par la société Anton Paar. Pour éviter la modification des propriétés mécaniques du pansement, une force normale de 0,2 N (Newton) a été appliquée sur l'échantillon hydraté. Le module de conservation G' et module de perte G" ont été observé en mode d'oscillation et enregistré en fonction du temps à température constante (35°C).

Comme démontré sur la figure 25 le pansement de l'invention sous forme de gel possède les propriétés de transition gel-solution inhérente à la technologie Enzgel.

Le fait de garder ces capacités de transition de phase apporte des propriétés avantageuses pour un pansement. En effet, chaque gel possède une élasticité propre. Les propriétés collantes de ces gels sont corrélées directement à leur élasticité. Le changement d'élasticité du pansement de l'invention au court du temps, permet avantageusement un changement du pouvoir collant au cours du temps tel que démontré avec l'expérience suivante décrite sur la figure 26.

La capacité d'adhésion/force collante du pansement a été étudiée. Le but de cette expérience était de mesurer la force nécessaire pour décoller un pansement selon l'invention.

Le pansement selon l'invention a été hydraté pendant 1 h30 dans une solution contenant 142 mMol.$L^{-1}$ de NaCl à température ambiante, c'est-à-dire à 25°c Les échantillons ont été déposés sur le même rhéomètre que précédemment et une force normale de 0,2 N à été appliquée par un plateau. Après 5 min à 35°C, une pression de 10 N a été appliquée et le plateau a été levé à une vitesse de 1mm.$s^{-1}$. La force normale nécessaire à l'arrachage du pansement au rhéomètre à été suivie et mis en rapport à l'écart entre le plateau et le rhéomètre. L'ensemble de la force normale nécessaire à été intégré (iNF) en utilisant le logiciel ORIGINE 7 (marque déposée) commercialisé par la société OriginLab Corporation.

La figure 26 représente les résultats obtenus de l'intégration en fonction du pansement. L'intégration représentée par le bâton 1 figure 26, correspond à un pansement qui, une fois gonflé, possède un G' de 35 Pa. Cette valeur correspond à l'élasticité du pansement au moment de sa pose sur la plaie. L'intégration représentée par la par le bâton 3 figure 26 correspond à un pansement qui, une fois gonflé, possède un G' de 3 Pa, ce qui correspond à un pansement après 48h de contact avec une plaie modèle. Tel que démontre sur la figure 26, il a été observé qu'il faut 3 fois moins de force pour décoller un pansement selon l'invention après 48h de pose (de -3 s.$N^{-1}$ à -1 s.$N^{-1}$).

Ainsi, le pansement selon l'invention possède donc avantageusement, l'adhésivité nécessaire à une pose facile. En outre, le pansement perd avantageusement, son pouvoir d'adhésion au cours de l'interaction avec la plaie et du temps, ce qui facilite son retrait et permet de garder les cellules nouvellement formées dans la plaie intactes.

Outre les propriétés d'adhérences liées à la transition gel-sol, le gel lyophilisé, mis sous forme de pansement grâce à son association avec le non tissé, a acquis avantageusement de nouvelles propriétés inattendues.

La capacité d'absorption a, par exemple, été étudié selon le protocole de l'exemple 4, et représente sur la figure 27. Ainsi, tel que démontré sur cette figure, le pansement de l'invention est avantageusement capable d'absorber de grande quantité de liquide jusqu'à 47g d'eau/100$cm^2$ en 6 heures.

En outre, les inventeurs ont démontré par microscopie électronique à balayage, que les pores formés par la lyophilisation disparaissent au cours de l'hydratation du pansement. Les pores se remplissent de liquide ; le gel lyophilisé absorbe ce liquide et retrouve son intégrité. Ce qui veut dire que, dans un premier temps, les pores créés par la lyophilisation se remplissent d'eau et le gel se reforme, c'est l'étape de réhydratation du lyophilisat, dans un second temps la cinétique devient plus lente, c'est l'étape de gonflement du gel.

Une fois gonflé, le gel atteint un équilibre d'échange aqueux. Ainsi, si la plaie secrète encore des exsudats, le

pansement de l'invention continue avantageusement de les absorber, mais si la sécrétion s'arrête le pansement de l'invention n'asséchera pas la plaie et pourra même la garder humide, permettant ainsi avantageusement de favoriser la cicatrisation.

Afin de montrer que le pansement selon l'invention a des propriétés avantageuses sur la cicatrisation des plaies, des plaies rachidienne dermo épidermique de 5x5 cm de longueur sur 0,5 cm de profondeur ont été réalisées sur des Porc (Sus scrofa) de type Seghers Hybrid .

La performance et la tolérance locale ont été évaluées grâce à des observations macroscopiques montrées en figure 28 et par une analyse histologique réalisée au cours des 30 jours qui suivent la création plaie. A chaque changement de pansement, chaque blessure a été nettoyée avec une solution saline NaCl 142 $mMol.L^{-1}$, préalablement chauffée à 37°C, puis un examen macroscopique des pansements et des plaies a été réalisé. La capacité de rétention des exsudats par un pansement selon l'invention ainsi que la production d'exsudats par la plaie ont été évalué de 0 à 4 (absent, léger, modéré, marqué, sévère).

À la fin de l'étude (jour 39), le porc a été pesé et une prémédication par une injection intra-musculaire d'atropine (Atropinum sulfuricum, Aguettant, en France, 0,05 $mg.kg^{-1}$) à été réalisée. L'anesthésie a été induite par une injection intramusculaire de tiletamine-zolazepam (Zoletil 100, Virbac, France) et les animaux ont été sacrifiés par une injection létale de barbituriques (Dolethal ND, Vétoquinol, France).

Une analyse histologique a été réalisée en échantillonnant les blessures (environ 3,5 x 3,5 cm). Les échantillons ont étés fixés dans une solution de 10% de formaline tamponnée. Après fixation, les échantillons ont été déshydratés dans des solutions d'alcool de concentrations croissantes, nettoyés dans le xylène et inclus dans de la paraffine. Les échantillons fixés ont été coupés en tranche de 4 à 7 $\mu$m en utilisant un microtome (MICROM (marque déposée), France). Deux sections centrales par bloc ont été préparés, l'une colorées avec du trichrome de Masson et l'autre avec une solution d'hématoxyline-éosine-Safran. Les photos de micrographies ont été prises dans les zones d'intérêt à des fins d'illustration seulement.

La production d'exsudat de la plaie ainsi que leur rétention par le pansement de l'invention ont été évalués selon le protocole cité précédemment dans l'exemple et exposé figure 29.

La figure 29 représente l'absorption de l'exsudat par un pansement selon l'invention en fonction du temps et des sécrétions de la plaie.

Tel que démontré sur la figure 29 que le pansement s'adapte avantageusement à la quantité d'exsudat de la plaie. Lorsque la plaie exsude beaucoup, le pansement retient les exsudats, mais lorsqu'il y a peu d'exsudat, il en retient peu et permet ainsi de garder la plaie humide.

Une étude histologique a été réalisée selon le protocole décrit ci-dessus, afin de qualifier la qualité de la cicatrisation des plaies traitées par le pansement de l'invention (figure 30), les échantillons ont été observés à faible magnitude (encart A) ou à moyenne magnitude (encart B).

La figure 30 représente des photographies de coupe histologiques obtenues et observées à faible grossissement (figure 30 A), ou à moyen grossissement (figure 30 B).

La performances globale s'est révélée très bonne, en raison d'un tissu de granulation épais et bien vascularisé, dominé par les fibroblastes matures, avec une quantité légère à modérée de cellules inflammatoires (lymphocytes et macrophages) ainsi qu'un dépôt marqué de collagène.

En outre, l'épithélialisation complète constituée d'un épithélium de surface bien différencié a été obtenue. L'adhésion de l'épiderme pour le derme a été trouvée bonne dans tous les sites.

Tel que démontré dans cet exemple, le pansement selon l'invention est occlusif et permet en fonction de l'état de la plaie, d'absorber ou de donner de l'eau à la plaie afin de remplir avantageusement tous les critères de la cicatrisation humide décrit par Winter (Winter GD. Formation of the scab and the rate of epithelialisation of superficial wounds in the skin of the young domestic pig. Nature 1962; 193,293- 294 [1]).

La chronicité d'une plaie est induite par une surexpression de facteurs inflammatoires. Le pansement selon absorbe ces facteurs suivant deux cinétiques distinctes. Comme dé montré, la première correspond à une absorption simple alors que la seconde correspond au gonflement d'un gel. La diffusion d'une molécule est plus lente au sein d'un gel que d'un réseau poreux : Les molécules absorbées dans la première phase sont alors retenu lorsque le pansement est gélifié. Ainsi le pansement permet d'absorber la surproduction de facteurs inflammatoires sans les redonner à la plaie.

La gélatine, composant majoritaire du pansement selon l'invention, est connue pour être un bon substrat pour les enzymes de dégradation matricielle comme les MMP. Il semble donc que le pansement lors de l'absorption des exsudats, immobilisent avantageusement ces facteurs inflammatoires en son sein et inhibe leur action sur la plaie par un excès de substrat réactionnel.

Le pansement selon l'invention crée ainsi avantageusement un environnement favorable à la cicatrisation en inhibant seulement l'excès de facteurs inflammatoire.

En conclusion, il est clairement démontré dans cet exemple que le pansement de l'invention est un pansement biocompatible, possédant des propriétés de transition sol-gel puis gel-sol, ainsi que sa formulation sous forme lyophilisée associée à un non tissé, permettent de manière nouvelle et avantageuse de faciliter la cicatrisation des plaies chroniques

tout en gardant une fonctionnalité et une capacité d'utilisation adaptée à l'ensemble des différents types de plaies.

**Références bibliographiques :**

1. Winter GD. Formation of the scab and the rate of epithelialisation of superficial wounds in the skin of the young domestic pig. Nature 1962; 193,293- 294

## Revendications

1. Pansement comprenant un support comprenant sur une de ces faces, un biomatériau capable de faire des transitions gel/solution comprenant une phase aqueuse et un réseau de polymères comprenant un polymère protéique ou saccharidique ou un mélange de polymères protéiques et saccharidiques, dans lequel le réseau de polymères et la phase aqueuse forment un gel, une première enzyme de dégradation dudit polymère, et une seconde enzyme capable d'effectuer des liaisons covalentes entre les monomères, ledit biomatériau étant lyophilisé, et ledit support étant un support non tissé.

2. Pansement selon la revendication 1, dans lequel ladite première enzyme est choisie dans le groupe comprenant les enzymes de la famille des métalloprotéinases, la famille des sérines protéases, la famille des cystéines et aspartate protéases, la famille ADAM, les glycosidases dont l'amylase, la cellulase, la dextranase, la pullulanase, la pectinase, la chitinase, la xanthanase,
la chitosanase, la hyaluronidase, et les lyases dont l'hydroxyacétyle lyase, la chondroïtinase, l'héparinase, l'alginate lyase.

3. Pansement selon la revendication 2, dans lequel la première enzyme est une collagénase.

4. Pansement selon l'une quelconque des revendications précédentes, dans lequel ladite seconde enzyme est choisie dans le groupe comprenant les enzymes de la famille des transglutaminases, des lysyls oxydases, les protéines disulfide isomérases, les sulfhydrile thiol oxydases, les peroxidases, les lipoxygènases, les épimèrases dont les alginates épimèrases, les glucuronate isomérases, la cellobiose épimèrase et les galactose-6-sulfurylases.

5. Pansement selon la revendication 4, dans lequel ladite seconde enzyme est une transglutaminase.

6. Pansement selon l'une quelconque des revendications précédentes, dans lequel le polymère protéique est choisi dans le groupe comprenant la fibrine, la gliadine, la myosine, la globuline (7S et 11S), l'actine, la myoglobine, le collagène et ses dérivés, les protéines du lait, les protéines du soja, les protéines du blé, et les protéines du jaune et du blanc d'oeuf, les protéines du pois, les protéines de féverole, les protéines du lin, les protéines de soie, la fibronectine, la laminine, l'élastine, la vitronectine.

7. Pansement selon l'une quelconque des revendications précédentes, dans lequel le polymère saccharidique est choisi dans le groupe comprenant les carraghénanes, les alginates, le xanthane, le chitosan, la chitine, l'acide hyaluronique, les glycosaminoglycanes sulfatés, le glycogène, la cellulose et ses dérivés, les pectines, l'amidon et ses dérivés, les dextrans et les xylanes.

8. Pansement selon l'une quelconque des revendications précédentes, dans lequel le polymère est de la gélatine.

9. Pansement selon l'une quelconque des revendications précédentes dans lequel, le biomatériau comprend en outre un composé cryoprotecteur choisi parmi des polyols, le glycérol, le sorbitol, le polyéthylène glycol (PEG), sorbitol glycérol, l'érythritol, le xylitol, l'arabitol, le ribitol, le dulcitol, le galactitol, le mannitol, le volemitol, le maltitol, l'isomaltitol, le lactitol ou lactositol, le glycol et ses polymères dérivés

10. Pansement selon l'une quelconque des revendications précédentes dans lequel le diamètre des mailles du non-tissé est compris de 5 à 500 $\mu$m.

11. Pansement selon l'une quelconque des revendications précédentes dans lequel le non tissé est en viscose.

**12.** Procédé de fabrication d'un pansement selon l'une quelconque des revendications 1 à 11, comprenant les étapes de :

a. préparation d'une solution polymère comprenant un polymère protéique et/ou un polymère saccharidique par mélange dudit polymère dans une solution tampon, afin d'obtenir une solution de polymère
b. préparation d'une solution enzyme comprenant une première et une seconde enzyme par mélange desdites enzymes dans une solution tampon, afin d'obtenir une solution d'enzyme
c. mélange de la solution polymère et de la solution enzyme,
d. dépôt du mélange de l'étape (c) sur une face d'un non-tissé,
e. réticulation en gel du mélange de l'étape (c) déposé sur le non tissé,
f. lyophilisation du gel formé sur le non tissé,

**13.** Procédé selon la revendication 12, comprenant en outre une étape de stérilisation du pansement.

**14.** Procédé selon la revendication 12 ou 13, dans lequel dans l'étape (a) le polymère est à une concentration comprise de 0,5 à 5 % en poids.

**15.** Procédé selon l'une quelconque des revendications 12 à 14, dans lequel l'activité de la première enzyme dans la solution enzyme est comprise de $10^{-7}$ à $10^{-4}$ unités/g de pansement.

**16.** Procédé selon l'une quelconque des revendications 12 à 15, dans lequel la de la seconde enzyme dans la solution enzyme est comprise de 0,5 à 10 Unités/g de pansement.

**17.** Procédé selon l'une quelconque des revendications 12 à 16, dans lequel la solution tampon des étapes (a) ou (b) est une solution avec un pH compris de 6,5 à 8.

**18.** Procédé selon l'une quelconque des revendications 12 à 17, dans lequel la solution de polymère comprend en outre un cryoprotecteur.

**19.** Procédé selon la revendication 18, dans lequel la concentration de cryoprotecteur est de 0,1 à 1 M, de préférence de 0,2 à 0,5 M.

**20.** Procédé selon l'une quelconque des revendications 13 à 19, dans lequel l'étape de stérilisation est réalisé par irradiation avec des rayonnements gamma ou béta.

**21.** Procédé selon la revendication 20, dans lequel la quantité de rayonnement absorbé est comprise de 0,5 à 50 kGy.

**22.** Procédé selon l'une quelconque des revendications 12 à 21, dans lequel à l'étape (c) les solutions enzyme et polymère sont mélangés selon un rapport minimal de 1/5 et soumis à agitation.

**23.** Procédé selon l'une quelconque des revendications 12 à 22, dans lequel l'étape f de lyophilisation est effectuée sous une pression de $75 \times 10^{-3}$ Pa à $300 \times 10^{-3}$ Pa et comprend outre l'étape de congélation, les étapes suivantes:

g. Etape de sublimation : augmentation de la température de -45 °C à une température comprise entre 5 et 30 °C à une vitesse de 0,01 °C par minute,
h. Etape de dessiccation : maintient de la température obtenue à l'étape de sublimation pendant un temps de 10 à 70 heures.

## Patentansprüche

**1.** Verband, umfassend einen Träger, auf dessen einer Fläche sich ein Biomaterial befindet, das die Fähigkeit hat, vom Gel- in den Lösungszustand überzugehen, enthaltend eine wässrige Phase und ein Netz aus Polymeren enthaltend ein Protein- oder Saccharidpolymer oder ein Gemisch aus Protein- und Saccharidpolymeren, in denen das Polymernetz und die wässrige Phase ein Gel bilden, ein erstes Abbauenzym dieses Polymers und ein zweites Enzym, das die Fähigkeit hat, kovalente Bindungen zwischen den Monomeren zu bilden, wobei dieses Biomaterial gefriergetrocknet und der besagte Träger aus einem Vliesstoff besteht.

**2.** Verband nach Anspruch 1, in dem das besagte erste Enzym ausgewählt wird aus der Gruppe umfassend die Enzyme

der Metalloproteinasen, der Familie der Serinproteasen, der Familie der Cysteine und Aspartatproteasen, der ADAM-Familie , der Glycosidasen darunter Amylase, Cellulase, Dextranase, Pullulanase, Pektinase, Chitinase, Xanthanase, Chitosanase, Hyaluronidase und die Lyasen, darunter Hydroxyacetyl-Lyase, Chrondroitinase, Heparinase und Alginat-Lyase.

**3.** Verband nach Anspruch 2, in dem das erste Enzym eine Kollagenase ist.

**4.** Verband nach einem beliebigen der vorherigen Ansprüche, bei dem das besagte zweite Enzym ausgewählt wird aus der Gruppe umfassend die Enzyme der Familie der Transglutaminasen, Lysyloxydasen, Protein-Disulfid-Isomerasen, Sulfhydryl-Thiol-Oxydasen, Peroydasen, Lipoxygenasen, Epimerasen, darunter Alginatepimerasen, Glukonuratisomerasen, Cellobiose-Epimerase und Galaktose-6-Sulfurylasen.

**5.** Verband nach Anspruch 4, in dem das besagte zweite Enzym eine Transglutaminase ist.

**6.** Verband nach einem beliebigen der vorherigen Ansprüche, bei dem das Proteinpolymer ausgewählt wird aus der Gruppe umfassend Fibrin, Gliadin, Myosin, Globulin (7S und 11S), Aktin, Myoglobin, Kollagen und seine Derivate, Milchproteine, Sojaproteine, Weizenproteine, Eigelb- und Eiweißproteine, Erbsenproteine, Ackerbohnenproteine, Leinsamenproteine, Seidenproteine, Fibronektin, Laminin, Elastin, Vitronektin.

**7.** Verband nach einem beliebigen der vorherigen Ansprüche, bei dem das Saccharidpolymer aus der Gruppe umfassend Carrageen, Alginate, Xanthan, Chitosan, Chitin, Hyaluronsäure, Glukosaminoglukane als Sulfate, Glykogen, Cellulose und ihre Derivate, Pektine, Stärke und ihre Derivate, Dextrane und Xylane ausgewählt wird.

**8.** Verband nach einem beliebigen der vorherigen Ansprüche, bei dem es sich bei dem Polymer um Gelatine handelt.

**9.** Verband nach einem beliebigen der vorherigen Ansprüche, bei dem das Biomaterial außerdem eine Gefrierschutzverbindung enthält, ausgewählt aus Polyolen, Glycerin, Sorbit, Polyethylenglykol (PEG), Sorbitglycerin, Erythrit, Xylit, Arabit, Ribit, Dulcit, Galactit, Mannit, Volemit, Maltit, Isomaltit, Lactit bzw. Lactosit, Glykol und den davon abgeleiteten Polymeren.

**10.** Verband nach einem beliebigen der vorherigen Ansprüche, bei dem der Maschendurchmesser des Vliesstoffes zwischen 5 und 500 $\mu$m liegt.

**11.** Verband nach einem beliebigen der vorherigen Ansprüche, bei dem der Vliesstoff aus Viskose ist.

**12.** Herstellungsverfahren für einen Verband nach einem beliebigen der Ansprüche 1 bis 11, umfassend folgende Schritte:

a. Herstellung einer Polymerlösung umfassend ein Protein- oder Saccharidpolymer durch Mischung des besagten Polymers in einer Pufferlösung, um eine Polymerlösung zu erhalten.
b. Herstellung einer Enzymlösung umfassend ein erstes und ein zweites Enzym durch Mischung besagter Enzyme in einer Pufferlösung, um eine Enzymlösung zu erhalten.
c. Mischung der Polymer- mit der Enzymlösung,
d. Auftragen der Mischung aus Schritt (c) auf eine Fläche eines Vliesstoffes,
e. Retikulierung der auf den Vliesstoff aufgetragenen Mischung aus Schritt (c) in ein Gel,
f. Lyophilisierung des auf dem Vliesstoff gebildeten Gels,

**13.** Verfahren nach Anspruch 12 umfassend außerdem einen Sterilisierungsschritt des Verbands.

**14.** Verfahren nach Anspruch 12 oder 13, bei dem in Schritt (a) das Polymer eine Konzentration zwischen 0,5 und 5 Gewichts-% aufweist.

**15.** Verfahren nach einem beliebigen der Ansprüche 12 bis 14, bei dem die Aktivität des ersten Enzyms in der Enzymlösung zwischen $10^7$ und $10^4$ Einheiten/g Verband liegt.

**16.** Verfahren nach einem beliebigen der Ansprüche 12 bis 15, bei dem die des zweiten Enzyms in der Enzymlösung zwischen 0,5 und 10 Einheiten/g Verband liegt.

17. Verfahren nach einem beliebigen der Ansprüche 12 bis 16, bei dem die Pufferlösung der Schritte (a) oder (b) eine Lösung mit einem pH zwischen 6,5 und 8 ist.

18. Verfahren nach einem beliebigen der Ansprüche 12 bis 17, bei dem die Polymerlösung außerdem eine Gefrierschutzverbindung umfasst.

19. Verfahren nach Anspruch 18, bei dem die Konzentration der Gefrierschutzverbindung zwischen 0,1 bis 1 M, vorzugsweise zwischen 0,2 und 0,5 M liegt.

20. Verfahren nach einem beliebigen der Ansprüche 13 bis 19, bei dem der Sterilisierungsschritt durch Bestrahlung mit Gamma- oder Betastrahlen erfolgt.

21. Verfahren nach Anspruch 20, bei dem die absorbierte Strahlenmenge zwischen 0,5 und 50 kGy liegt.

22. Verfahren nach einem beliebigen der Ansprüche 12 bis 21, bei dem der Schritt (c) der Enzym- und Polymerlösungen nach einem Mindestverhältnis von 1/5 unter Rühren gemischt werden.

23. Verfahren nach einem beliebigen der Ansprüche 12 bis 22, bei dem der Schritt f der Lyophilisierung unter einem Druck von $75x10^3$ Pa bis $300x10^3$ Pa erfolgt und abgesehen vom Gefrierschritt folgende Schritte umfasst:

    g. Sublimierungsschritt: Temperaturerhöhung um -45° C auf eine Temperatur zwischen 5 und 30° C bei einer Geschwindigkeit von 0,01 ° C pro Minute,
    h. Austrocknungsschritt: Beibehaltung der beim Sublimationsschritt erhaltenen Temperatur über 10 bis 70 Stunden.

**Claims**

1. A wound dressing comprising a carrier having on one side thereof, a biomaterial capable of making a gel / solution transition comprising an aqueous phase and a polymer network comprising a protein or saccharide polymer or a mixture of protein and saccharide polymers, wherein the polymer network and the aqueous phase form a gel, a first degrading enzyme of said polymer and a second enzyme capable of forming covalent bonds between monomers, said biomaterial being lyophilized, and said carrier being a nonwoven backing.

2. A wound dressing according to claim 1 wherein said first enzyme is selected from the group comprising enzymes of the metalloproteinase family, the serine protease family, the cysteine and aspartate protease family, the ADAM family, glycosidases, including amylase, cellulase, dextranase, pullulanase, pectinase, chitinase, xanthanase, chitosanase, hyaluronidase, and lyases, including hydroxyacetyl lyase, chondroitinase, heparinase and alginate lyase.

3. A wound dressing according to claim 2 wherein the first enzyme is a collagenase.

4. A wound dressing according any preceding claim wherein said second enzyme is selected from the group comprising enzymes of the transglutaminases familly, lysyls oxidase, disulfide isomerase proteins, sulfhydryl thiol oxidases, peroxidases, lipoxygenases, epimerases, including alginate epimerases, glucuronate isomerases, cellobiose epimerase and galactose-6-sulfurylases

5. A wound dressing according to claim 4 wherein the second enzyme is a transglutaminase.

6. A wound dressing according any preceding claim wherein the protein polymer is selected from the group comprising fibrin, gliadin, myosin, globulin (7S and 11 S), actin, myoglobin, collagen and its derivatives, milk proteins, soy proteins, wheat proteins, egg-yolk and egg-white proteins, pea proteins, horse bean proteins, flax proteins, silk proteins, fibronectin, laminin, elastin and vitronectin.

7. A wound dressing according any preceding claim wherein the saccharide polymer is selected from the group comprising carraghenans, alginates, xanthan, chitosan, chitin, hyaluronic acid, sulfated glycosaminoglycans, glycogen, cellulose and its derivatives, pectins, starch and its derivatives, dextrans and xylans.

8. A wound dressing according any preceding claim wherein the polymer is gelatin.

**9.** A wound dressing according any preceding claim wherein the biomaterial further comprises a cryoprotectant compound selected from polyols, glycerol, sorbitol, polyethylene glycol (PEG), sorbitol, glycerol, erythritol, xylitol, the arabitol, ribitol, dulcitol, galactitol, mannitol, volemitol, maltitol, isomaltitol, lactitol or lactositol, glycol and their derivatives polymers.

**10.** A wound dressing according to any preceding claim wherein the meshes diameter of the nonwoven is comprised from 5 to 500 $\mu$m.

**11.** A wound dressing according to any preceding claim wherein the nonwoven is viscose.

**12.** A method of manufacturing a wound dressing according to any one of claims 1 to 11, comprising the steps of ::

a. preparing a polymer solution comprising a protein polymer and/or a saccharide polymer by mixing said polymer in a buffer solution, to obtain a polymer solution,
b. preparing an enzyme solution comprising a first and a second enzyme by mixing of said enzyme into a buffer solution, to obtain an enzyme solution,
c. mixing of the polymer solution and the enzyme solution
d. depositing the mixture of step (c) onto a nonwoven surface,
e. crosslinking the mixture of step (c) deposited on the nonwoven into gel,
f. lyophilizing the gel formed on the nonwoven.

**13.** A method of manufacturing according to claim 12 further comprising a step of sterilizing the dressing.

**14.** A method of manufacturing according to claim 12 or 13 wherein in step a) the polymer is at a concentration of 0.5 to 5% by weight.

**15.** The method according to any claim 12 to 14, wherein the activity of the first enzyme in the enzyme solution is comprised from $10^{-7}$ to $10^{-4}$ units/g of dressing.

**16.** The method according to any of claim 13 to 15, wherein the activity of the second enzyme in the enzyme solution is comprised from 0.5 to 10 units/g of dressing.

**17.** The method according to any of claim 13 to 16, wherein the buffer solution of steps a) or b) is a solution with a pH comprises from 6 to 8,5.

**18.** The method according to any of claim 13 to 17, wherein the buffer solution further comprising a cryoprotectant.

**19.** The method according to claim 18, wherein the cryoprotectant concentration is from 0.1 to 1 M, preferably from 0.2 to 0.5 M.

**20.** The method according to any one of claims 13-19, wherein the sterilization step is carried out by irradiation with gamma or beta radiation.

**21.** The method according to claim 20, wherein the amount of absorbed radiation is from 0.5 to 50 kGy.

**22.** A method according to any one of claims 12 to 21, wherein in step (c) the enzyme and polymer solutions are mixed in a minimum ratio of 1/5 and stirred.

**23.** The method according any one of claims 13 to 22, wherein the lyophilization step f) is carried out under a pressure from $75 \times 10^{-3}$ Pa to $300 \times 10^{-3}$ Pa and further comprises the step of freezing, the following steps of:

g. Sublimation step: increasing the temperature from -45 °C to a temperature between 5 and 30 °C at a rate of 0.01 °C per minute,
h. Drying Step: maintains the temperature obtained during the step of sublimation time from 10 to 70 hours

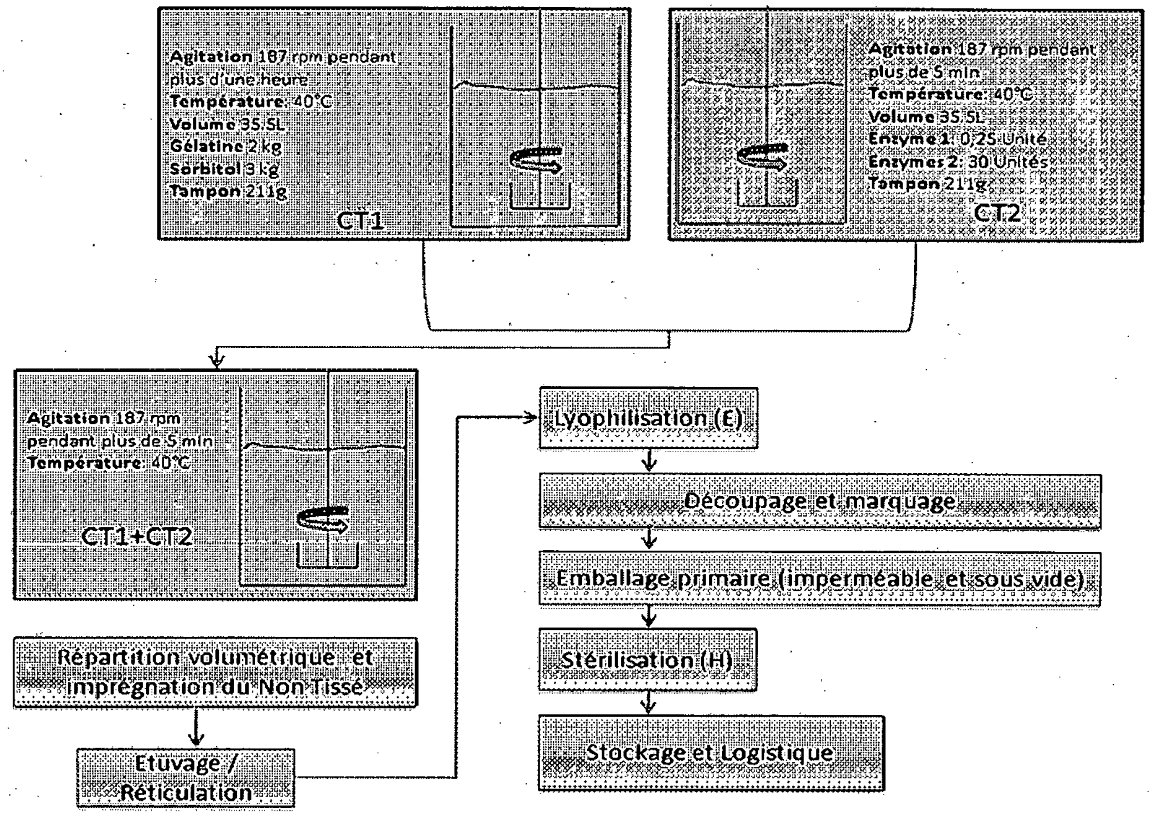
Agitation 187 rpm pendant plus d'une heure
Température: 40°C
Volume 35,5L
Gélatine 2 kg
Sorbitol 3 kg
Tampon 211g
CT1
Agitation 187 rpm pendant plus de 5 min
Température: 40°C
Volume 35,5L
Enzyme 1: 0,25 Unité
Enzymes 2: 30 Unités
Tampon 211g
CT2
Agitation 187 rpm pendant plus de 5 min
Température: 40°C
CT1+CT2
Répartition volumétrique et imprégnation du Non Tissé
Etuvage / Réticulation
Lyophilisation (E)
Découpage et marquage
Emballage primaire (imperméable et sous vide)
Stérilisation (H)
Stockage et Logistique

FIGURE 1

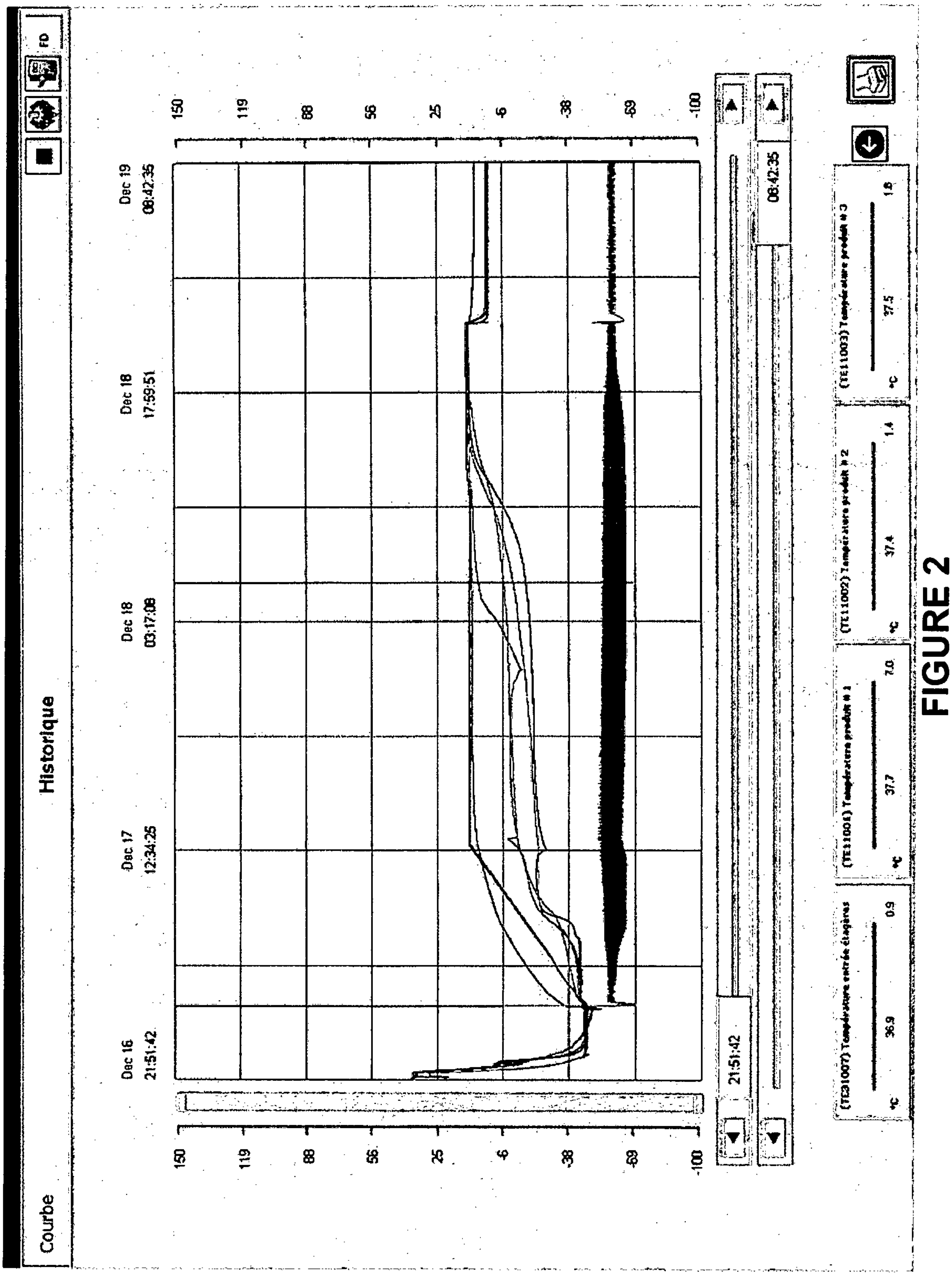
Courbe
Historique
150
119
88
56
25
-6
-38
-69
-100
Dec 16
21:51:42
Dec 17
12:34:25
Dec 18
03:17:08
Dec 18
17:59:51
Dec 19
08:42:35
21:51:42
08:42:35

FIGURE 2

Sans Sorbitol Avec Sorbitol

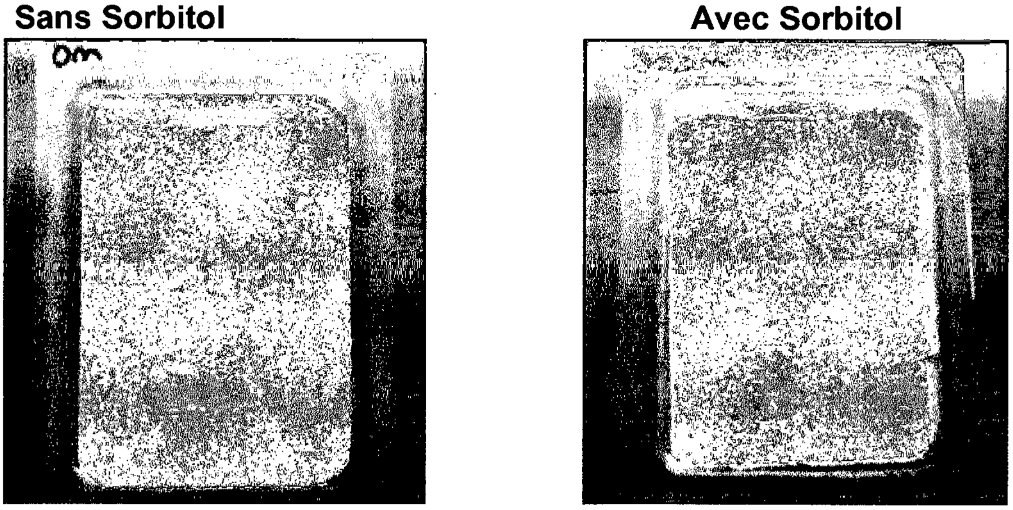

Collant, rigide collant souple

FIGURE 3

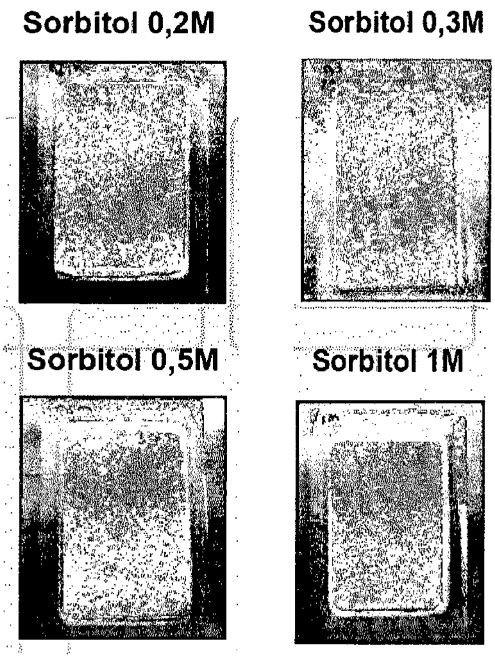


FIGURE 4

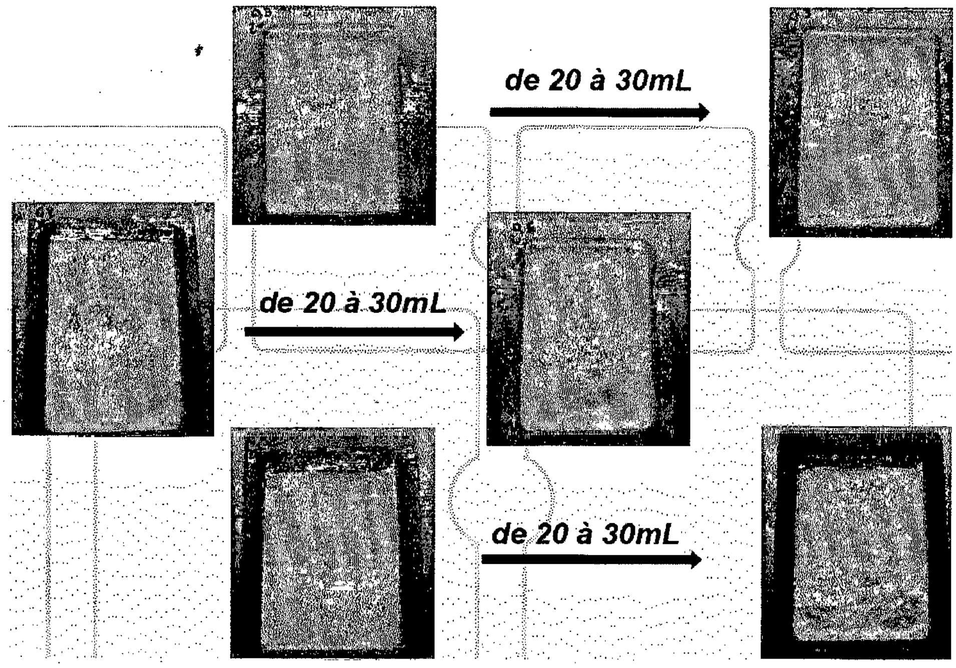


FIGURE 5

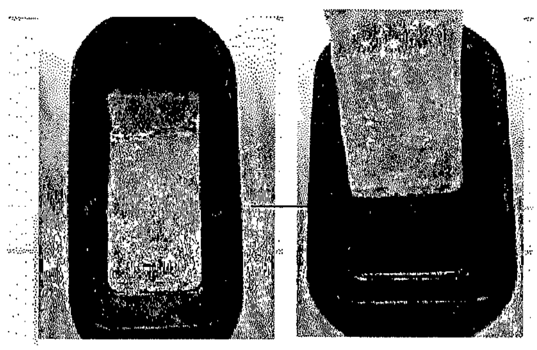

FIGURE 6

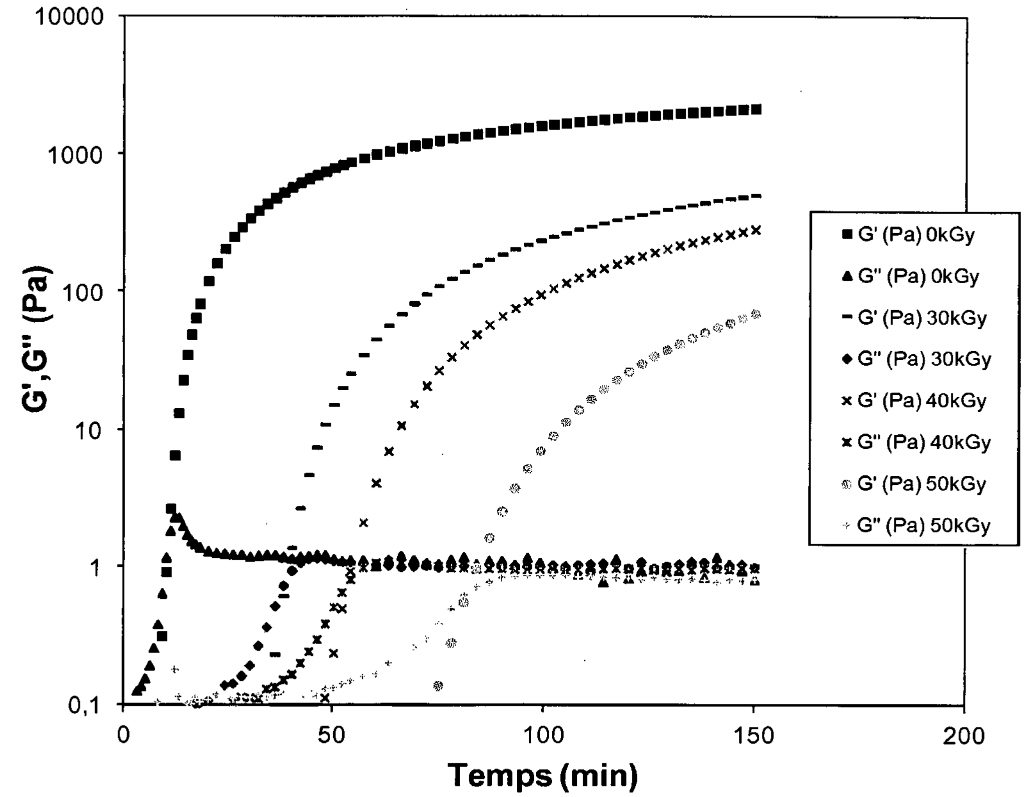


**Figure 7 A**

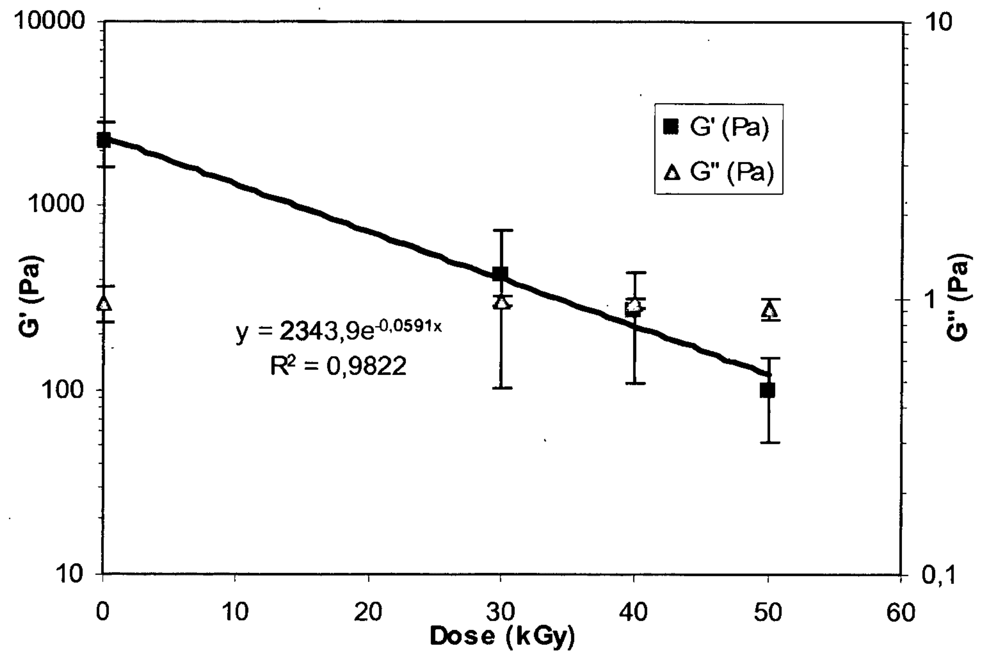


**Figure 7B**

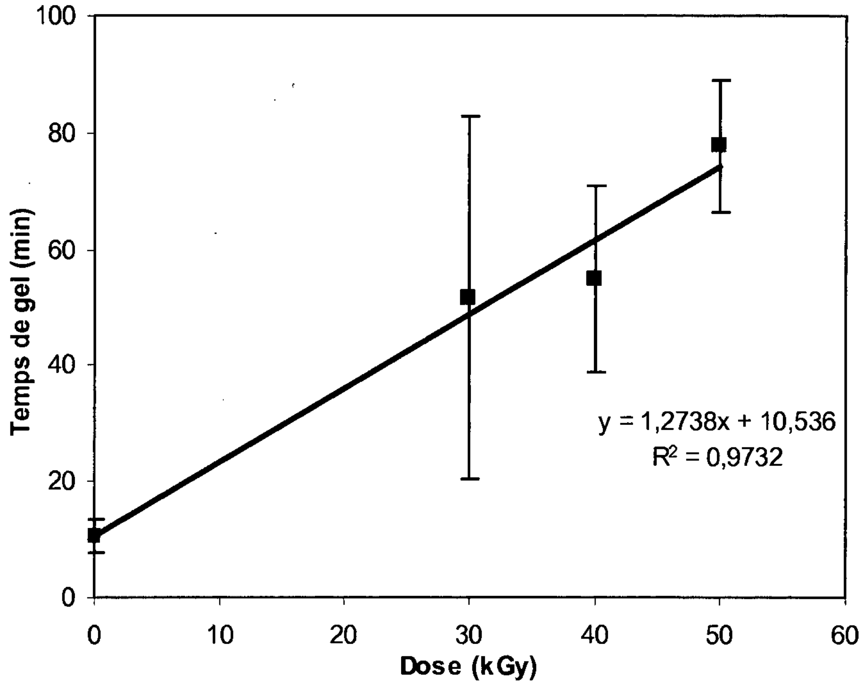


Figure 7 C

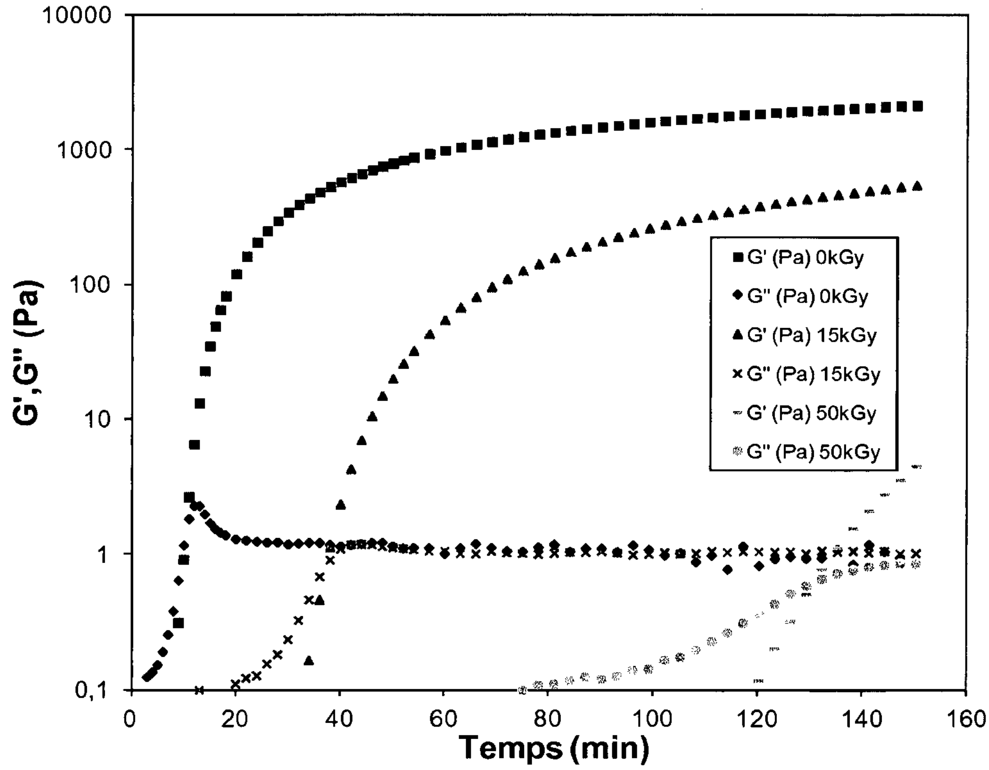


Figure 8 A

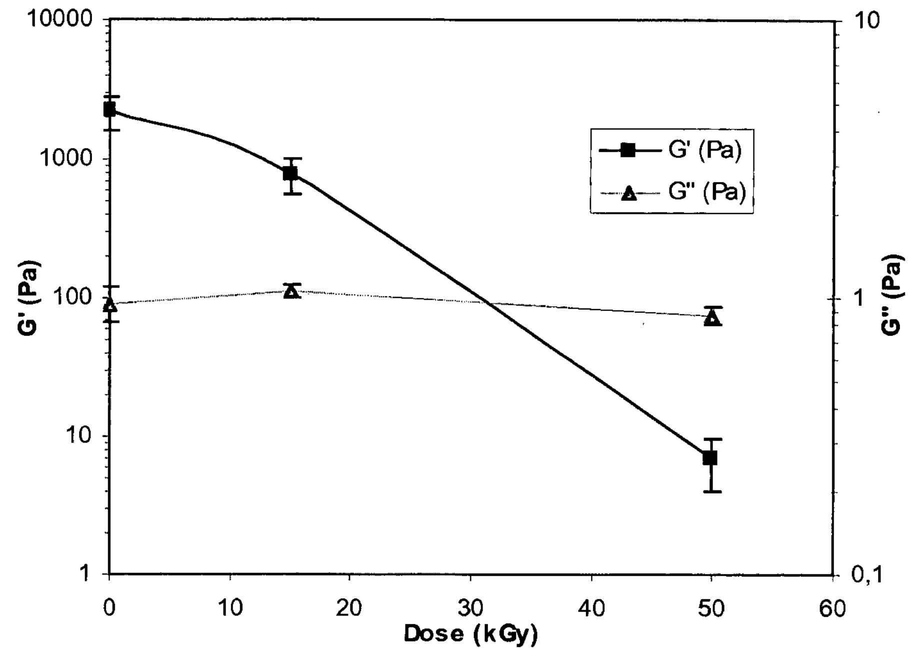


Figure 8 B

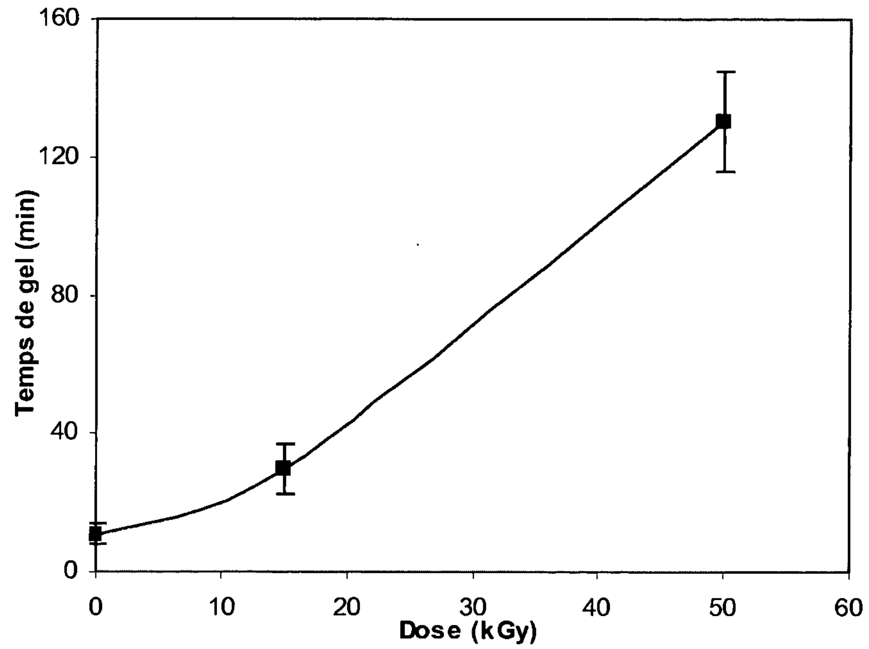


Figure 8 C

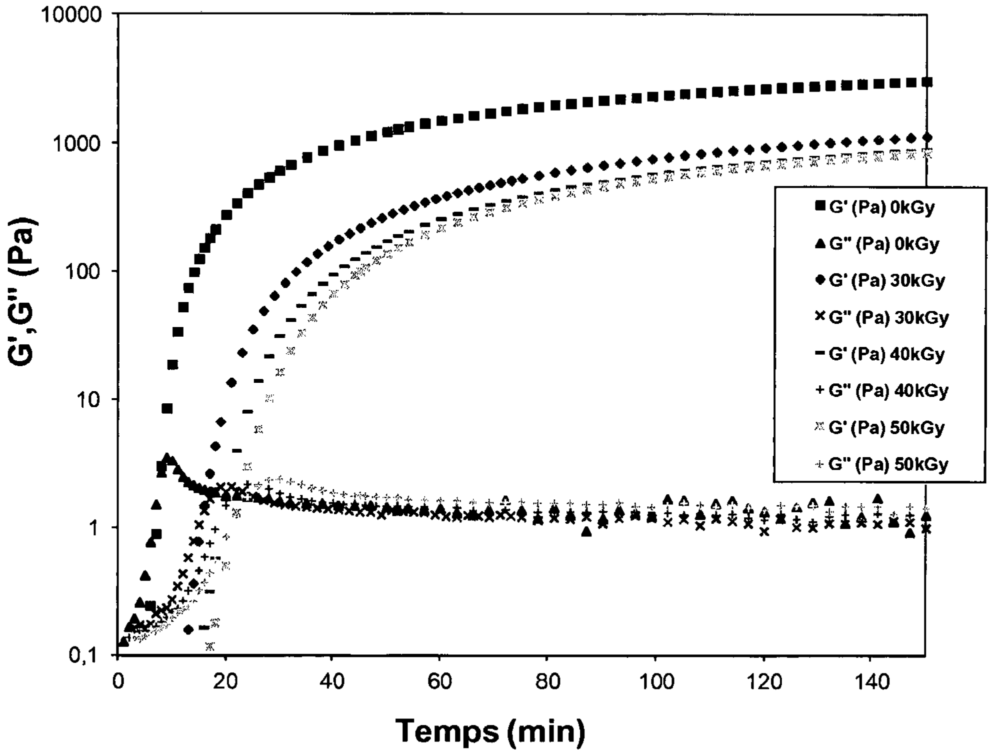


Figure 9 A

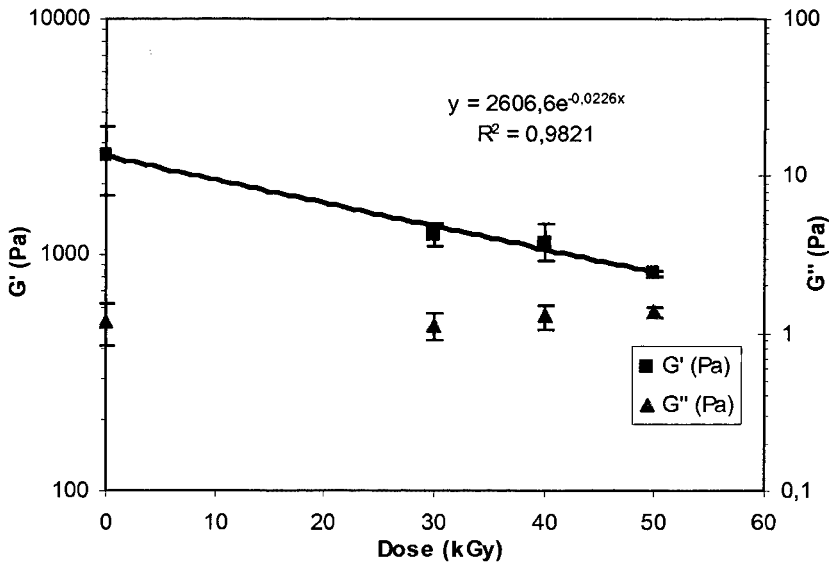


Figure 9 B

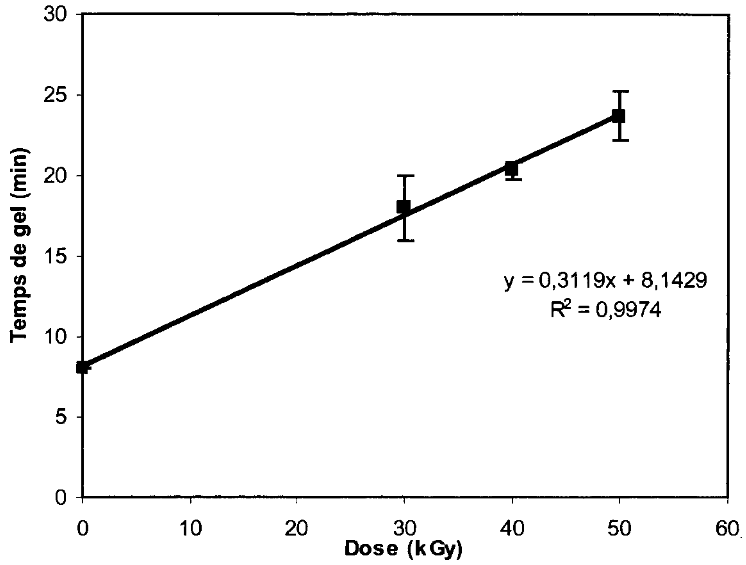


Figure 9 C

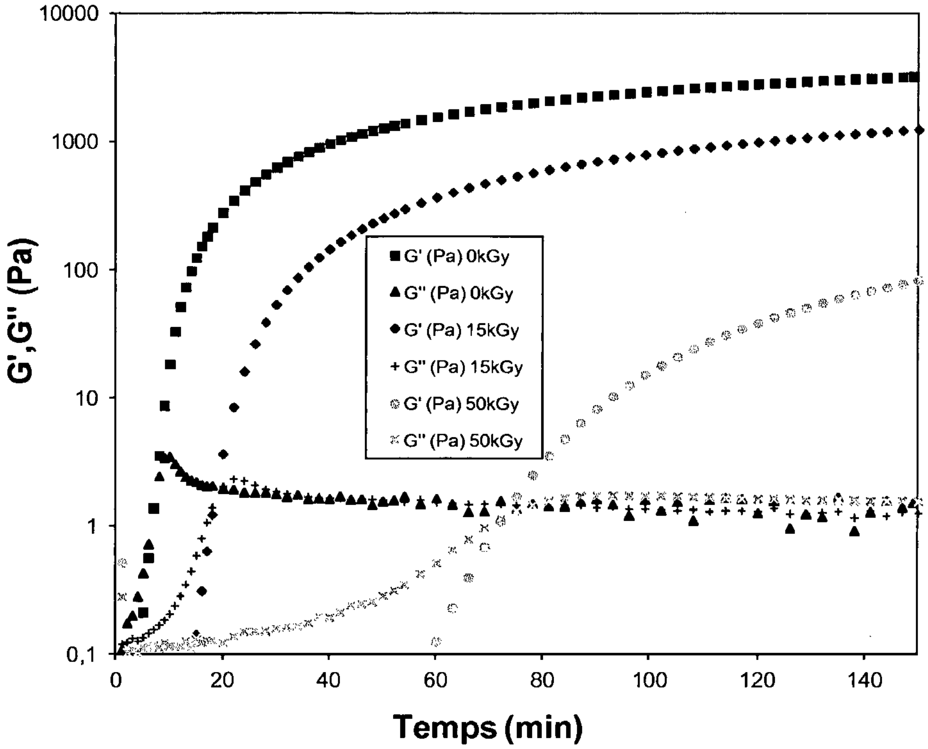


Figure 10 A

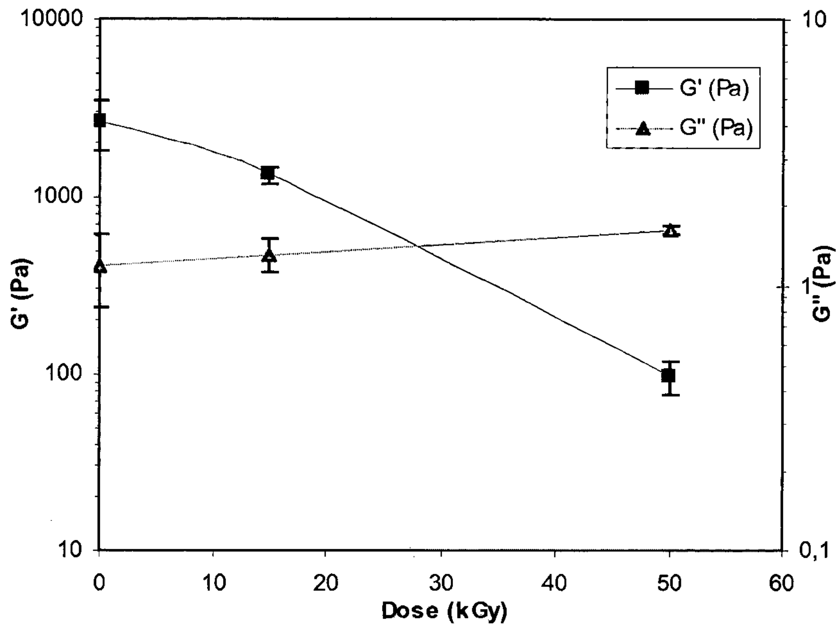


Figure 10 B

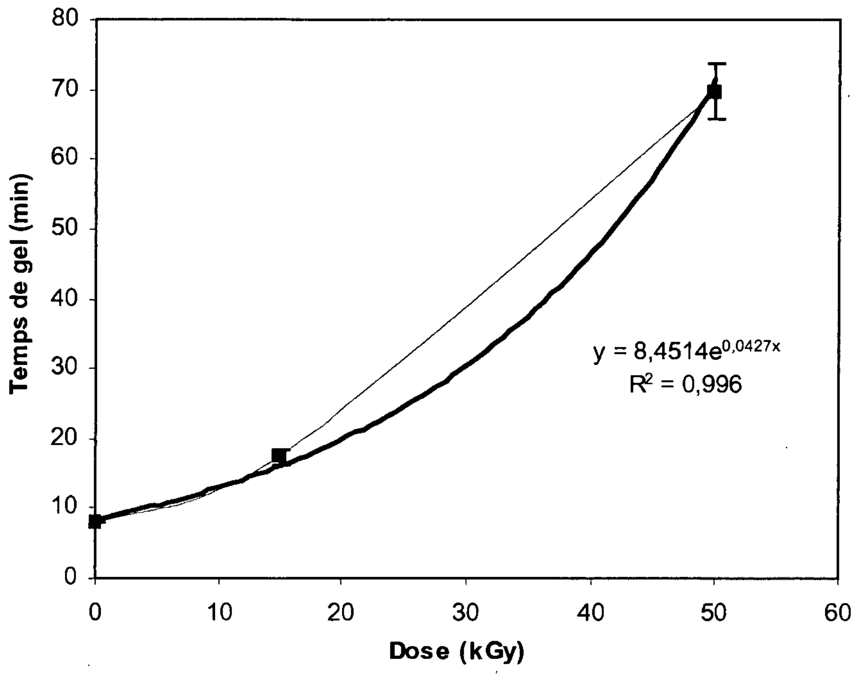


Figure 10 C

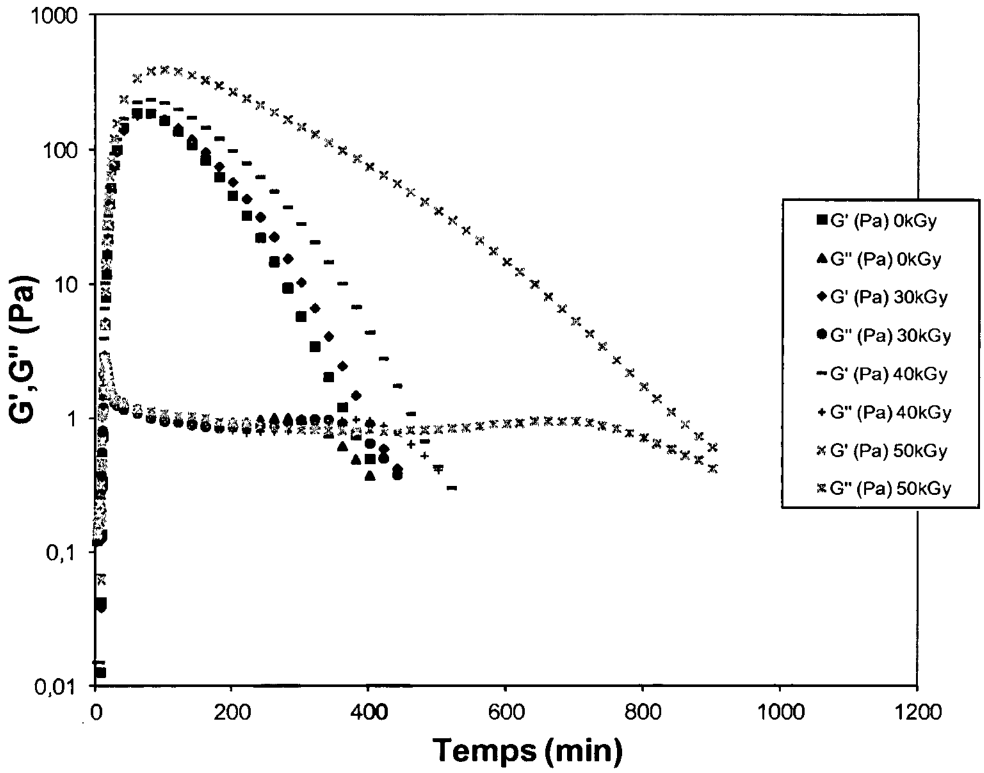


**Figure 11A**

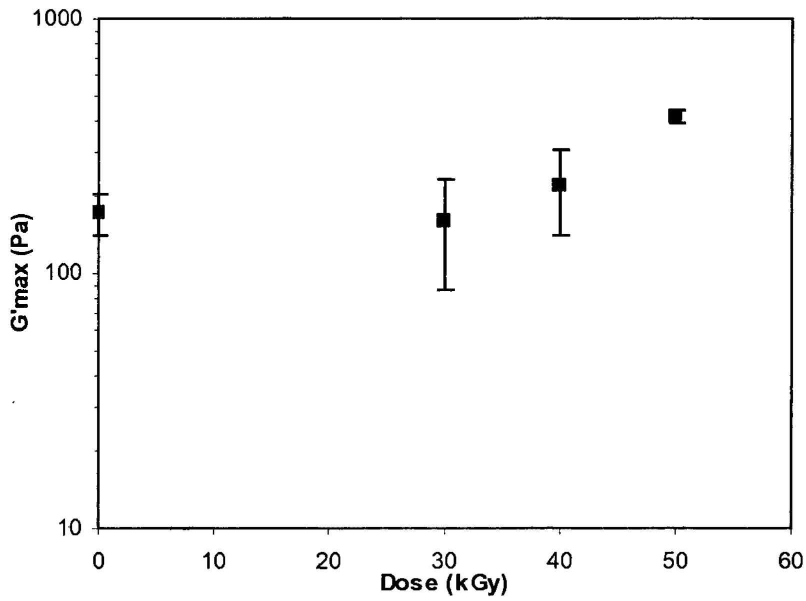


**Figure 11 B**

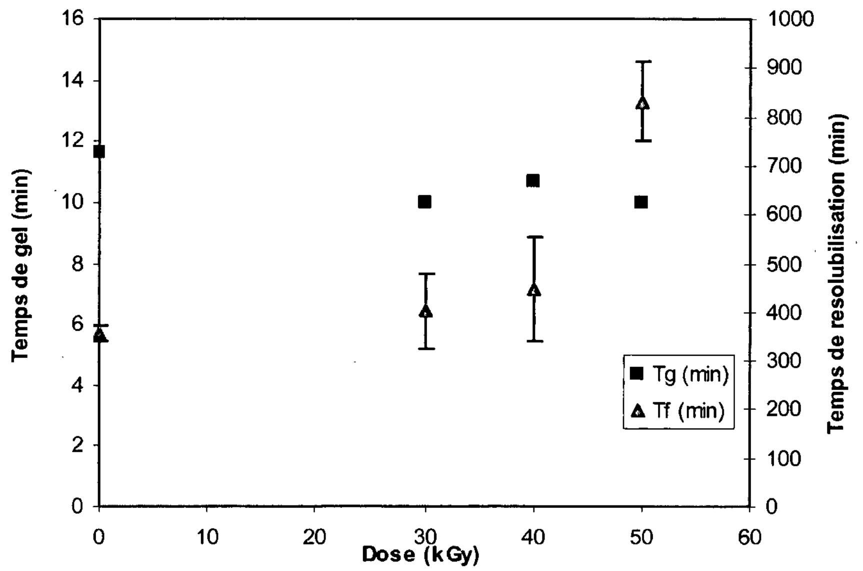


Figure 11 C

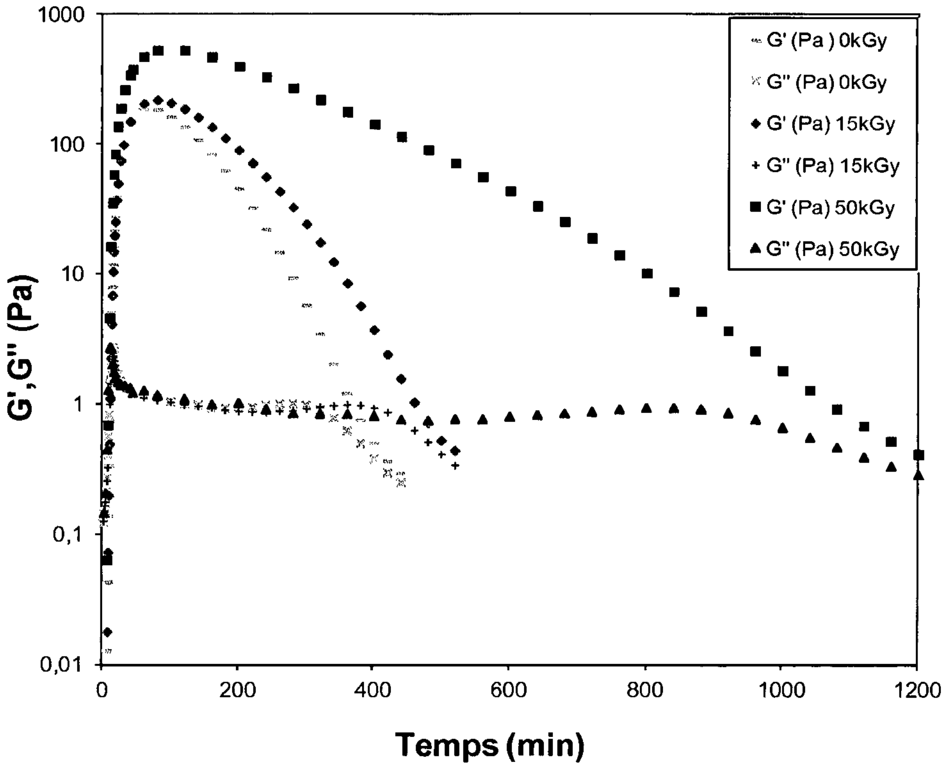


Figure 12 A

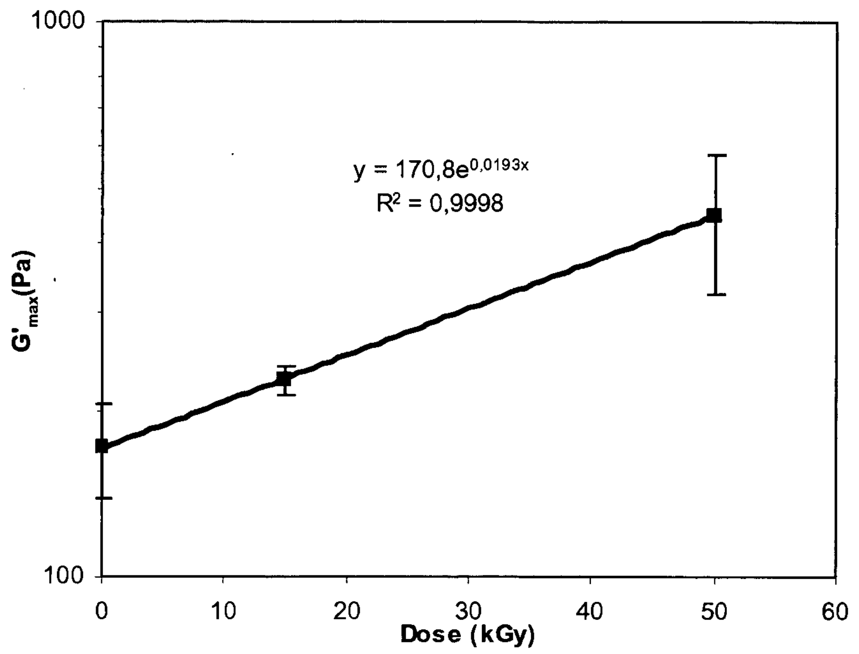


Figure 12 B

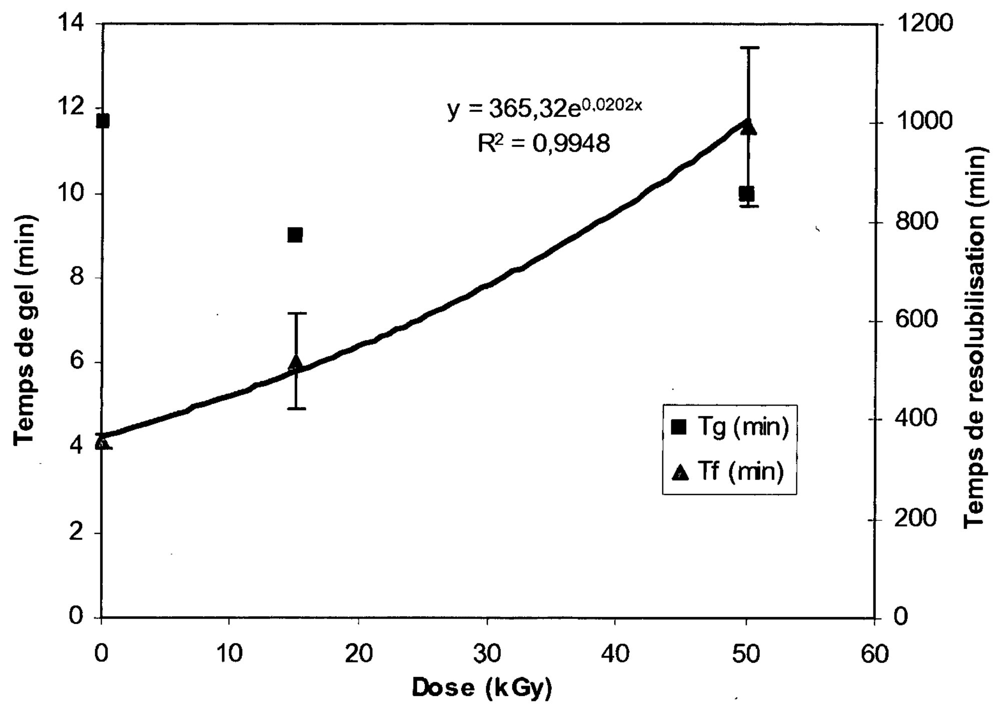


Figure 12 C

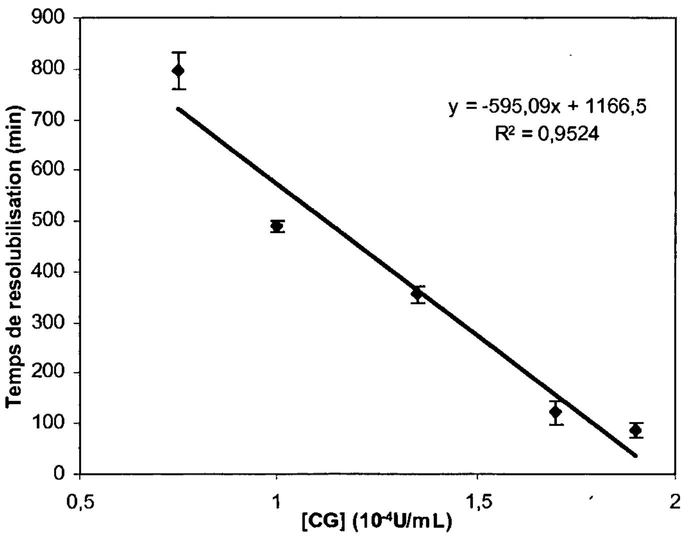


Figure 13 A

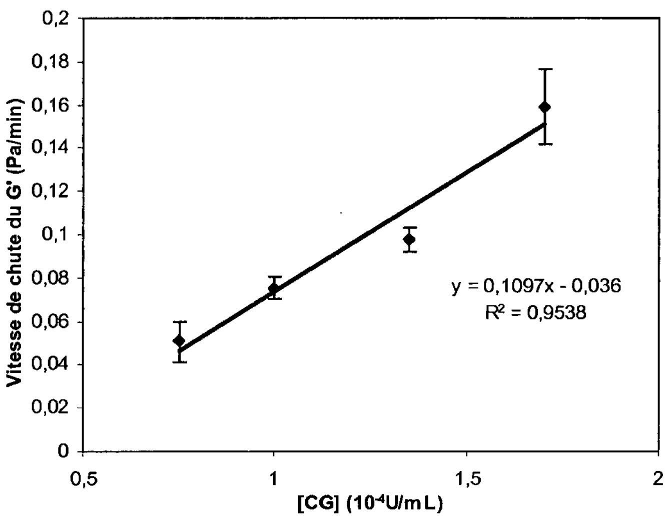


Figure 13 B

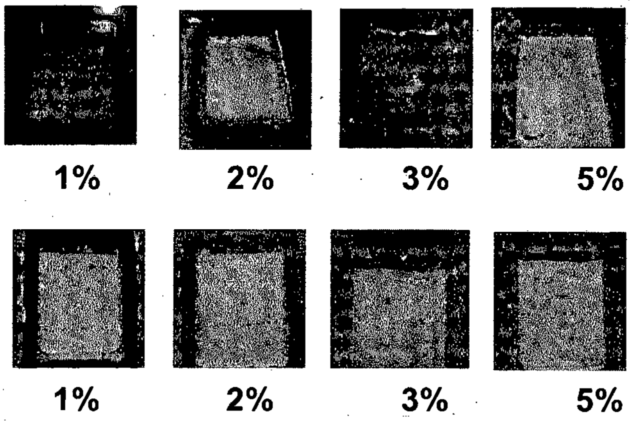


Figure 14

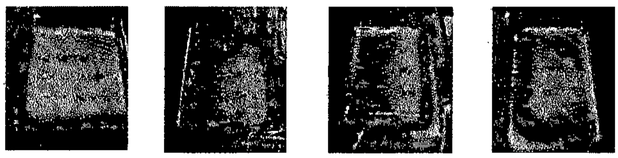

1% 2% 3% 5%

Figure 15

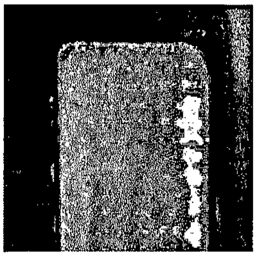

Lyophal

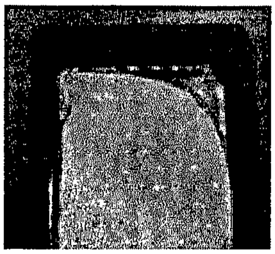

MA50 + PE

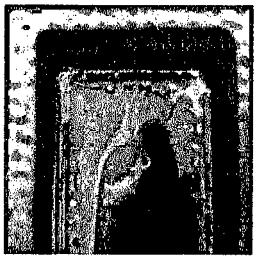

MA50 + PE

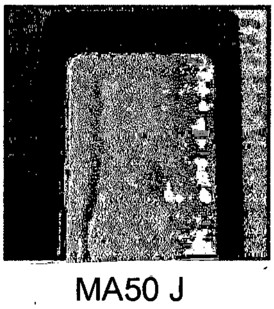

MA50 J

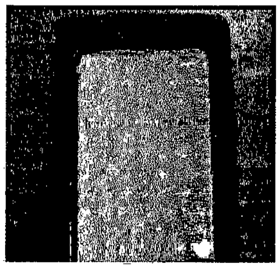

MA57 S

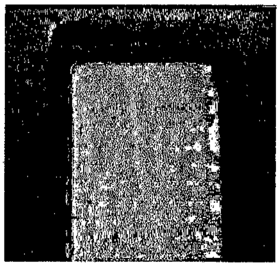

WSV 100

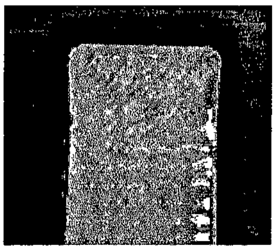

Tissue Ig + PE

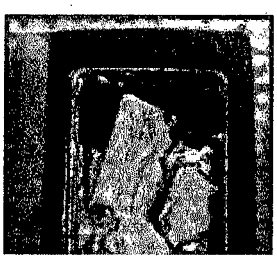

Tissue Ig + PE

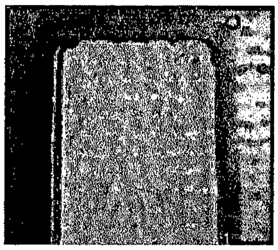

MAP50

**Figure 16**

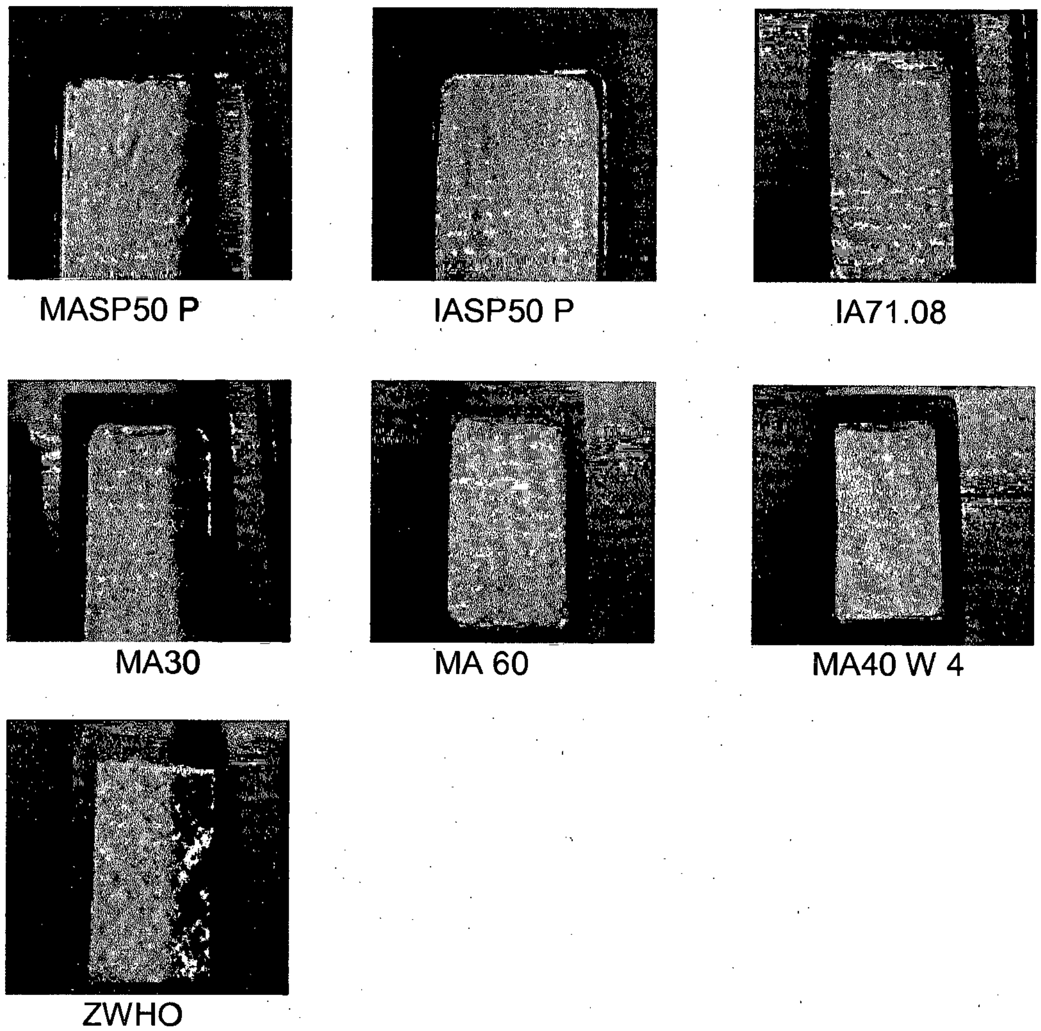


**Figure 17**

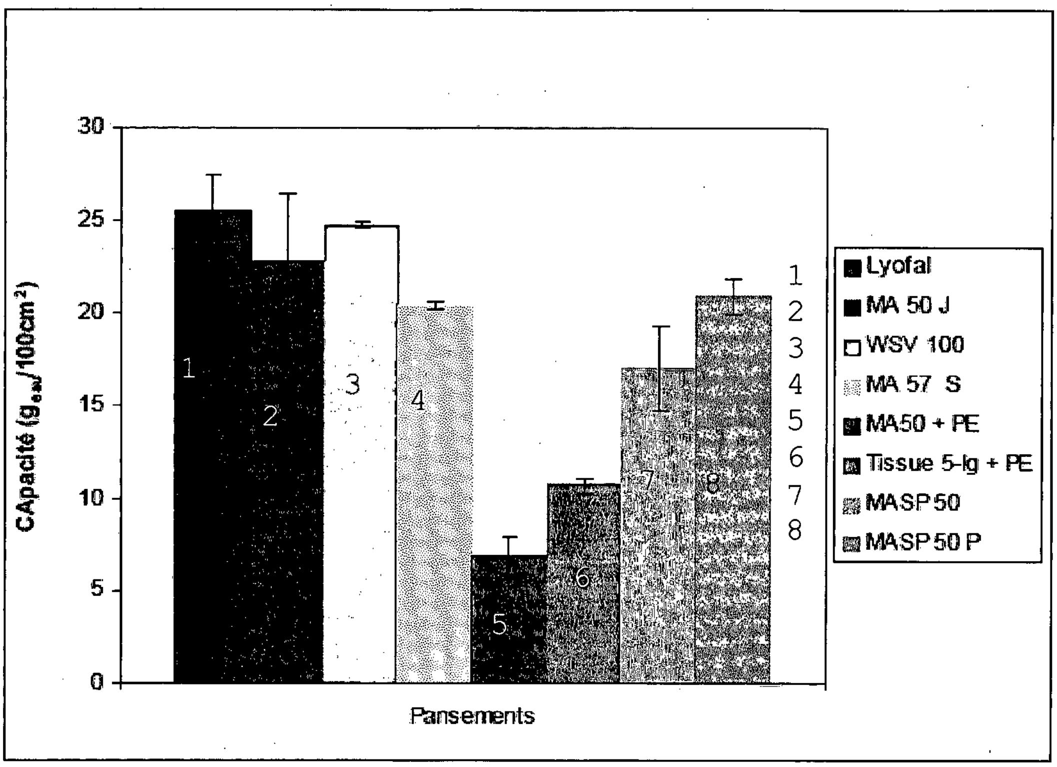

30
25
20
15
10
5
0
CApacité (g$_{eau}$/100cm$^2$)
1
2
3
4
5
6
7
8
Pansements
1
2
3
4
5
6
7
8
Lyofal
MA 50 J
WSV 100
MA 57 S
MA50 + PE
Tissue 5-lg + PE
MASP 50
MASP 50 P


Figure 18

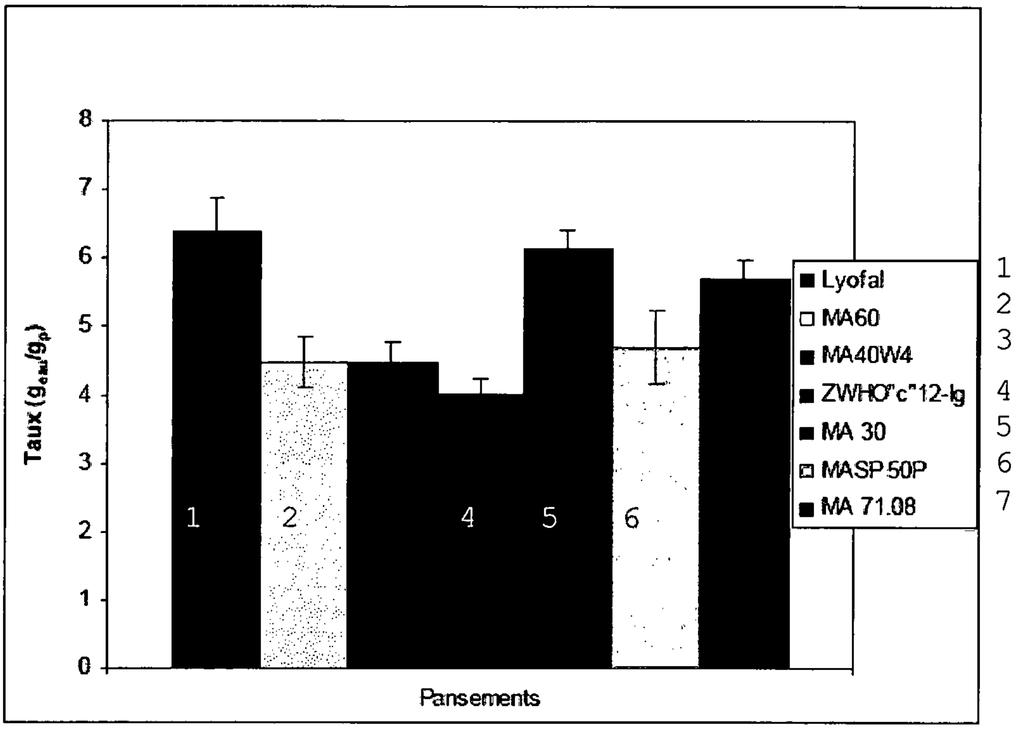


**Figure 19**

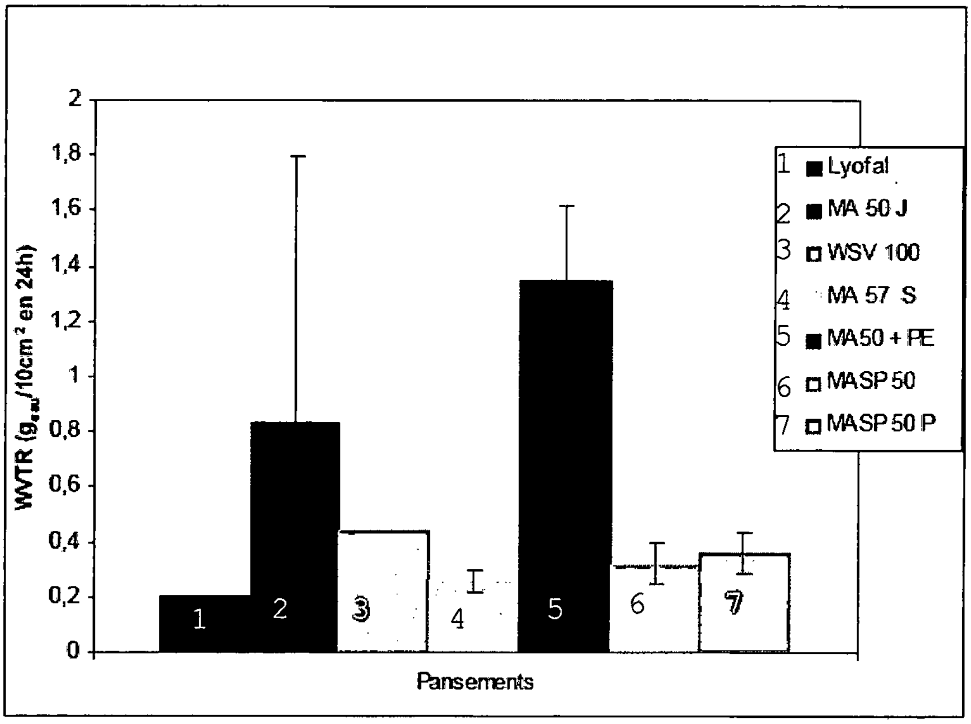


**Figure 20**

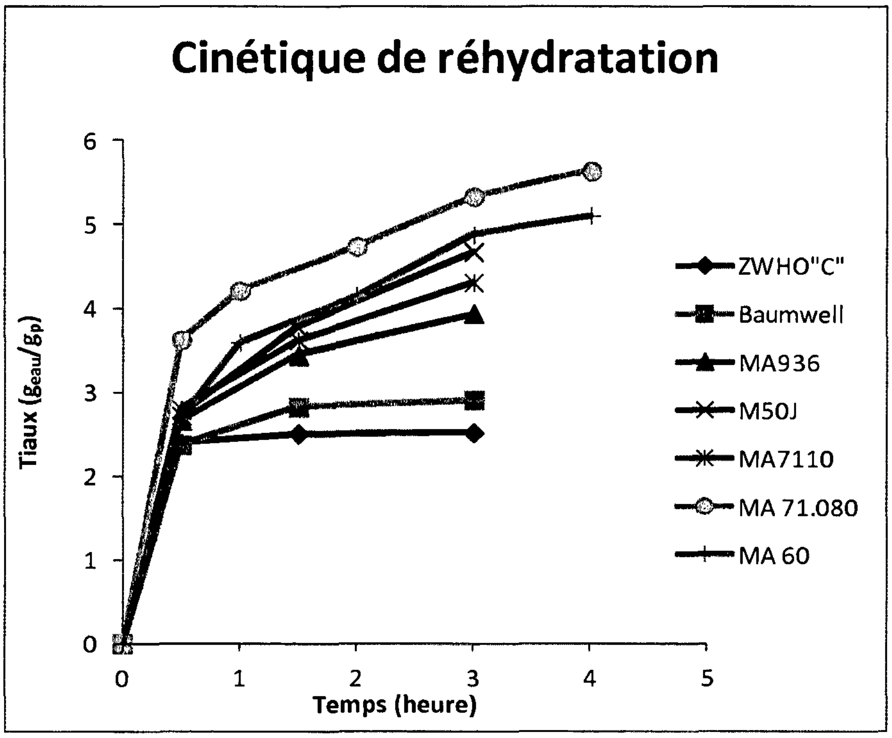


Figure 21

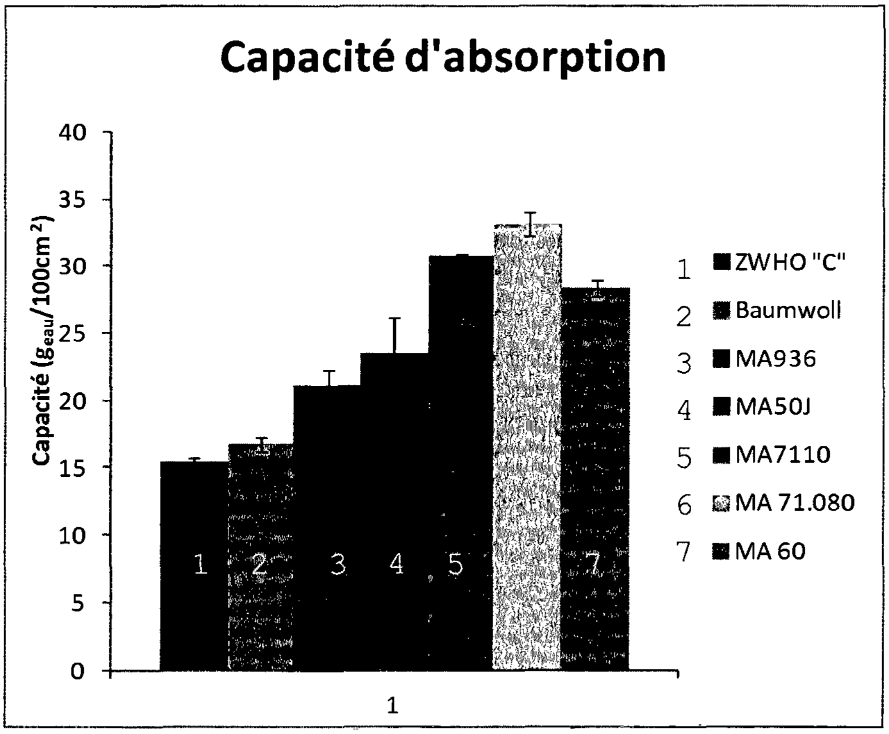


Figure 22

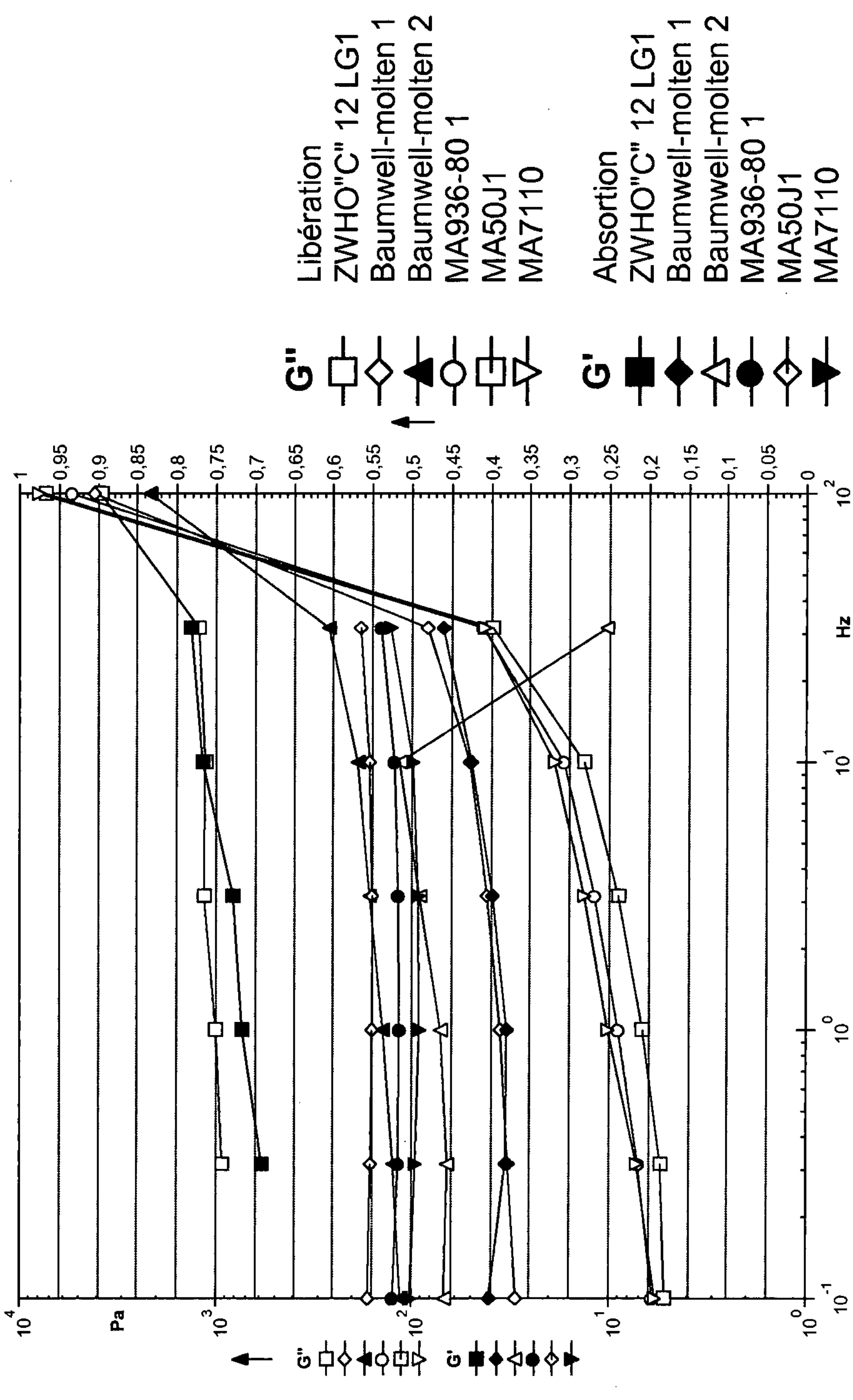
G''
Libération
ZWHO"C" 12 LG1
Baumwell-molten 1
Baumwell-molten 2
MA936-80 1
MA50J1
MA7110
G'
Absortion
ZWHO"C" 12 LG1
Baumwell-molten 1
Baumwell-molten 2
MA936-80 1
MA50J1
MA7110
Pa
Hz

Figure 23

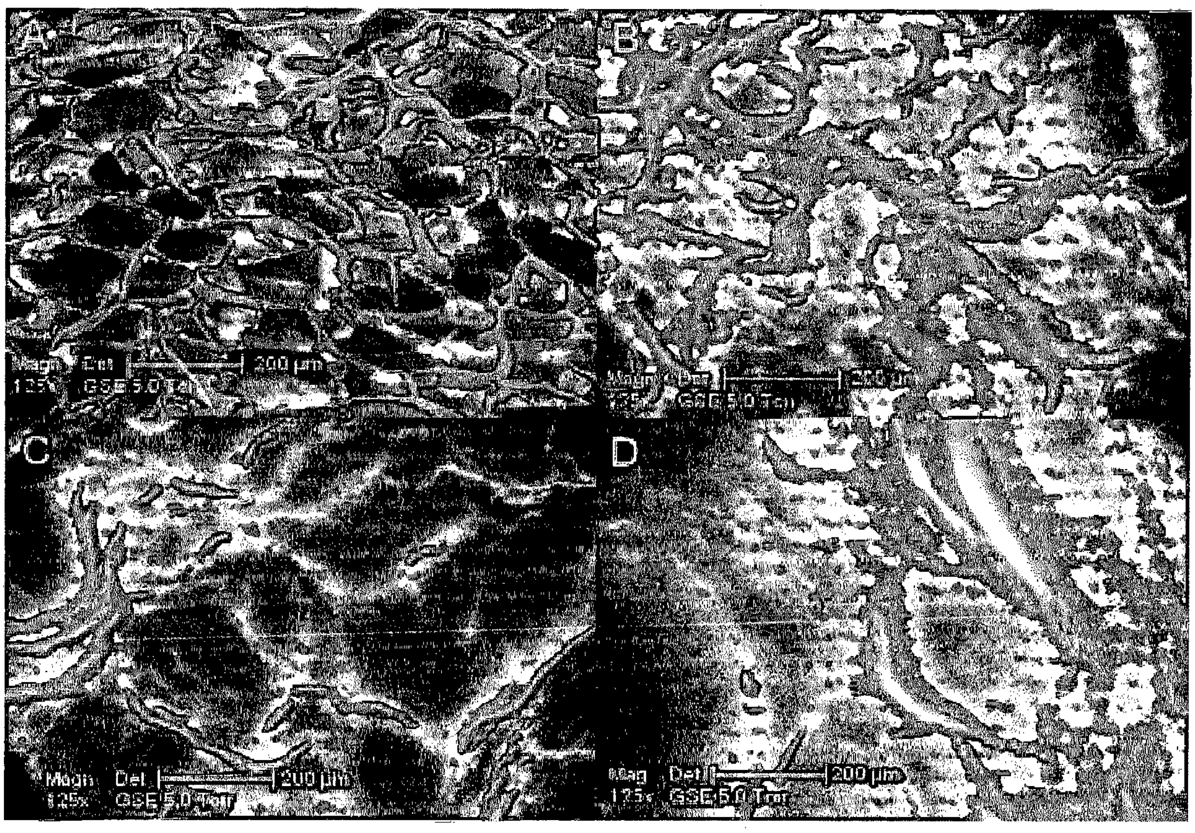

Figure 24

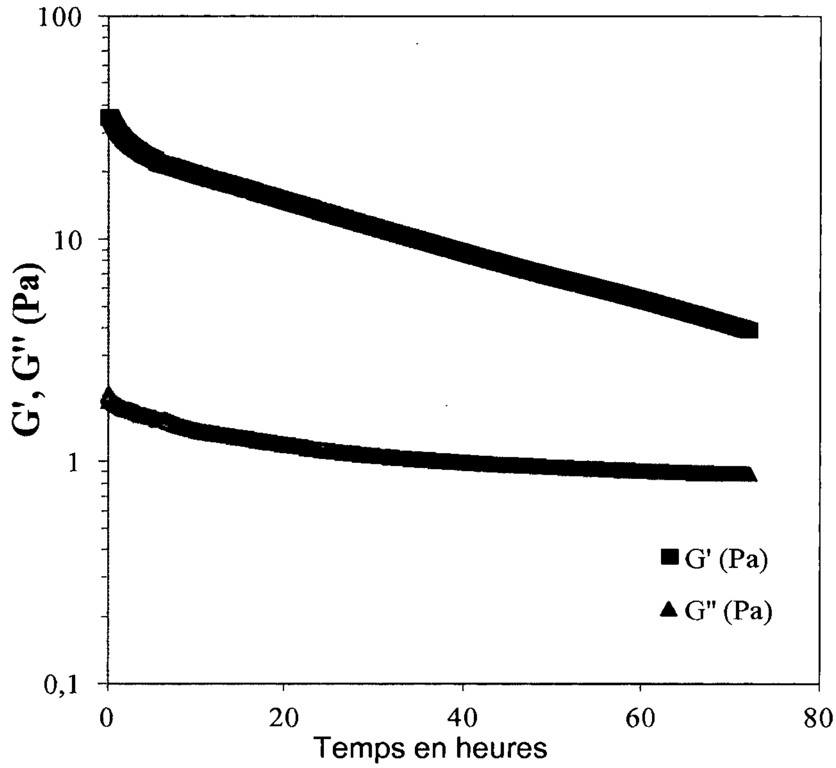


**Figure 25**

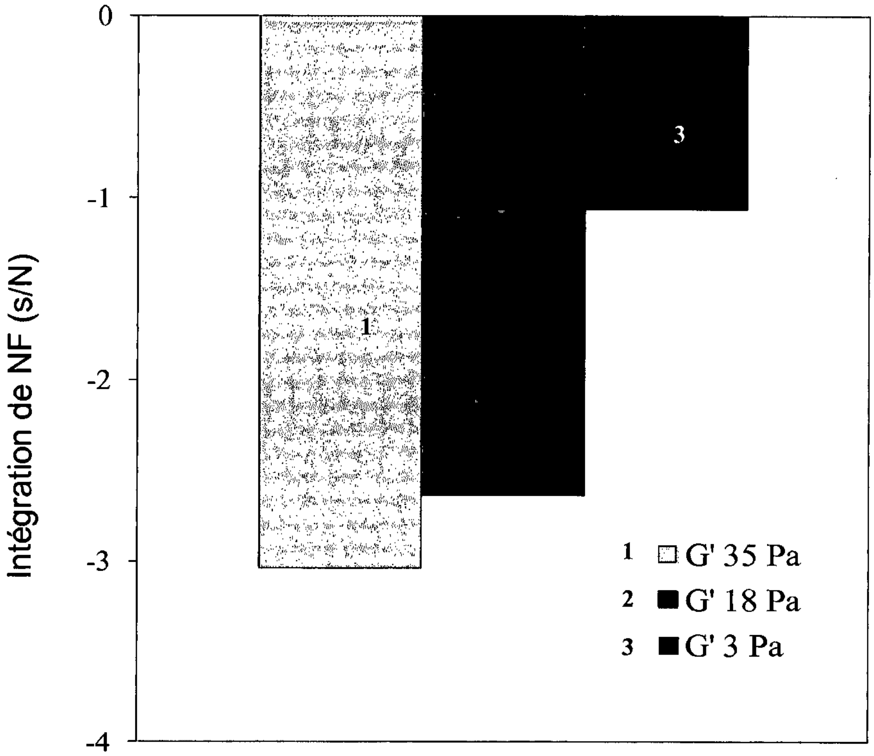


Figure 26

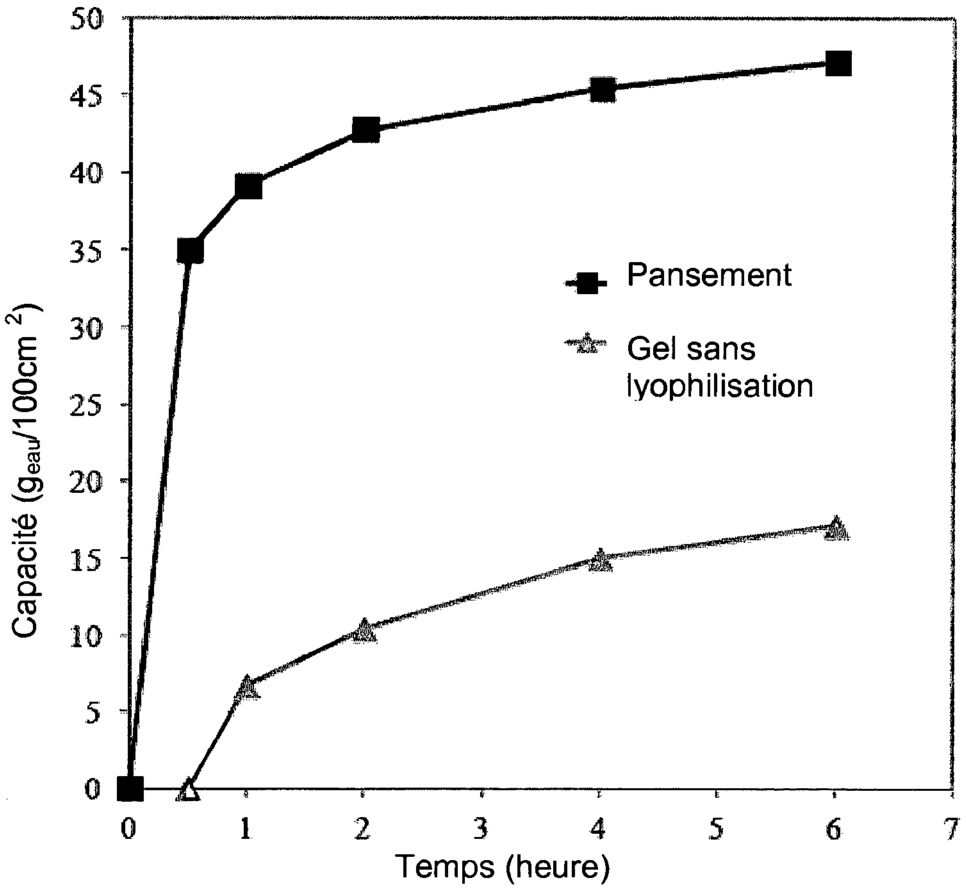
50
45
40
35
30
25
20
15
10
5
0
Capacité ($g_{eau}$/100cm $^2$)
0
1
2
3
4
5
6
7
Temps (heure)
Pansement
Gel sans lyophilisation

**Figure 27**

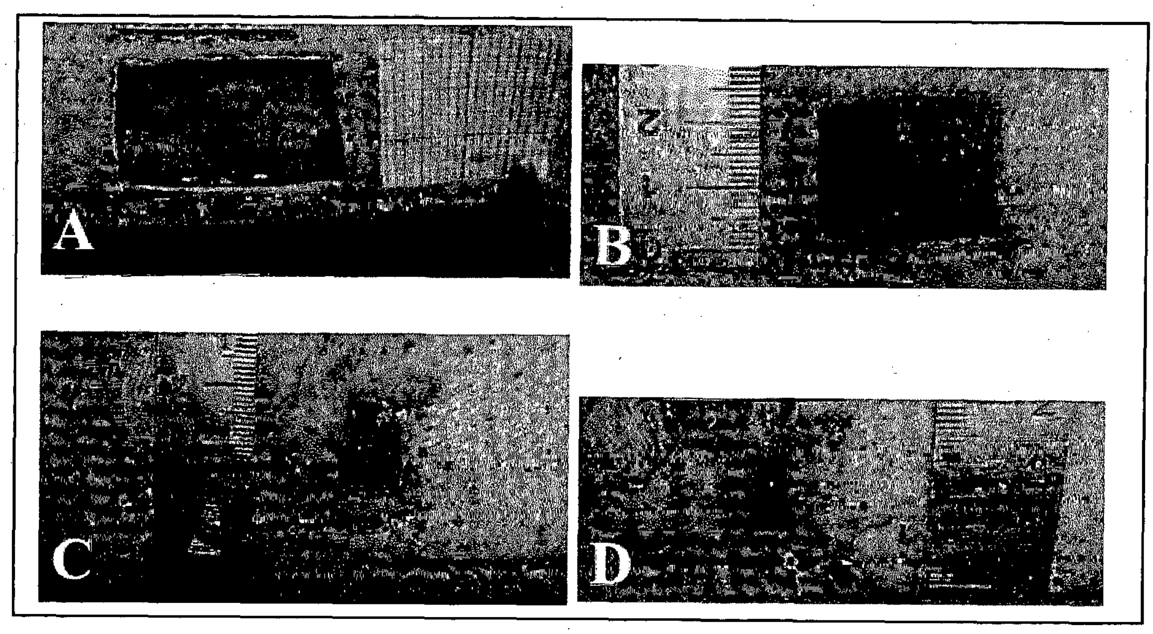


Figure 28

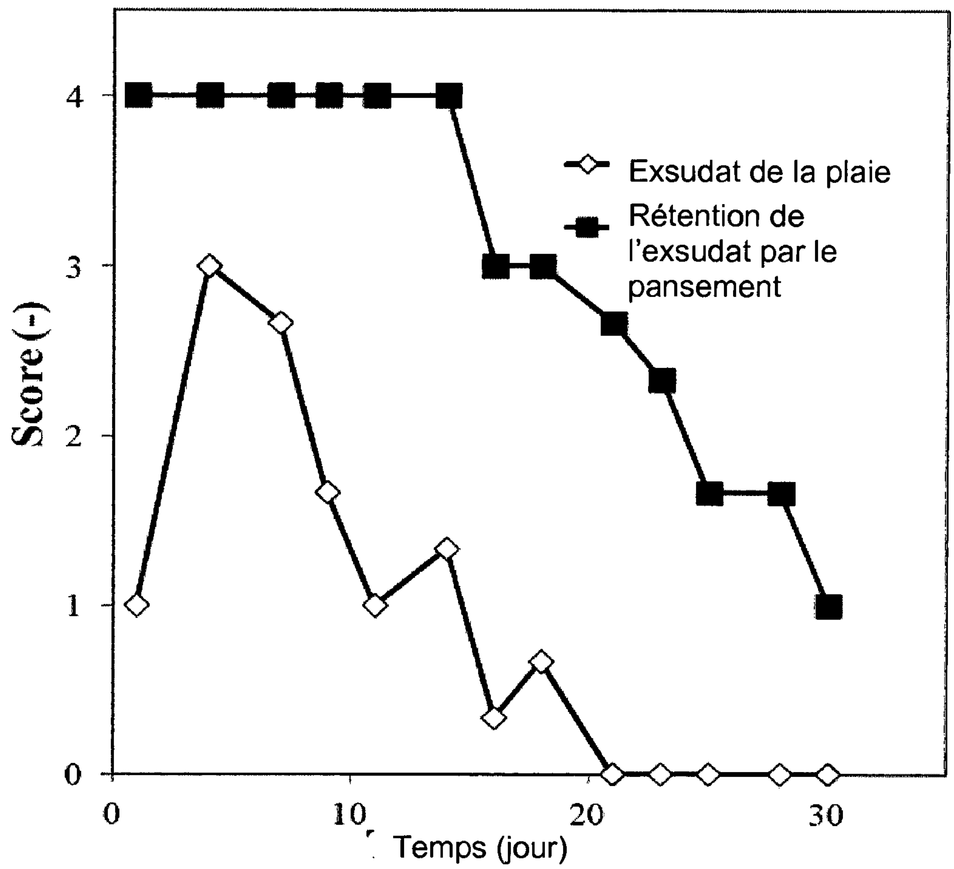


**Figure 29**

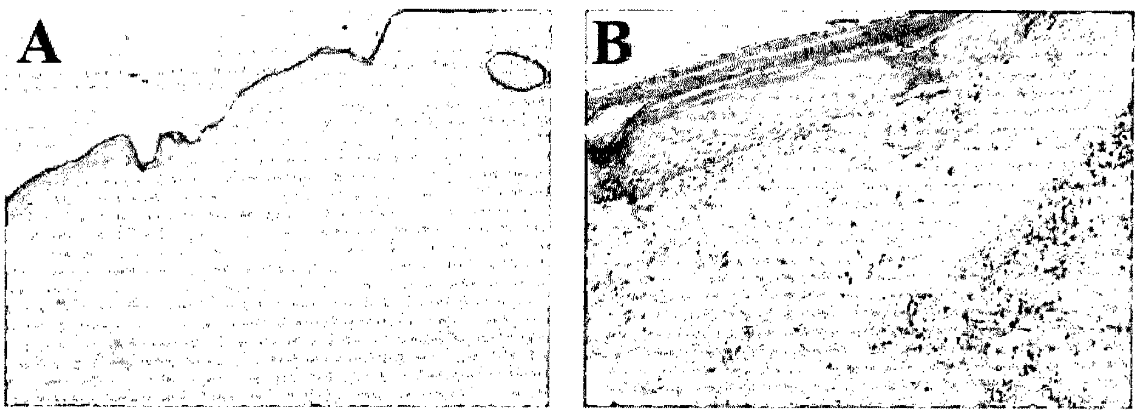


**Figure 30**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GB 2382305 A
- WO 2006056700 A


### Littérature non-brevet citée dans la description


- **WINTER GD.** Formation of the scab and the rate of epithelialisation of superficial wounds in the skin of the young domestic pig. *Nature,* 1962, vol. 193, 293-294